(19) Europäisches Patentamt
European Patent Office
Office européen des brevets

(11) Numéro de publication: **0 432 017 B1**

(12) **FASCICULE DE BREVET EUROPEEN**

(45) Date de publication du fascicule du brevet: **16.08.95**

(51) Int. Cl.[6]: **C07D 491/16**, //(C07D491/16, 311:00,221:00,221:00)

(21) Numéro de dépôt: **90403379.2**

(22) Date de dépôt: **28.11.90**

Le dossier contient des informations techniques présentées postérieurement au dépôt de la demande et ne figurant pas dans le présent fascicule.

# (54) Dérivés de tétrahydro-2,3,6,7,1H,5H,11H-(1)benzopyrano(6,7,8,ij)quinolizinone-11 utilisables comme marqueurs de composés organiques en vue de la détection de ces composés par chimiluminescence ou fluorescence.

(30) Priorité: **30.11.89 FR 8915789**

(43) Date de publication de la demande: **12.06.91 Bulletin 91/24**

(45) Mention de la délivrance du brevet: **16.08.95 Bulletin 95/33**

(84) Etats contractants désignés: **AT BE CH DE DK ES FR GB GR IT LI LU NL SE**

(56) Documents cités:
**WO-A-89/12052**

**ANALYTICA CHIMICA ACTA, vol. 223, 1989, pages 309-317, Elsevier Science Publishers B.V., Amsterdam, NL ; M. TOD et al. :"Chromatographic and luminescence properties of a 7-aminocoumarin derivative with peroxyoxalate chemiecitation"**

(73) Titulaire: **LABORATOIRES EUROBIO**
**20, boulevard St. Germain**
**F-75005 Paris (FR)**

(72) Inventeur: **Reveilleau, Pierre**
**7, av. Vavin**
**F-75006 Paris (FR)**
Inventeur: **Mahuzier, Georges**
**7, rue Morère**
**F-75014 Paris (FR)**
Inventeur: **Chalom, Joseph**
**17, rue de Colonel Oudot**
**F-75012 Paris (FR)**
Inventeur: **Farinotti, Robert**
**Faculté de Pharmacie,**
**Rue Jean-Baptiste Clément**
**F-92290 Chatenay-Malabry (FR)**
Inventeur: **Tod, Michel**
**2 Allée Georges Sand**
**F-95580 Margency (FR)**
Inventeur: **Barre, Edith**
**9 rue de Madagascar**
**F-75014 Paris (FR)**

(74) Mandataire: **Des Termes, Monique et al**
**c/o Société de Protection des Inventions**
**25, rue de Ponthieu**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 432 017 B1

Rank Xerox (UK) Business Services
(3.10/3.09/3.3.3)

## Description

La présente invention concerne de nouveaux marqueurs, utilisables pour le marquage de composés organiques en particulier de composés biologiques, comportant des groupements -SH, -NH-, $-NH_2$, ou -COO-, en vue de la détection de ces composés par chimiluminescence ou fluorescence dans des procédés de dosages tels que les dosages par chromatographie liquide, ou des techniques immunochimiques.

On sait que le dosage par chromatographie liquide d'amines, d'acides et de leurs anhydrides correspondants, dépourvus de propriétés spectrales nécessite, pour atteindre une grande sensibilité, de transformer ces composés à l'aide de réactifs marqueurs pour leur conférer des propriétés d'absorption, de fluorescence ou de chimiluminescence.

L'utilisation de marqueurs de ce type pour la détection de dérivés carboxyliques ou d'amines est décrite en particulier par H. Cisse, R. Farinotti, S. Kirkiacharian et A. Dauphin dans Journal of Chromatography, 225(1981), p. 509-515; par M. Tsitini Tsamis, A.M. Mange, R. Farinotti et G. Mahuzier dans Journal of Chromatography 277(1983), p. 61-69; par N. Kubab, R. Farinotti et G. Mahuzier dans Analusis, 1986, v.14, n°3, p. 125-130; et par D. Amir et E. Haas dans Int. J. Peptide Protein Res. 26(1986), p. 7-17.

Dans les procédés de dosage par chromatographie liquide, on recherche en particulier des marqueurs chimiluminescents car l'analyse par chimiluminescence est en plein développement et permet d'améliorer la sensibilité du dosage. En effet, les méthodes par chimiluminescence permettent d'atteindre une limite de détection de l'ordre de la femtomole, alors que, dans les méthodes par spectrofluorimétrie, la limite de détection atteinte est couramment de l'ordre de quelques centaines de femtomoles.

Une méthode de détection par chimiluminescence en chromatographie en phase liquide est décrite en particulier par M. Tod, R. Farinotti et G. Mahuzier dans "Analusis, 1986, v.14, n°6, p. 271-280".

Les marqueurs chimiluminescents actuellement utilisés tels que l'ortophtalaldéhyde, la fluorescamine et les dérivés anthracéniques et dansylés, présentent certains inconvénients. Ainsi l'orthophtalaldéhyde et la fluorescamine sont peu sensibles. Les dérivés anthracéniques sont inhibés par l'oxygène dissous et les dérivés dansylés sont inhibés par certains produits de la réaction d'excitation. De plus, certains de ces marqueurs chimiluminescents présentent une toxicité non négligeable, c'est le cas en particulier des dérivés anthracéniques et des dérivés dansylés.

A la suite de recherches récentes, on a découvert que des noyaux coumariniques présentaient un certain intérêt car ce sont des composés ayant une sensibilité équivalente aux produits les plus performants, qui ne présentent ni toxicité, ni sensibilité vis-à-vis des inhibiteurs usuels tels que l'oxygène dissous, les halogénures et les dérivés nitrés. Par ailleurs, ils sont faciles à mettre en oeuvre dans des conditions d'excitation et de chromatographie variées.

Ainsi, M. Tod et al ont décrit dans Anal. Chem. Acta, 223, (1989), p. 309-317, un nouveau dérivé de 7-amino coumarine, la Luminarine-1, utilisable comme marqueur des amines primaires et secondaires, qui répond à la formule :

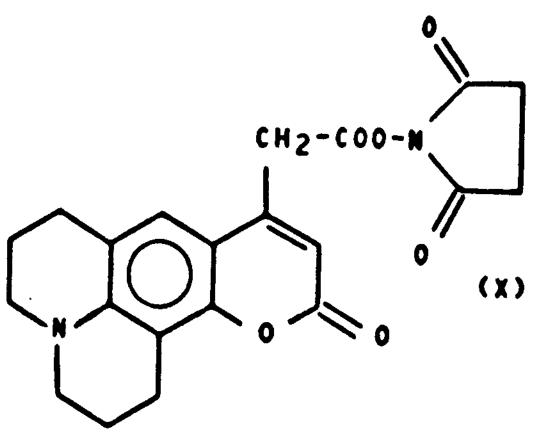

(X)

Le document WO-A- 89/12052 qui a été publié le 14 décembre 1989, décrit également la luminarine-1 ainsi que des analogues de celle-ci comportant une chaîne hydrocarbonée entre le groupe succinimidoxycarbonyle terminal et le noyau coumarine.

Bien que les propriétés de la Luminarine-1 soient très intéressantes, les recherches ont été poursuivies pour trouver notamment d'autres dérivés qui, d'une part, aient une meilleure hydrosolubilité que la

Luminarine-1 et, d'autre part, soient plus adaptés au marquage de molécules biologiques telles que les anticorps et les sondes oligonucléotidiques et autres ligands d'affinité.

La présente invention a précisément pour objet de nouveaux dérivés coumariniques qui sont des dérivés de tétrahydro-2,3,6,7,1H,5H,11H-(1)benzopyrano(6,7,8.ij)quinolizinone-11 présentant ces propriétés améliorées.

Selon l'invention, les dérivés de tétrahydro-2,3,6,7,1H,5H,11H-(1) benzopyrano (6,7,8,ij) quinolizinone-11 répondant à la formule :

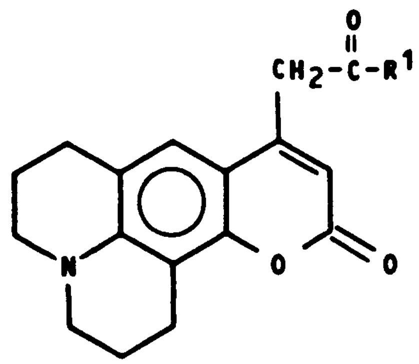

(I)

dans laquelle $R^1$ représente :

1°) $NH-(CH_2)_n-R^2$

avec n étant un nombre entier allant de 1 à 20 et $R^2$ représentant $-N=C=S$ ou $NH-CO-CH_2X$ avec X représentant I, Br ou Cl, ou

2°) $NH-(CH_2-CH_2-O)_m-CH_2-CH_2-R^3$

avec m un nombre entier allant de 1 à 30 et $R^3$ représentant un groupe choisi parmi les groupes de formule

$-NH_2$

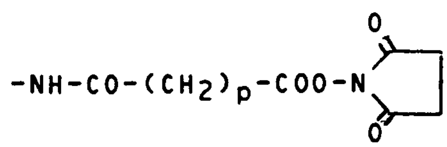

$-NH-CO-(CH_2)_p-CO-NH-NH_2$,

$-N=C=S$, et

$-NH-CO-CH_2X$

dans lesquelles p est un nombre entier allant de 1 à 10 et X représente Br, Cl ou I.

Dans ces nouveaux dérivés, l'utilisation du noyau coumarinique de formule :

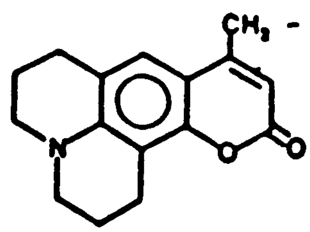

permet d'obtenir des propriétés de fluorescence et de chimiluminescence améliorées; par ailleurs, le choix d'un groupement $-CO-R^1$ approprié fixé sur ce noyau coumarinique, permet d'obtenir des marqueurs susceptibles d'être utilisés pour un grand nombre de composés organiques et notamment de substances biologiques.

Ainsi, les nouveaux dérivés de l'invention ont l'avantage d'avoir une bonne sensibilité, de pouvoir être utilisés comme marqueurs pour différents composés, d'être utilisables en phase normale ou inversée, de ne pas être inhibés par les quenchers usuels tels que l'oxygène dissous, les halogénures et les nitrates, de ne

pas présenter de toxicité et de réagir rapidement avec les composés à marquer dans des conditions relativement douces.

Selon un premier mode de réalisation de l'invention, les dérivés de tétrahydro-2,3,6,7,1-H,5H,11H-(1) benzopyrano (6,7,8,ij) quinolizinone-11 répondent à la formule:

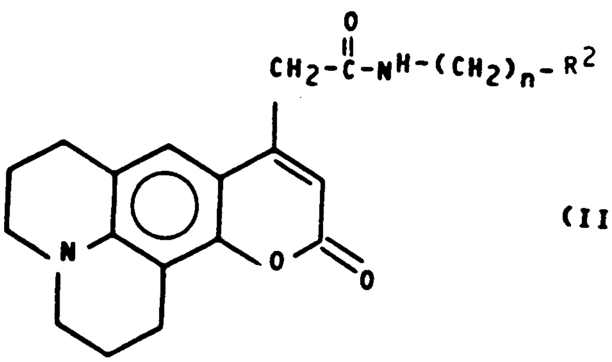

(II)

dans laquelle n est un nombre entier allant de 1 à 20 et $R^2$ est N=C=S ou $NH\text{-}COCH_2X$ avec X représentant I, Br ou Cl.

De préférence dans ce premier mode de réalisation n est un nombre allant de 1 à 12.

A titre d'exemple de dérivés correspondant à ce premier mode de réalisation de l'invention, on peut citer ceux pour lesquels n est égal à 4 et $R^2$ représente $NHCOCH_2I$ ou N = C = S.

Les dérivés correspondant à ce premier mode de réalisation de l'invention qui comportent un groupement terminal halogéno acétamido $NH\text{-}CO\text{-}CH_2X$, par exemple iodo-acétamido, sont très intéressants car ils sont susceptibles de marquer des groupements sulfhydryle (ou thiol), soit dans de petites molécules telles que des médicaments comme le Captopril, la cystéine, et la cystéamine, soit dans des protéines possédant le même groupement.

En effet, ils peuvent réagir avec des composés comportant ce groupement thiol. Le dérivé formé par cette réaction peut être séparé, par exemple par chromatographie en phase liquide et détecté par absorptiométrie, par fluorimétrie ou par chimiluminescence.

Dans ce premier mode de réalisation, les dérivés dans lesquels $R^2$ est -N=C=S sont également très intéressants. En effet, ces molécules sont susceptibles de réagir avec des amines primaires et peuvent être utilisées en particulier pour marquer des protéines et notamment des anticorps.

A titre d'exemple, lorsqu'on fait réagir un de ces dérivés avec la pentylamine, on obtient le dérivé correspondant dans des conditions particulièrement douces.

Selon un second mode de réalisation de l'invention, les dérivés de tétrahydro-2,3,6,7,1-H,5H,11H-(1) benzopyrano (6,7,8,ij) quinolizinone-11, répondent à la formule:

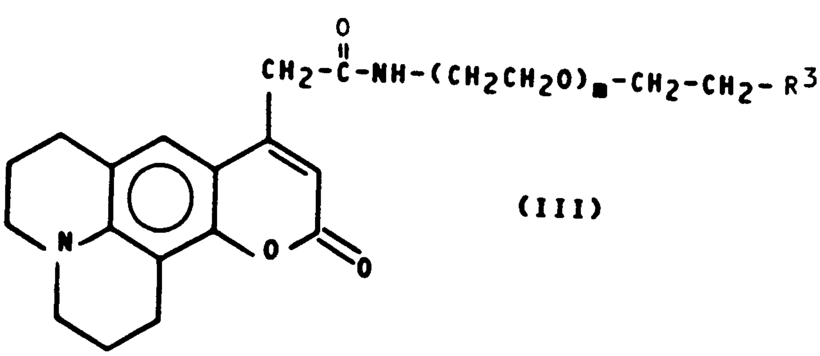

(III)

dans laquelle m est un nombre entier allant de 1 à 30 et $R^3$ est tel que défini ci-dessus.

De préférence m est un nombre allant de 1 à 6.

A titre d'exemple de tels dérivés, on peut citer ceux pour lesquels m est égal à 5.

Les dérivés correspondant à ce second mode de réalisation de l'invention comportent dans leur chaîne latérale une partie poly (oxy 1,2 éthanediyl) s'apparentant au polyéthylène glycol. Cette partie confère une hydrosolubilité aux dérivés qui restent, de plus, solubles dans la plupart des solvants organiques.

Cette propriété d'hydrosolubilité a de grands avantages dans le marquage des molécules biologiques (protéines ou ADN): elle supprime la présence, à proximité immédiate de la molécule biologique, d'un groupement hydrophobe tel que le noyau principal quinolizinone, ou une chaîne latérale à 4 carbones comme dans le cas des dérivés correspondant au premier mode de réalisation de l'invention. Cet avantage est encore renforcé par la longueur du bras poly (oxo 1,2 éthanediyl), qui peut toujours être adaptée au besoin précis.

Aussi, ces dérivés peuvent avoir des applications encore plus adaptées que les précédents, en particulier en immunologie (marquage d'anticorps) ou en biologie moléculaire (sondes ADN).

De plus, la présence du bras oxo-1,2 éthanediyl favorise la formation de liaisons hydrogène. Cet effet est bénéfique notamment au niveau de liaisons antigène-anticorps ou en hybridation moléculaire (sondes nucléiques).

Dans ce second mode de réalisation, la réactivité de chaque dérivé est déterminée par la nature du groupement $R^3$.

Ainsi, lorsque $R^3$ représente $NH_2$, les dérivés peuvent réagir avec les composés et substances biologiques comportant une fonction carboxylique (en présence d'un agent de couplage), leurs anhydrides et leurs esters, en particulier avec tous les acides sous forme activée tels que les esters d'hydroxysuccinimide, par exemple avec les plus intéressants en immunologie comme la NHS biotine de formule:

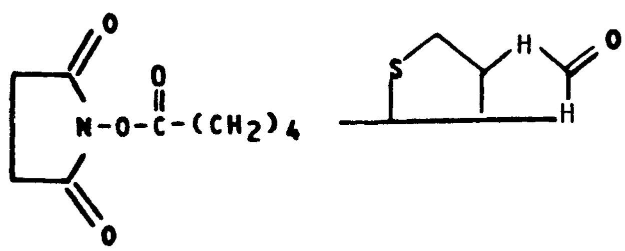

ou la NHS-LC-biotine.

Ceci est très intéressant car la biotine est extrêmement utilisée en biologie (immunologie, etc.) en mettant à profit sa forte affinité ($10^{15} M^{-1}$) pour l'avidine qui est une glycoprotéine tirée du blanc d'oeuf ayant une masse de 66000, et composée de quatre sous-unités identiques qui sont capables de fixer chacune une molécule de biotine.

Ainsi, on peut utiliser les dérivés de formule (III) avec $R^3$ représentant $NH_2$ pour détecter un composé comportant un groupement -COO-.

Dans ce cas, on fait réagir le dérivé de formule (III) avec le composé à détecter, pour former un dérivé du composé à détecter, que l'on détecte par absorptiométrie, fluorimétrie ou chimiluminescence, en utilisant la chromatographie en phase liquide, ou des méthodes biochimiques mettant ou non en jeu des ligands spécifiques.

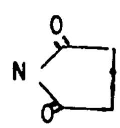

Lorsque R3 représente $-NH-CO-(CH_2)_p-CO-O-$où p est un nombre entier allant de 1 à 10, de préférence de 1 à 3, par exemple 2, les dérivés peuvent réagir avec des amines primaires et secondaires en milieu aqueux ou non aqueux ; ceci est particulièrement intéressant pour le marquage de fonctions amines de substances biologiques telles que les anticorps, ou les sondes ADN modifiées possédant une fonction amine primaire.

Lorsque $R_3$ représente $-NH-CO-(CH_2)_p$ - $CO-NH-NH_2$, les dérivés sont susceptibles de réagir avec des aldéhydes (et en particulier avec les groupements aldéhyde obtenus par oxydation périodique des glycoprotéines). Ils peuvent aussi réagir sur des chaînes d'ADN par transamination de groupements cytosine.

Lorsque $R_3$ représente $N=C=S$ ou $-NH-CO-CH_2X$, les dérivés possèdent la même réactivité que les dérivés du premier mode de réalisation de l'invention pour lesquels $R^2$ est $-N=C=S$ ou $-NH-CO-CH_2X$.

Le dérivé formé après réaction avec l'amine primaire ou secondaire, peut être détecté par absorptiométrie, fluorimétrie ou chimiluminescence, en utilisant la chromatographie en phase liquide, ou des méthodes

biochimiques mettant ou non en jeu des ligands spécifiques.

Dans ce dernier cas qui correspond à la sensibilité maximale, la réaction d'excitation peut utiliser un ester oxalique et du peroxyde d'hydrogène qui forment le peroxyoxalate, intermédiaire réactionnel à haute énergie, qui transfère son énergie au dérivé formé qui se désexcite en émettant de la lumière. Ce dosage par chimiluminescence peut être appliqué à des molécules d'intérêt biochimique ou pharmacologique en vue d'études de métabolisme ou de pharmacocinétique, notamment aux dosages des acides aminés, des amines biogènes du type histamine, des neuromédiateurs du type catécholamine, des polypeptides hormonaux et des métabolites des phénothiazines, des imipraminiques etc.

Dans le cas des applications en biologie, les dérivés peuvent réagir sur des substances biologiques aminées, par exemple anticorps, oligonucléotide modifié par un groupement aminé, ou autre ligand d'affinité portant un groupement aminé. Les produits marqués ainsi formés peuvent réagir en phase homogène (liquide), ou en phase solide-liquide, sur un support approprié, sur un ligand complémentaire.

Les nouveaux dérivés coumariniques de l'invention peuvent être préparés par des procédés classiques.

Ainsi, on peut préparer les dérivés de formule (II) pour lesquels $R^2$ représente $NHCOCH_2X$, par un procédé consistant à faire réagir un dérivé de tétrahydro-2,3,6,7,1H,5H,11H-(1) benzopyrano (6,7,8,ij) quinolizinone-11 de formule

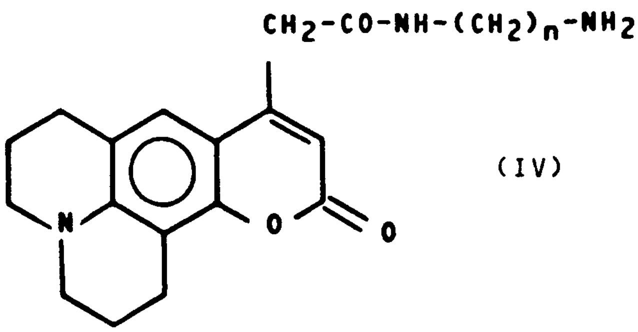

(IV)

avec un anhydride halogéno acétique, en présence d'une trialkyl amine telle que la triéthylamine.

Ceci correspond à la réaction suivante:

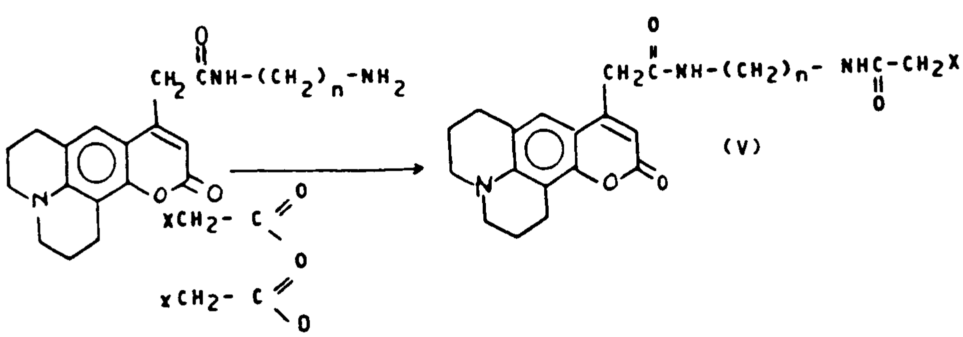

(V)

Le dérivé de formule (IV) peut être préparé par un procédé comprenant les étapes successives suivantes:

1°) faire réagir la 8-hydroxyjulolidine de formule:

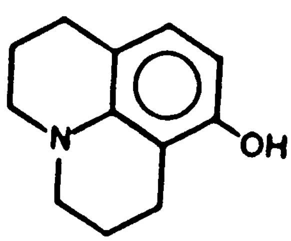

(VI)

avec un ester alkylique de l'acide oxo-3-glutarique de formule:

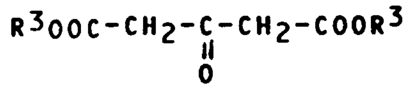

dans laquelle $R^3$ est un radical alkyle, pour former un composé de formule:

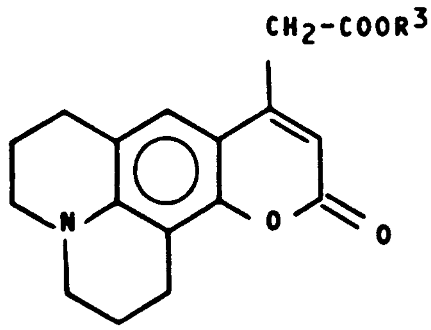

(VII)

2°) hydrolyser l'ester alkylique de formule (VII) obtenu précédemment pour obtenir l'acide de formule:

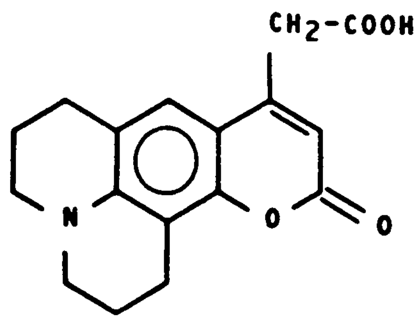

(VIII)

3°) faire réagir l'acide de formule (VIII) ainsi obtenu avec l'oxalate de dihydroxysuccinimide de formule:

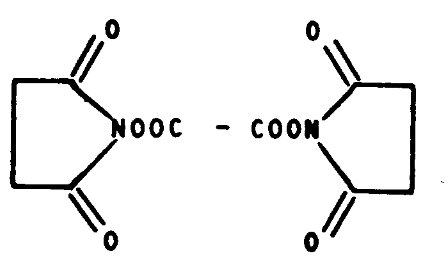

(IX)

ou l'hydroxysuccinimide en présence d'un agent de couplage pour former le dérivé de formule:

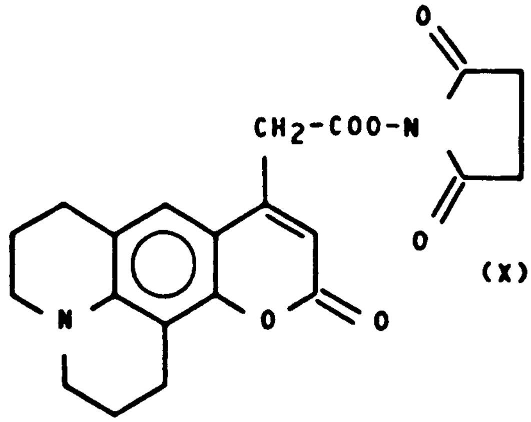

(X)

4°) faire réagir le dérivé de formule (X) avec un diamino alcane de formule:

$H_2N-(CH_2)_n-NH_2$ (XI)

dans laquelle n a la signification donnée ci-dessus.

Dans ce dernier procédé, la première étape peut être effectuée en portant au reflux sous agitation la 8-hydroxyjulolidine avec l'ester alkylique de l'acide oxo-3-glutarique, en présence de chlorure de zinc anhydre, et en solution alcoolique. Le composé de formule (VII) obtenu peut être séparé par extraction au moyen d'un solvant organique tel que l'acétate d'éthyle.

L'ester alkylique de l'acide oxo-3-glutarique est de préférence l'ester méthylique ou éthylique.

Dans la deuxième étape du procédé, on peut réaliser l'hydrolyse de l'ester alkylique de formule (VII) par une solution aqueuse de soude dans une solution alcoolique constituée par exemple de méthanol.

La troisième étape du procédé peut être réalisée par exemple en faisant réagir l'acide de formule (VIII) avec l'oxalate de dihydroxysuccinimide dans un solvant organique anhydre, en présence de triéthylamine anhydre. Dans ces conditions, on peut obtenir le dérivé coumarinique de formule (X) avec un degré de pureté élevé, d'environ 90%.

Les dérivés coumariniques répondant à la formule (XII)

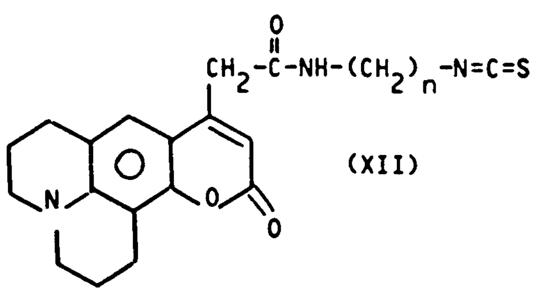

(XII)

peuvent être préparés par action du thiocarbonyl diimidazole sur le dérivé de formule (IV) décrit ci-dessus.

Selon le schéma réactionnel suivant:

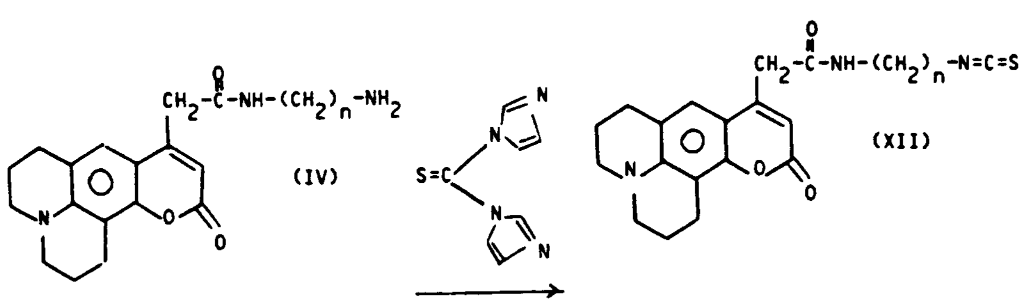

(IV)

(XII)

Les dérivés coumariniques répondant à la formule:

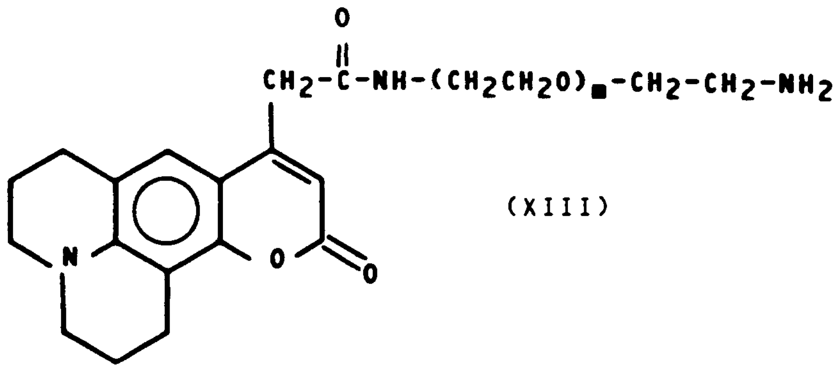

(XIII)

dans laquelle m est un nombre entier allant de 1 à 30, peuvent être préparés par un procédé consistant à faire réagir le composé de formule:

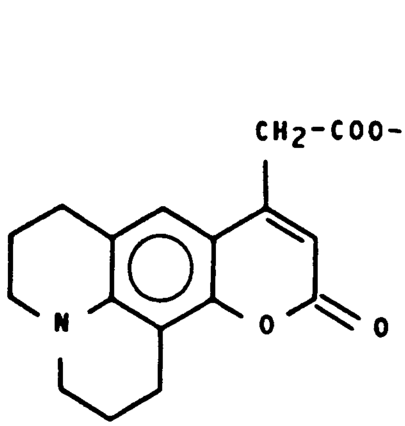

(X)

avec un excès de poly(oxy-1,2 éthane diyl) diamine de formule:

$H_2N\text{-}(CH_2CH_2O)_m\text{-}CH_2\text{-}CH_2NH_2$ (XIV)

dans laquelle m a la signification donnée ci-dessus.

Les dérivés coumariniques répondant à la formule:

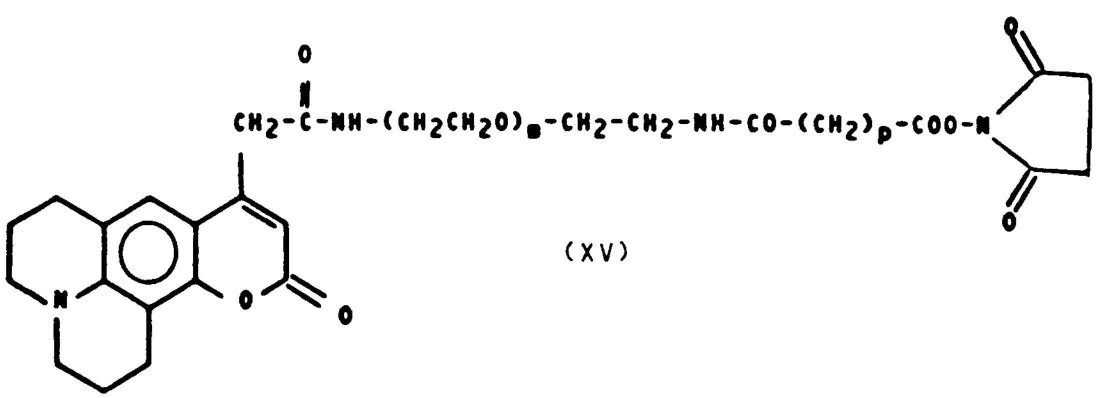

(XV)

dans laquelle m est un nombre entier allant de 1 à 30 et p est un nombre entier allant de 1 à 10, peuvent être préparés par un procédé comprenant les étapes successives suivantes:

1°) faire réagir un composé de formule:

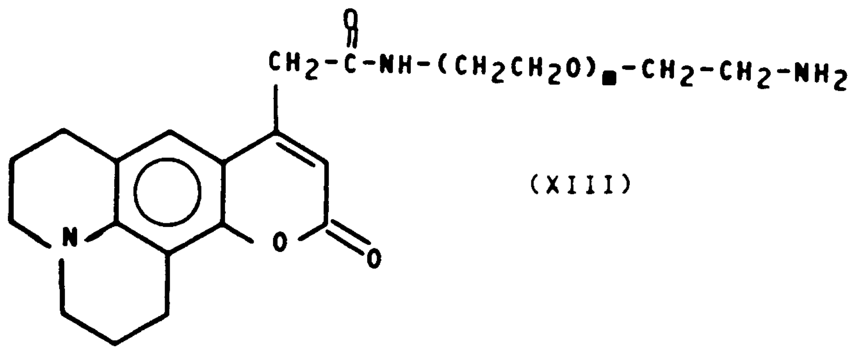

(XIII)

dans laquelle m a la signification donnée ci-dessus avec un anhydride de diacide de formule:

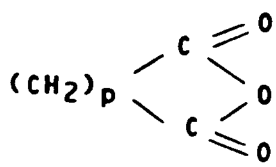

(XVI)

pour obtenir un acide de formule:

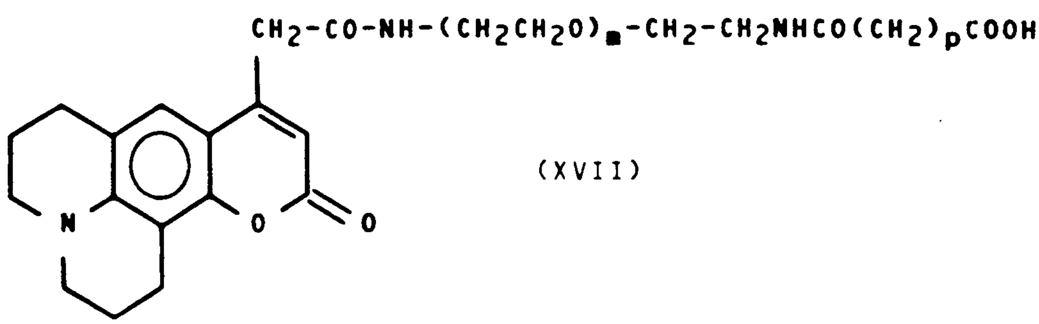

(XVII)

2°) faire réagir ensuite l'acide de formule (XVII) avec l'hydroxysuccinimide en présence d'un agent de couplage pour former le dérivé de formule (XV).

L'agent de couplage peut être par exemple le dicyclohexylcarbodiimide.

Les dérivés de formule (XV) notamment ceux pour lesquels p = 1 peuvent aussi être préparés par réaction du dérivé de formule (XIII) avec le monoester benzylique de l'acide dicarboxylique de formule:

$HOOC\text{-}(CH_2)_p\text{-}COOCH_2C_6H_5$

suivie d'une débenzylation par hydrogénation pour obtenir l'acide de formule (XVII) que l'on fait réagir ensuite comme précédemment avec l'hydroxysuccinimide.

La préparation de l'acide (XVII) correspond au schéma suivant:

$(XIII) + HOOC - (CH_2)_p - COOCH_2C_6H_5$ →

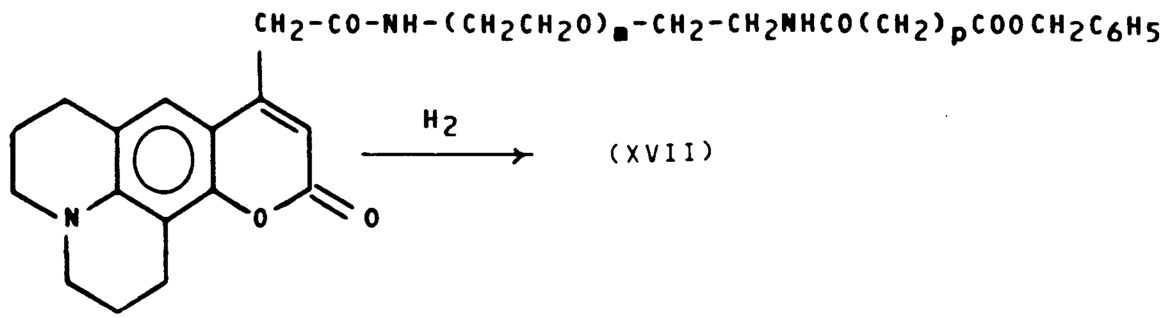


(XVII)

Les dérivés coumariniques répondant à la formule:

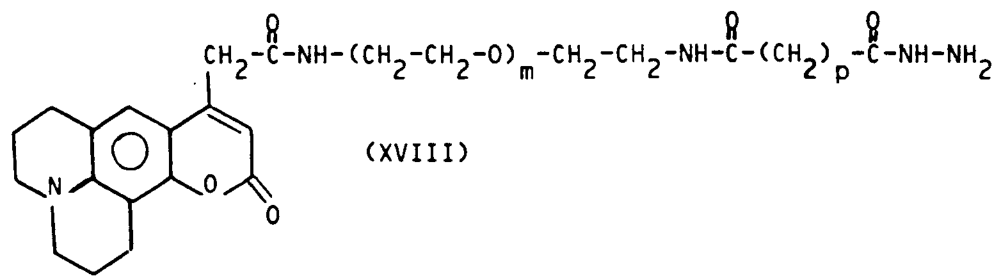

(XVIII)

peuvent être par exemple préparés, par action d'un excès d'hydrate d'hydrazine, par exemple en phase DMF/eau, sur des composés de formule (XV) selon le schéma suivant:

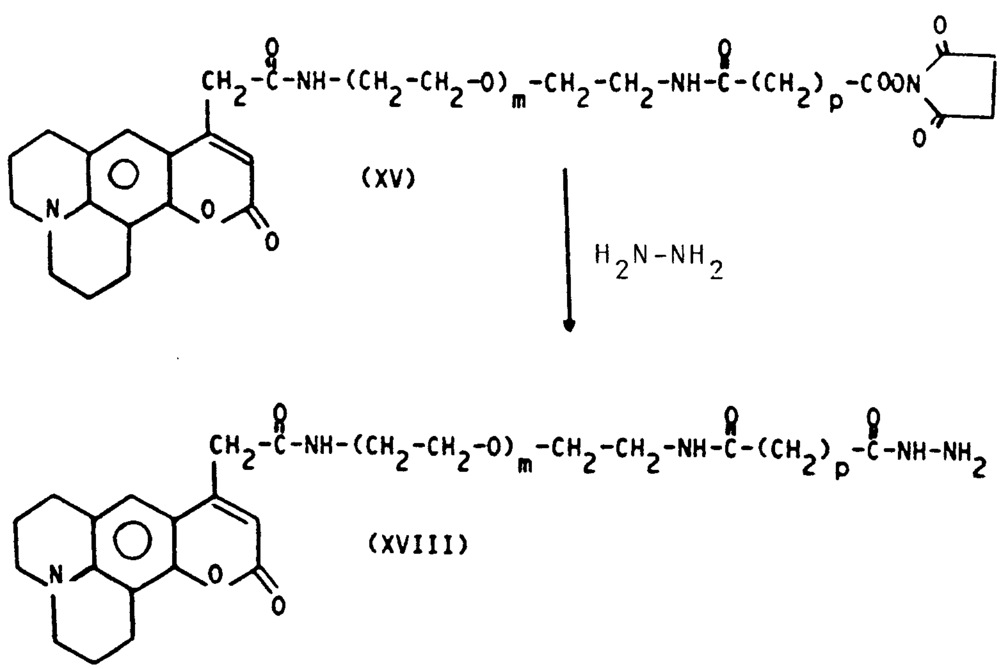


(XV)

(XVIII)

Les dérivés coumariniques répondant à la formule:

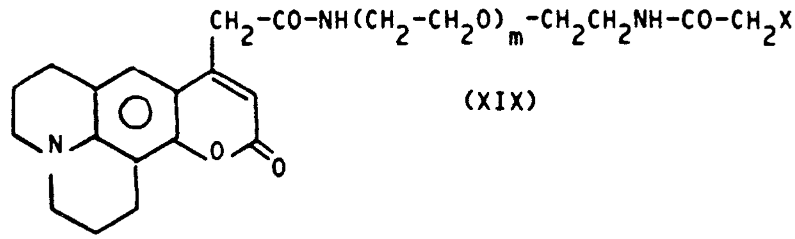

(XIX)

peuvent être préparés à partir des dérivés de formule (XIII) par réaction de ceux-ci avec un anhydride halogénoacétique, en présence d'une trialkylamine telle que la triéthylamine. Ceci correspond au schéma réactionnel suivant:

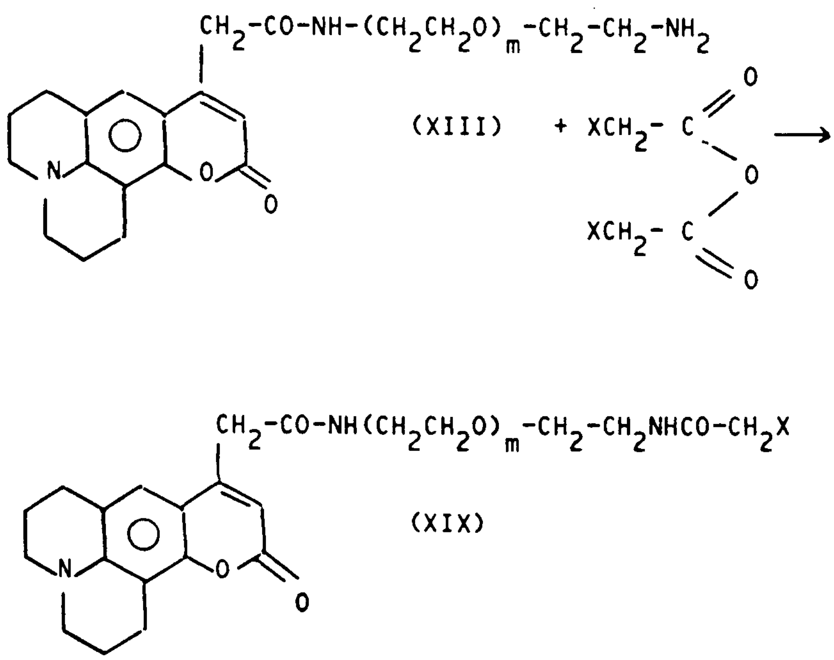

(XIII)

(XIX)

Les dérivés coumariniques répondant à la formule:

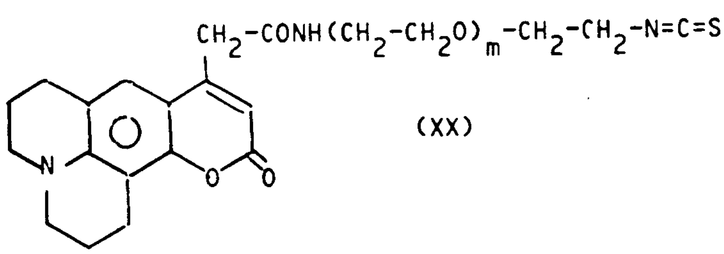

(XX)

peuvent être préparés à partir des dérivés de formule (XIII) par réaction avec du thiocarbonyldiimidazole selon le schéma réactionnel suivant:

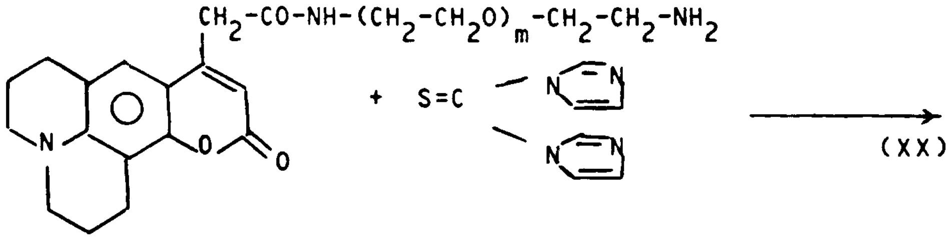

(XX)

Les produits de départ utilisés dans ces différents modes de préparation des dérivés coumariniques de l'invention sont des produits du commerce ou peuvent être préparés par des procédés classiques à partir de produits du commerce.

Comme on l'a indiqué précédemment, les dérivés coumariniques de l'invention peuvent réagir avec différents composés organiques, par exemple des amines, des thiols, des acides carboxyliques, leurs anhydrides ou leurs esters.

Aussi, ces dérivés coumariniques peuvent être utilisés comme marqueurs de composés organiques comportant des fonctions amine primaire ou secondaire, thiol, ou acide carboxylique, et servir à la détection de ces composés dans des méthodes de dosage, par exemple par chromatographie en phase liquide ou dans des méthodes immunochimiques mettant ou non en jeu des ligands spécifiques. La détection peut être effectuée par absorptiométrie, fluorimétrie ou chimiluminescence, en utilisant les méthodes classiques telles que celles décrites par M.Tsitini Tsamis et al dans Journal of chromatography, 277, (1983), 61-69; par D. Amir et E. Haas dans Int. J. Peptide Protein Res. 26, 1986, p.7-17; par H. Cisse et al dans Journal of Chromatography, 225, (1981), p. 509-515 et par M. Tod et al dans Analusis, 1986, v14, n°6 p. 271-280.

Aussi, l'invention a également pour objet un procédé de détection d'un composé comportant une fonction amine primaire ou secondaire, qui consiste à faire réagir le composé à détecter avec un dérivé répondant à la formule:

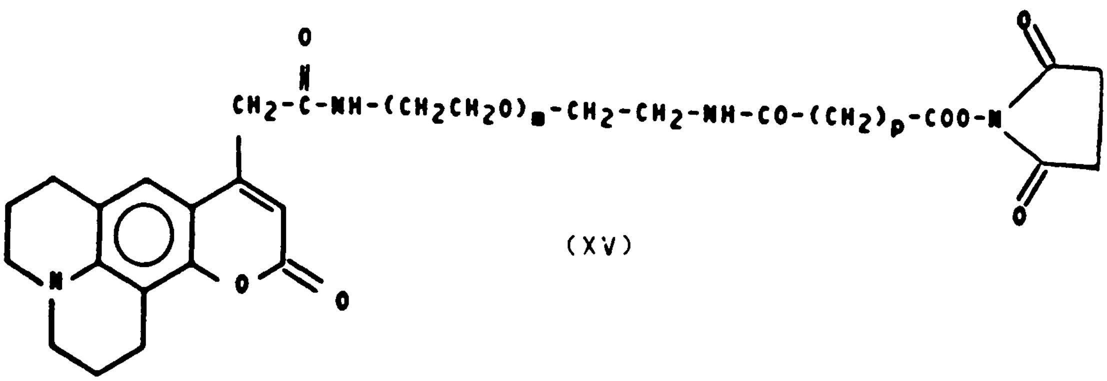

(XV)

dans laquelle m est un nombre entier de 1 à 30 et p est un nombre entier allant de 1 à 10, pour former un dérivé du composé à détecter, à réaliser ensuite une séparation de ce dérivé et à détecter ensuite ce dérivé par absorptiométrie, fluorimétrie, ou chimiluminescence.

Dans ce procédé de détection de composés comportant une fonction amine, on peut aussi utiliser les dérivés de formule (XII) ou (XX).

On peut détecter par le même procédé un composé comportant un groupe SH, ou un groupe -COO- en utilisant respectivement les dérivés de formule (V) ou (XIX) ou de formule (XIII).

Dans tous les cas, on réalise de préférence la détection par chimiluminescence en utilisant un ester oxalique et du peroxyde d'hydrogène.

A titre d'exemple, l'ester oxalique peut être le bis-(trichloro-2,4,6 phényl)oxalate (TCPO) ou le bis-(dinitro-2,4 phényl)oxalate (DNPO).

L'invention sera mieux comprise à la lecture des exemples suivants, donnés bien entendu à titre illustratif et non limitatif, en référence au dessin annexé sur lequel:

- la figure 1 représente le spectre de masse de la Luminarine-6,

- la figure 2 représente le spectre de masse du dérivé obtenu par réaction de l'anhydride succinique avec la Luminarine-6,
- la figure 3 représente le spectre de masse de la Luminarine-7,
- la figure 4 représente le spectre de masse de la Luminarine-8,
- la figure 5 représente le spectre de masse de la Luminarine-9, et
- la figure 6 représente le spectre de masse de la Luminarine-9 après réaction avec la pentylamine.

**Exemple 1**:Synthèse de la Luminarine-5 répondant à la formule:

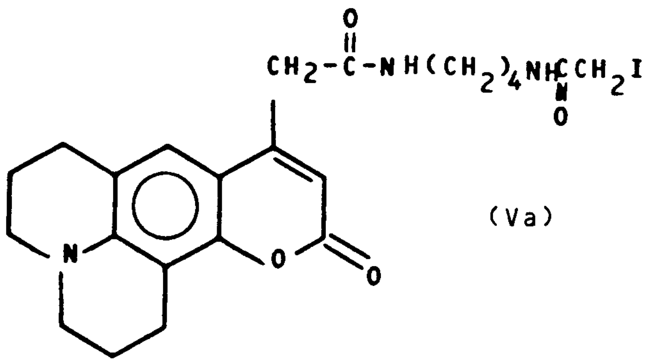

(Va)

Ce dérivé répond à la formule (V) avec n = 4 et X = I.

1° Préparation du dérivé de formule (IV) avec n = 4 (IVa)

a) préparation du composé de formule (VII) avec $R^3$ représentant le radical éthyle. (VIIa)

La réaction effectuée ici correspond au schéma réactionnel suivant:

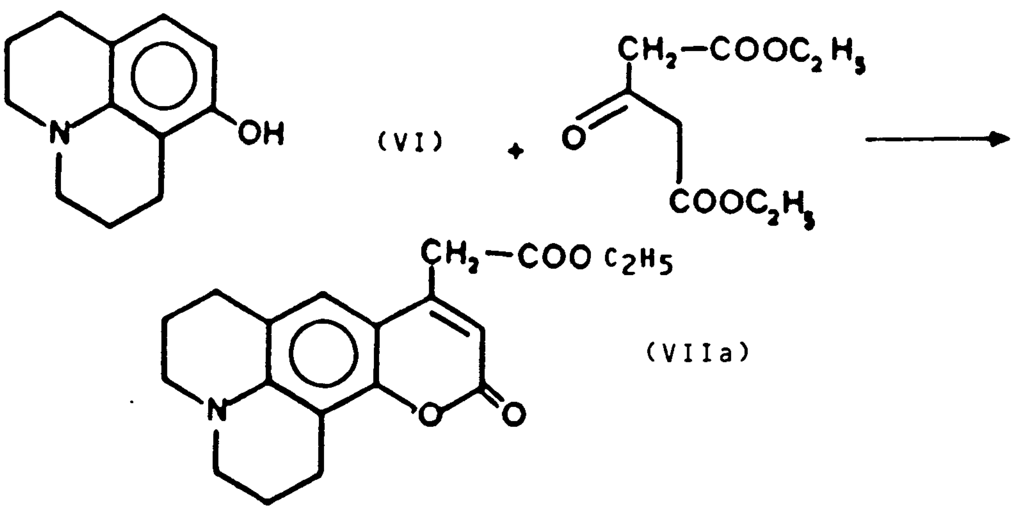

On introduit dans un récipient 2,12 g de 8-hydroxyjulolidine, 2,22 g d'ester éthylique de l'acide oxo-3-glutarique, 1,71 g de chlorure de zinc anhydre et 6 ml d'éthanol anhydre, et on porte au reflux pendant 24 h, sous agitation à l'abri de l'humidité. Après refroidissement, on introduit la solution dans 200 ml d'eau, puis on extrait par 200 ml, puis 100 ml d'acétate d'éthyle. On lave la phase organique à l'eau, on la sèche sur sulfate de magnésium et on la concentre à sec. On recristallise ensuite dans 5 parties d'acétate d'éthyle. On obtient ainsi l'ester éthylique de formule (VIIa) avec un rendement de 56%.

b) Hydrolyse de l'ester éthylique de formule (VIIa)obtenu précédemment.

On introduit dans un récipient 2 g de l'ester éthylique obtenu précédemment avec 42 ml d'une solution aqueuse de NaOH à 1,2% P/V et 40 ml de méthanol, puis on porte à 45 °C pendant une heure. Après refroidissement, on extrait le milieu réactionnel par 50 ml, puis 40 ml de chloroforme. Après dégazage, on acidifie la phase aqueuse avec 16 ml d'acide chlorhydrique 3N, puis on maintient le mélange réactionnel sous agitation pendant 15 min. On ajuste alors le pH à 6,5 avec 13 ml de soude 2,5N. On filtre alors le précipité formé, on le rince à l'eau et on le sèche. On obtient ainsi l'acide de formule (VIII) avec un rendement de 90%.

c) Obtention du dérivé de formule (X) Luminarine-1.

Dans cette troisième étape, on fait réagir l'acide de formule (VIII) obtenu dans l'étape précédente avec l'oxalate de dihydroxysuccinimide selon le schéma réactionnel suivant:

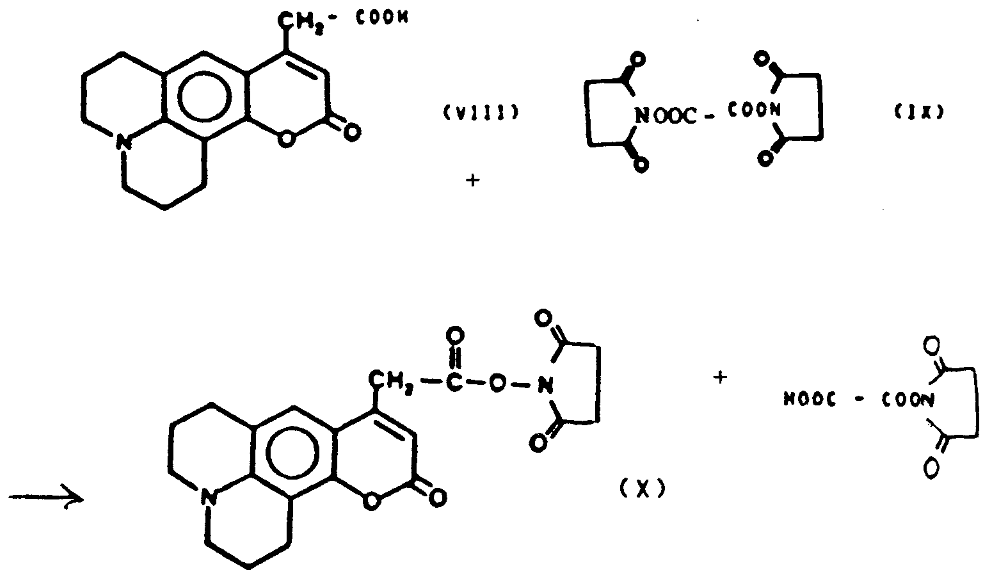
(VIII)
+
(IX)
(X)
+

On introduit dans un récipient 11,26 g (0,0377 mol) de l'acide de formule (VIII), 10,62 g (0,0415 mol) d'oxalate dihydroxysuccinimide, 3,81 g (0,0377 mol) de triéthylamine anhydre et 560 ml d'acétonitrile anhydre. On maintient le mélange sous agitation, à l'abri de l'humidité, pendant une heure à la température ambiante, puis pendant une heure à une température de 35-40 °C. On filtre alors un léger insoluble, puis on concentre sous vide et on purifie par chromatographie sur gel de silice en utilisant comme solvant d'élution le mélange dichlorométhane/tétrahydrofuranne (THF) avec un rapport en volume de 1/1. On concentre les fractions 11 à 14, et l'on obtient ainsi le dérivé de formule (X), Luminarine-1, avec un rendement de 21%.

d) Obtention ou dérivé de formule (IVa) avec n = 4.

On part directement du dérivé de formule (X), Luminarine-1 purifiée que l'on fait réagir sur le diamino-1,4 butane selon le schéma réactionnel suivant:

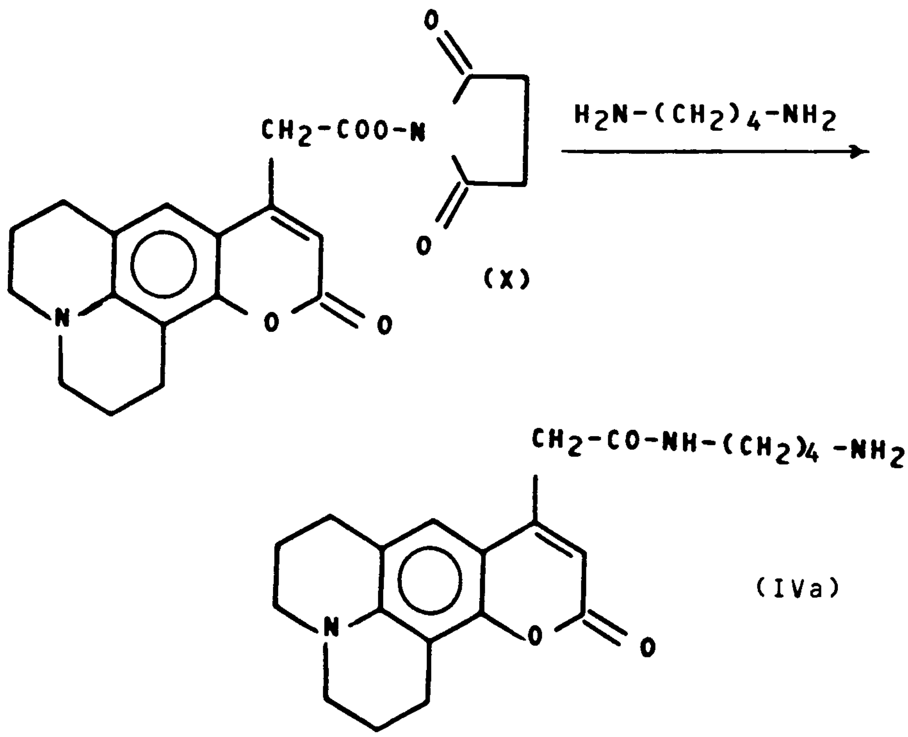


(X)

(IVa)

Dans un ballon d'un litre, on fait réagir 16,5 g de Luminarine-1 (X) (41,6 mmoles) et 18,3 g de 1,4-diaminobutane (107 mmoles) dans 400 ml de THF anhydre, sous agitation durant 24 heures.

On filtre un insoluble marron.

On concentre à sec les eaux mères; on reprend le résidu dans 100 ml de dichlorométhane et on extrait par 5 x 100 ml d'eau pour éliminer l'excès de 1-4 diaminobutane.

On concentre le solvant à sec.

Le résidu est mis sous agitation dans 25 ml de dichlorométhane.

Rendement: 10,6 g (69%).

On vérifie par spectrométrie de masse et RMN la structure du composé obtenu.

2°) <u>Préparation de la Luminarine-5, dérivé de formule (Va).</u>

On part du dérivé (IVa) que l'on fait réagir avec l'anhydride iodoacétique en présence de triéthylamine selon le schéma suivant:

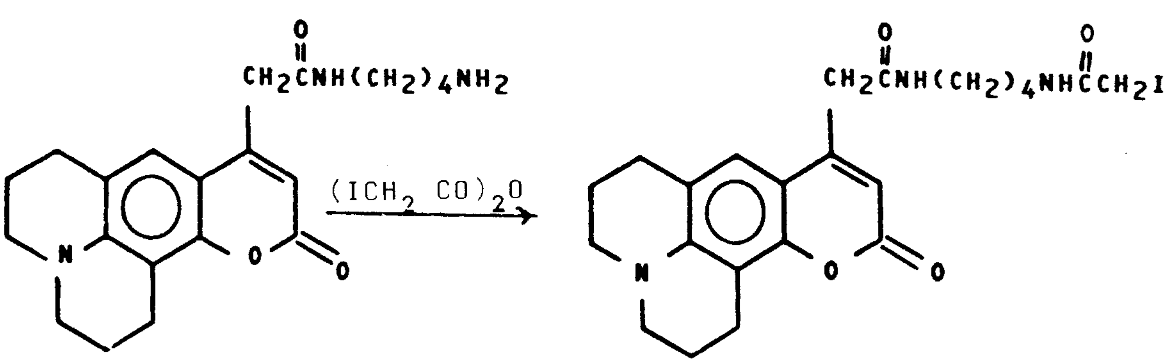


(IVa) (Va)

On dissout dans 1ml de diméthylformamide, 158 mg du dérivé (IVa)(0,4 mmol), 212,4 mg d'anhydride iodoacétique et 84 µl (0,6 mmol) de triéthylamine. On laisse réagir 2 heures à température ambiante, à

l'abri de la lumière. On précipite par ajout de 4ml d'eau distillée, puis on essore sous vide. On sèche sur anhydride phosphorique. On obtient ainsi la Luminarine-5 avec un rendement de 76%.

- Analyse élémentaire:
  I calculé = 23,6%
  I trouvé = 22,96%
- C.C.M.
  (chromatographie sur couche mince) Silice fluorescente
  Système BuOH/AcOH/$H_2O$ 70/20/10
  1 spot fluorescent (Rf = 0,75).

**Exemple 2** : Utilisation de la Luminarine-5 pour le marquage de la fonction thiol de la cystéamine.

On fait réagir dans un tampon borate à pH 8 (1 ml), 5,3 mg de Luminarine-5 ($1.10^{-2}$ mmol) et 7,7 mg de cystéamine (0,1 mmol); après une heure de réaction, on observe en C.C.M. la disparition totale de la Luminarine-5 et la formation du dérivé fluorescent (Rf = 0,4) du thiol qui a réagi suivant la réaction:

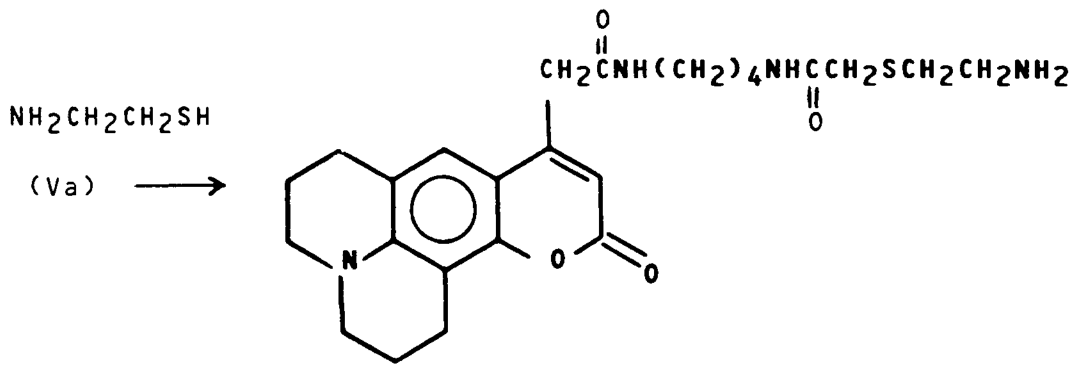


**Exemple 3 :** Synthèse de la Luminarine-6 de formule:

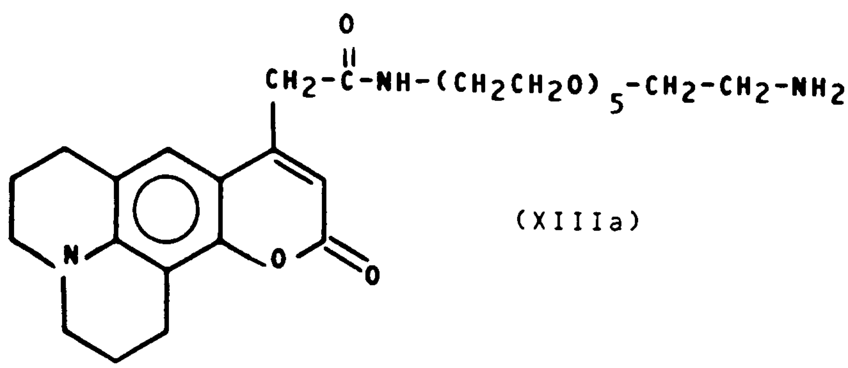

(XIIIa)

Ce dérivé répond à la formule (XIII) avec m = 5.

On fait réagir la Luminarine-1, dérivé (X), obtenue dans l'étape c) de l'exemple 1 avec le 1,17-diamino-3,6,9,12,15-pentaoxaheptadécane (XIVa), dans le dichlorométhane:

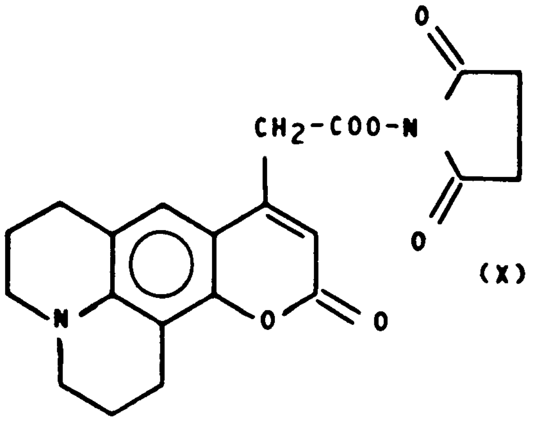

(X)

$+ 2HN-(CH_2CH_2O)_5CH_2CH_2NH_2 \longrightarrow$

(XIVa)

(XIIIa)

Dans un tube bouché à l'abri de la lumière, on met sous agitation durant 24 heures à la température ambiante 139 mg (0,35 mmol) de Luminarine-1 (X) et 392 mg (1,4 mmol) de 1,17 diamino 3,6,9,12,15 pentaoxaheptadécane (XIVa) dans 2 ml de dichlorométhane.

On évapore le dichlorométhane et on reprend par 10 ml d'eau, ce qui élimine une impureté non hydrosoluble; on extrait par 10 ml de dichlorométhane (le produit (XIIIa) passe dans le dichlorométhane et l'excès d'amine reste dans la phase aqueuse).

On sèche, puis on évapore la phase organique, on obtient ainsi la Luminarine-6 (XIIIa) sous la forme d'une huile jaune avec un rendement de 53%.

- C.C.M.:
  silice fluorescente
  système BuOH/AcOH/$H_2O$ 70/20/10:
  1 spot fluorescent et révélé
  à la ninhydrine (Rf = 0,35)
- Spectrométrie de masse (ionisation chimique à l'ammoniaque), appareil NERMAG: on retrouve bien le pic moléculaire +1 à 562 sur la figure 1 qui représente le spectre obtenu.

**Exemple 4 :** Utilisation de la Luminarine-6 pour le marquaqe de la biotine.

La biotine ou vitamine H est extrêmement utilisée en biologie (immunologie, etc....), en mettant à profit ses propriétés de très forte affinité ($10^{15}$ $M^{-1}$) pour une protéine tirée, en particulier, du blanc d'oeuf, l'avidine, ou une autre tirée du "streptomyces avidinii", la streptavidine. Dans les méthodes de diagnostic biologique, on peut par exemple marquer soit l'avidine, soit la biotine par un marqueur fluorescent ou chimiluminescent.

On fait réagir ici la biotine, activée par un groupement hydroxysuccinimide avec la Luminarine-6, selon la réaction suivante:

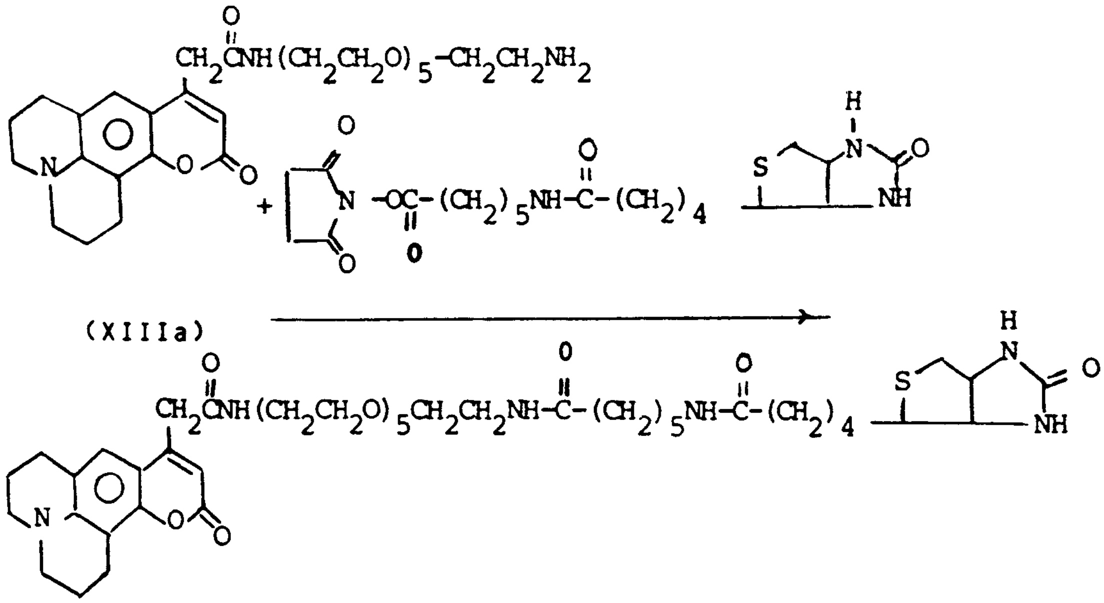

(XIIIa)

Dans un tube Eppendorf contenant 1,7 mg de biotine LC.NHS (de Pierce), on introduit 1,7 mg de Luminarine-6 dissoute dans 100 µl de solution de bicarbonate 0,05 M (pH 8,5). On fait réagir durant 1 heure à 4 °C. On contrôle l'efficacité du marquage par CCM, et on stocke à -20 °C, pour faire réagir, sans autre traitement, avec de l'avidine, au moment voulu.

Contrôle de marquage:

- C.C.M.:
  silice fluorescente
  Système $BuOH/AcOH/H_2O$ 60/20/20:

On observe la disparition du spot de la Luminarine-6 (fluorescent et révélé à la ninhydrine - Rf = 0,35) et l'apparition d'un spot de (biotine-Luminarine) de Rf = 0,5, fluorescent et révélé à l'iodoplatinate (réactif spécifique du noyau biotine).

**Exemple 5 :** Synthèse de la Luminarine-7 de formule:

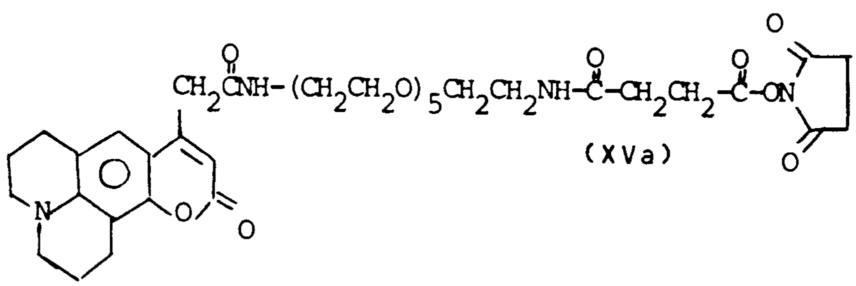

(XVa)

On part de la Luminarine-6 décrite dans l'exemple 3 et on réalise les deux réactions suivantes:

a) Action de l'anhydride succinique sur la Luminarine-6.

La réaction correspond au schéma réactionnel suivant:

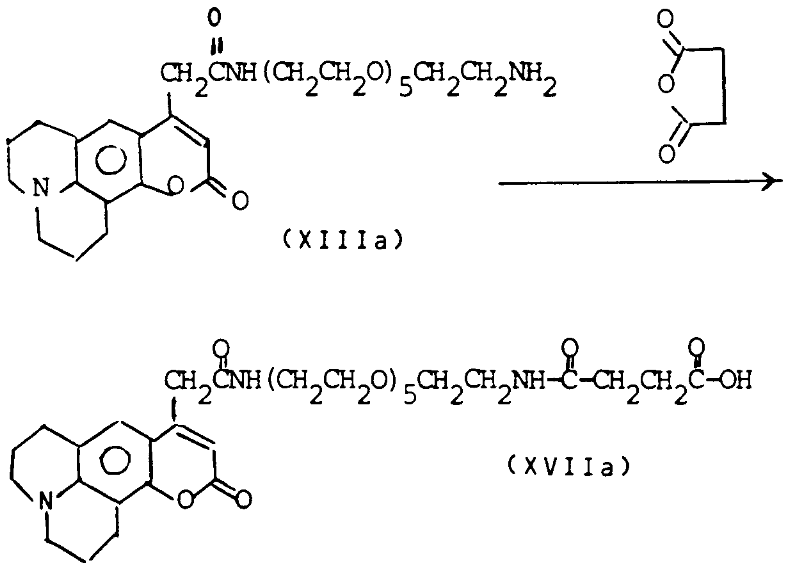

(XIIIa)

(XVIIa)

On met sous agitation durant 7 heures à 20 ° C, à l'abri de la lumière et de l'humidité, 784 mg (1,39 mmol) de Luminarine-6 et 140 mg (1,4 mmol) d'anhydride succinique dissous dans 10 ml de dichlorométhane.

On lave à l'eau, on séche, et on évapore à sec.

On obtient une huile marron que l'on utilisera telle quelle pour le stade suivant.

- C.C.M.:
  silice fluorescente
  système BuOH/AcOH/$H_2O$ 70/20/10

On observe la disparition du spot de Luminarine-6 et la formation d'un nouveau spot fluorescent de Rf = 0,55.

- Spectrométrie de masse (ionisation chimique à l'ammoniaque). On retrouve bien le pic moléculaire à M = 661 comme le montre la figure 2 qui représente le spectre du dérivé (XVIIa).

b) Couplage de l'acide (XVIIa) avec l'hydroxy succinimide en présence de dicyclohexyl carbodiimide, suivant le schéma suivant:

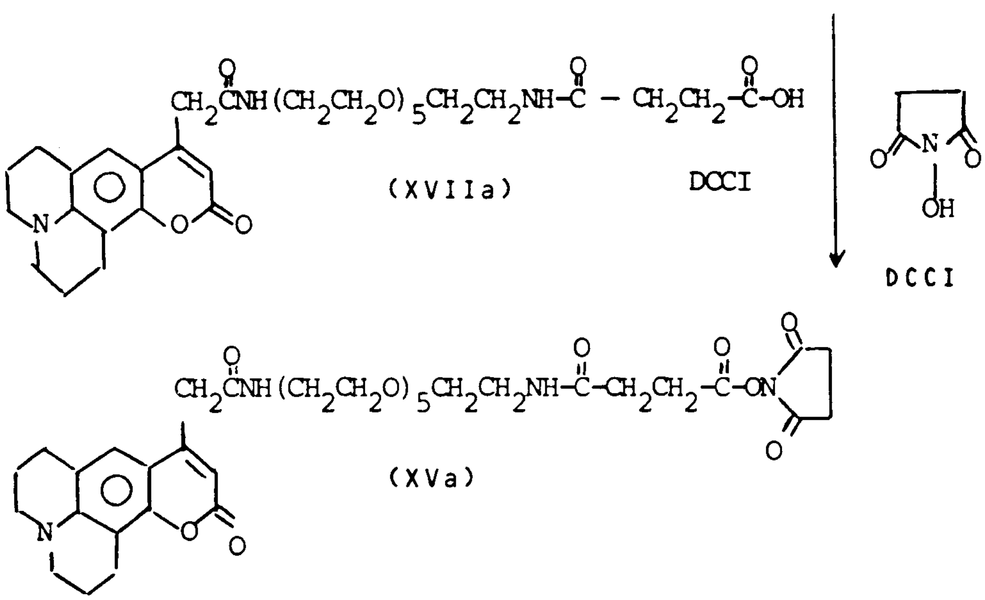


(XVIIa)

(XVa)

Dans un mélange de 250 mg (0,377 mmol) du dérivé (XVIIa) dans 1 ml de diméthylformamide anhydre, on introduit 43 mg (0,377 mmol) d'hydroxysuccinimide. On refroidit à 0 °C et on ajoute 93 mg de dicyclohexyl carbodiimide (0,45 mmol).

On agite durant 2 heures puis on filtre la dixyclohexyl-urée formée. Le produit restant, la Luminarine-7 est conservé en solution dans le diméthylformamide. Il se conserve pendant plusieurs mois à -20 °C sans altération (en présence de $P_2O_5$). Une partie aliquote mise à sec présente une hydrosolubilité totale.

- C.C.M.:
  silice en phase inversée $C_{18}$
  Système $EtOH/H_2O/NH_4OH$ 70/25/5
  Spot fluorescent à Rf = 0,84
- Contrôle de réactivité: le produit, mis en présence de 3 équivalents de pentylamine durant 2 heures, réagit et conduit à un nouveau produit fluorescent de Rf = 0,72 (même système de C.C.M.).
- Spectrométrie de masse (ionisation chimique à l'ammoniaque). Le pic de plus grande masse trouvée est de M = 661, comme le montre la figure 3 qui représente le spectre de la Luminarine-7. Ceci correspond bien au mode de fragmentation des esters d'hydroxysuccinimide.

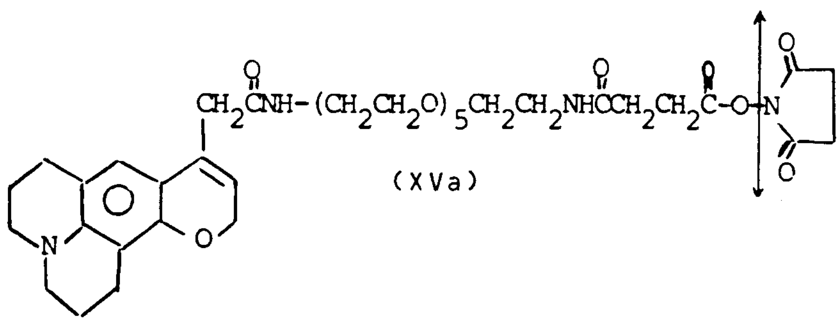

(XVa)

De plus, on ne peut pas le confondre avec le spectre de masse du produit précédent, car en dehors du pic à M = 661, les deux chromatogrammes ne sont pas superposables.

**Exemple 6 :** Utilisation de la Luminarine-7 pour le marquage de l'avidine.

On réalise le marquage de groupements aminés de la protéine (avidine) selon le schéma réactionnel suivant (rapport molaire avidine/Luminarine-7, 1/4,7):

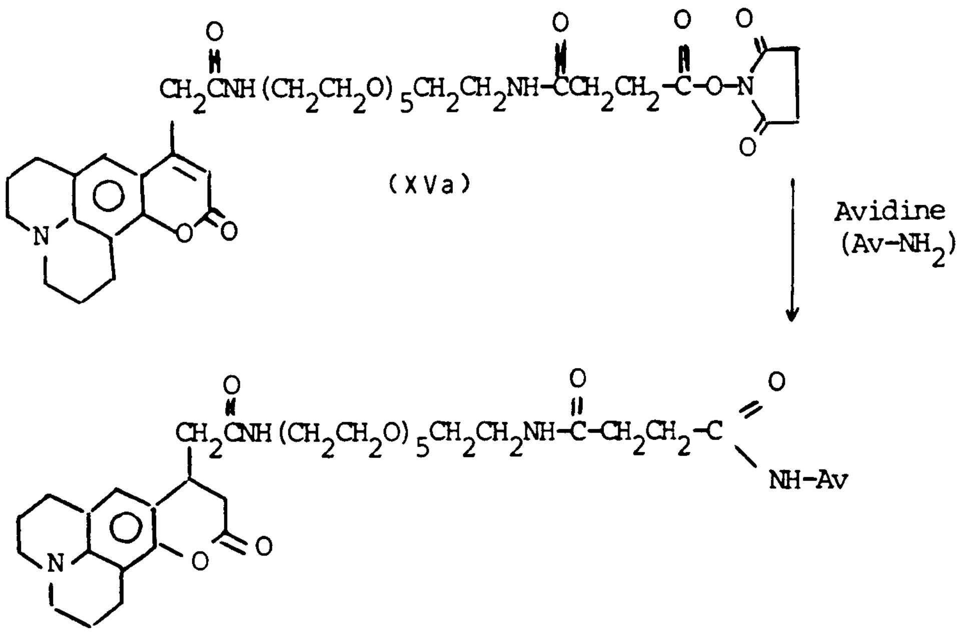


On prépare les solutions stocks suivantes:

avidine: 10 mg/1 ml, tampon bicarbonate 0,05M; pH 8,5

- Luminarine 250 mg/1 ml, DMF anhydre.

On introduit 1 ml de la solution d'avidine dans un tube Eppendorf. On ajoute 2,12 µl de la solution de Luminarine, sous bonne agitation.

On laisse une heure à 4 °C à l'abri de la lumière. On sépare ensuite les produits sur une colonne de gel G25 "fine grade" de Pharmacia, équilibrée et éluée avec du tampon bicarbonate. La fraction de tête, contenant l'avidine marquée, est contrôlée, partagée en parties aliquotes, et conservée à -20 °C.

Le contrôle est effectué par CCM.

- C.C.M.:
  silice fluorescente
  système BuOH/AcOH/$H_2O$ 60/20/20

Sur le chromatogramme on n'observe plus le spot de la Luminarine-7 à Rf 0,48. On observe seulement un spot non migré, ayant le comportement de l'avidine, et jaune fluorescent, attestant la fixation de la Luminarine.

**Exemple 7 :** Synthèse de la Luminarine-8 de formule :

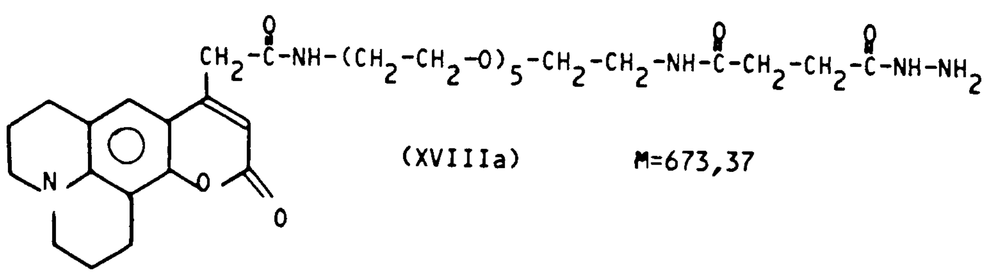

(XVIIIa) M=673,37

On part de la Luminarine-7 décrite dans l'exemple 5 et on réalise la réaction suivante: action de l'hydrate d'hydrazine en excès sur la Luminarine-7, selon le schéma réactionnel suivant:

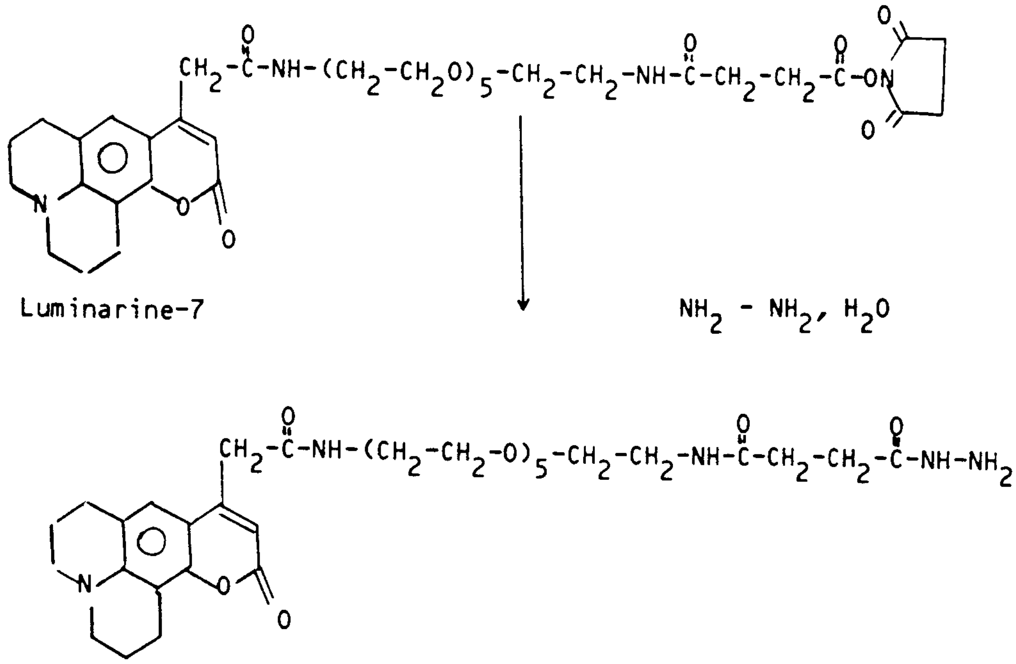


Luminarine-7

Luminarine-8

On part d'une solution de 630 mg de Luminarine-7 ($8{,}3.10^{-4}$ mmol) dans 3 ml de diméthylformamide (solution à 20%). On dilue avec 2 ml d'eau.

On introduit 1,5 ml (0,03 mmol) d'hydrate d'hydrazine et on agite durant 3 h.

On ajoute 5 ml d'eau et on extrait par 2x15 ml de chloroforme; on sèche et on évapore à sec, ce qui donne 0,3 g de Luminarine-8, ce qui correspond à un rendement de 54%.

Contrôle:

1°) C.C.M. silice fluorescente.
Système: BuoH/AcoH/$H_2O$ 60: 20: 20

On observe la disparition du spot de la Luminarine-7 (Rf = 0,42) et l'apparition d'un nouveau spot fluorescent de Rf = 0,23.

2°) Spectrométrie de masse (ionisation chimique à l'ammoniaque). Ce spectre est représenté sur la figure 4.

On retrouve bien le pic moléculaire à 676 + 1.

**Exemple 8 :** Synthèse de la Luminarine-9 de formule :

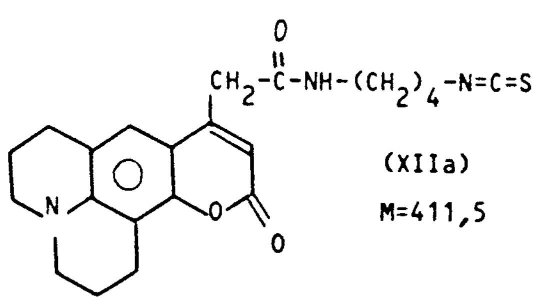

(XIIa)

M=411,5

On part de la Luminarine-4 (IVa) et on réalise la réaction suivante: action du thiocarbonyl diimidazole sur la Luminarine-4 selon le schéma réactionnel suivant:

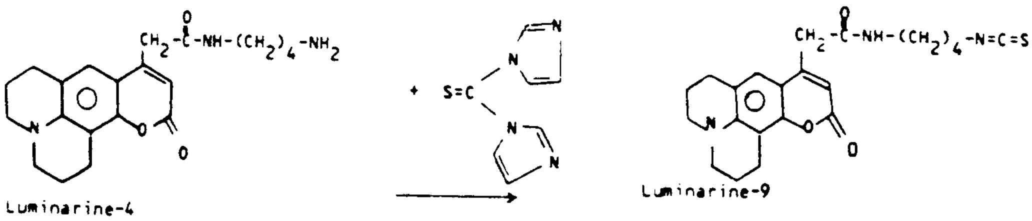


Dans un ballon de 25 ml, protégé de l'humidité, on dissout 535 mg (3 mmol) de thiocarbonyl diimidazole dans 15 ml de chloroforme anhydre.

On refroidit à 5°C et on introduit par fractions 1,1 g (3 mmol) de Luminarine-4, en maintenant la température entre 5° et 10°C.

Après 2h à température ambiante, on évapore à sec. Le résidu est purifié sur une colonne de gel de silice; éluant: $CH_2Cl_2$, puis $CH_2Cl_2$-THF 80: 20; on évapore à sec et on obtient 370 mg de produit jaune (Rt = 30%).

Contrôle :

1°) C.C.M. silice fluorescente.
Système: $CH_2Cl_2$: THF 80: 20

On observe la disparition du spot de la Luminarine-4 (Rf = 0,05) et l'apparition d'un nouveau spot fluorescent de Rf = 0,58

2°) Spectrométrie de masse (impact électronique). Le spectre est représenté sur la figure 5.

On retrouve bien le pic moléculaire à 411.

**Exemple 9 :** Réaction de la Luminarine-9 avec la pentylamine.

On réalise le marquage de la pentylamine avec la Luminarine-9, selon le schéma réactionnel suivant:

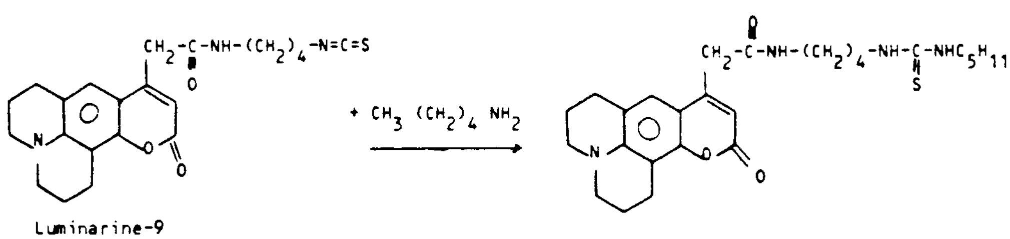


Dans un "réacti-vial" de 4 ml, on dissout 130 mg (0,315 mmol) de Luminarine-9 dans 2,6 ml de dichlorométhane. On introduit 109 µl (82 mg-0,94 mmol) de pentylamine.

On agite pendant 2 h à la température ambiante (le contrôle par CCM indique une réaction quantitative). On laisse cristalliser à 5°C durant 48h. On filtre et on sèche. Rt = 120 mg (77%).

Contrôle :

1°) C.C.M. silice fluorescente.
Système: $CH_2Cl_2$: THF 80: 20

On observe un spot unique fluorescent de Rf = 0,32 alors que la Luminarine-9 a un Rf = 0,58

2°) Spectrométrie de masse. Ce spectre est représenté sur la figure 6. On retrouve bien un petit pic moléculaire à 498, ainsi qu'un important fragment à 411, le spectre restant totalement différencié de celui de la Luminarine-9.

**Exemple 10.**

Pour mettre en évidence les propriétés améliorées des Luminarines de l'invention, on a effectué des mesures de fluorescence, d'absorbance et de chimiluminescence en utilisant comme fluorophore des coumarines de l'art antérieur qui sont la coumarine-1®, diéthylamino-7 méthyl-4 coumarine (DEMC) de formule:

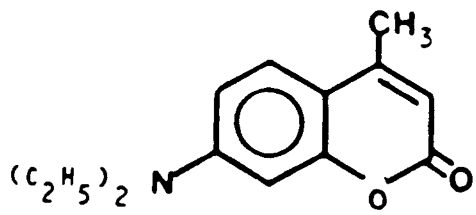

et la coumarine-311®, diméthylamino-7 méthyl-4 coumarine (DMMC) de formule:

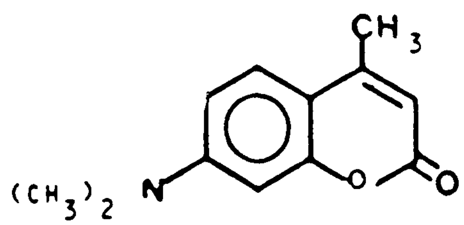

pour les comparer avec la coumarine-102®, tétrahydro-2, 3, 5, 6, 7, 1H, 5H, 11H-(1)- benzopyrano (6, 7, 8-ij) méthyl-9 quinolizinone-11 (TBMQ) qui est le noyau de base des dérivés de l'invention de formule:

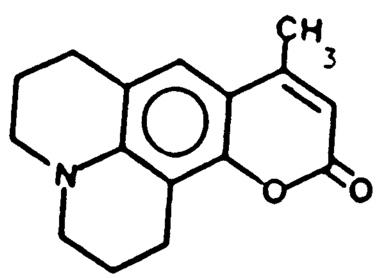

Les mesures de fluorescence sont effectuées avec un spectrofluorimètre Perkin Elmer LS5 avec des largeurs de fentes de 2 nm pour les deux monochromateurs.

Les mesures d'absorbance sont effectuées avec un spectrophotomètre Jobin Yvon JY3. Les mesures de chimiluminescence sont obtenues en utilisant un spectrofluorimètre Jobin Yvon JY4 en utilisant des largeurs de fentes de 10 nm. La cellule d'échantillons liquides est une cuvette de quartz de 10 x 10 mm d'une capacité de 3 ml remplie de 2 ml de solution, ce qui correspond à un volume réel de solution exposée au photomultiplicateur de 1,35 ml.

**Mesures de fluorescence et d'absorbance.**

Ces mesures ont été effectuées à $20^{+}$ 2°C, et on a déterminé les rendements quantiques de fluorescence $R_F$ des coumarines-1, 311 et 102 par rapport au bisulfate de quinine dont le rendement quantique de fluorescence est de 0,546, en suivant la méthode de Parker décrite dans Analyst, 85, (1960), p. 587, avec une solution de bisulfate de quinine à 2 mg/l dans $H_2SO_4$ 0,1N.

Pour les coumarines, on a utilisé une solution d'acétone et d'acétate d'éthyle dans un rapport en volume 75/25 avec une concentration en coumarine de $10^{-6}$ mol/l en assurant dans chaque cas une

absorbance inférieure à 0,05.

**Mesures de chimiluminescence.**

Pour déterminer les rendements quantiques de chimiluminescence $R_C$, on a estimé l'intensité en fonction du temps au maximum de l'émission. Lorsque la décroissance de l'intensité est assez basse, le spectre d'émission est tracé, puis l'intensité en fonction du temps est suivie jusqu'à ce que l'intensité soit tombée à 2% de l'intensité maximale.

Les autres paramètres de luminescence sont mesurés directement, il s'agit de l'intensité maximale (Imax), du temps pour atteindre l'intensité maximale (Tmax), et du temps pour atteindre la moitié de l'intensité maximale après le temps maximal (T1/2).

Le rendement de chimiluminescence $R_C$ est calculé en suivant la méthode de Rauhut décrite dans J. Am. Chem. Soc., 88, 15 (1966), 3604.

Le rendement d'excitation $R_{ex}$ est déterminé à partir de la formule:

$$RC = R_{ex} \times R_F$$

Pour les mesures de chimiluminescence avec le TCPO, on ajoute à 1 ml de la solution contenant $10^{-3}$ mol/l de coumarine, 0,5 ml d'une solution à $2.10^{-3}$ mol/l de TCPO dans de l'acétate d'éthyle, 0,5 ml d'une solution à $2.10^{-2}$ mol/l de $H_2O_2$ dans l'acétone et 10 $\mu$l d'un tampon imidazole nitrate, pH 7,5, à 0,1 mol/l. Lorsqu'on utilise le DNPO, on ajoute à la même solution de coumarine dans l'acétone, 0,5 ml d'une solution à $10^{-3}$ mol/l de DNPO dans l'acétate d'éthyle et 0,5 ml d'une solution à $10^{-2}$ mol/l de $H_2O_2$ dans de l'acétone.

Les propriétés de fluorescence et d'absorbance de ces coumarines sont données dans le tableau 1, et les résultats de chimiluminescence sont donnés dans le tableau 2.

Au vu des tableaux 1 et 2, on remarque que le noyau coumarinique utilisé dans l'invention qui correspond à la coumarine 102, présente les meilleures propriétés de fluorescence, d'absorbance et de chimiluminescence.

Dans le tableau 3, on a donné les résultats obtenus en ce qui concerne le rendement de chimiluminescence $R_C$ en utilisant TCPO et $H_2O_2$ en fonction de la concentration en coumarine-102, ainsi que les valeurs de Imax, Tmax et T1/2.

Au vu de ces résultats, on constate que le rendement de chimiluminescence augmente avec la concentration en coumarine-102.

Dans le tableau 4, on a donné les variations du rendement de chimiluminescence, de Imax, de Tmax et de T1/2, en fonction du pH de la solution.

Les résultats obtenus montrent que le rendement de chimiluminescence croît jusqu'à pH 6 et qu'il décroît ensuite.

En revanche les valeurs de Tmax et de T1/2 décroissent lorsque le pH augmente au-delà de 5. L'intensité maximale augmente de façon importante avec le pH.

TABLEAU 1

| | RF[1] | $\epsilon$ 365 nm | S $10^3$ |
|---|---|---|---|
| COUMARINE-102 | 0,57 | 23 100 | 36 |
| COUMARINE-1 | 0,55 | 38 000 | 57 |
| COUMARINE-311 | 0,52 | 26 900 | 40 |

RF = rendement quantique de fluorescence.
$\epsilon$ = coefficient d'extinction moléculaire
S = sensibilité de fluorescence

TABLEAU 2

| | DMPO | | TCPO | |
|---|---|---|---|---|
| | $R_C$ $10^2$E.mol$^{-1}$ | $R_E$ $10^2$ | $R_C$ $10^2$E.mol$^{-1}$ | $R_E$ $10^2$ |
| COUMARINE-102 | 0,26 | 0,45 | 0,37 | 0,65 |
| COUMARINE-1 | 0,18 | 0,33 | 0,37 | 0,67 |
| COUMARINE-311 | 0,13 | 0,24 | 0,17 | 0,32 |

$R_C$ = rendement quantique de chimiluminescence
$R_E$ = rendement d'excitation
DNPO = bis-(dinitro-2,4 phényl)oxalate
TCPO = bis-(trichloro-2,4,6 phényl) oxalate.

TABLEAU 3

| ($C_{102}$) E. mol$^{-1}$ | $R_C$ x $10^4$ E. mol$^{-1}$ | Imax A.U. | Tmax min. | T1/2 min. |
|---|---|---|---|---|
| 1.4 x $10^{-3}$ | 12,0 | 301 | 0,2 | 0,10 |
| 1.4 x $10^{-6}$ | 4,14 | 75 | 0,2 | 0,15 |
| 1.4 x $10^{-7}$ | 2,31 | 31 | 0,2 | 0,12 |
| 1.4 x $10^{-8}$ | 0,40 | 3,2 | 0,2 | 0,10 |
| 1.4 x $10^{-9}$ | 0,20 | 0,3 | 0,2 | 0,08 |

TABLEAU 4

| pH | $R_C$ x $10^4$ E. mol$^{-1}$ | Imax A.U. | Tmax min. | T1/2 min. |
|---|---|---|---|---|
| 4 | 1,52 | 24 | 0,8 | 5,0 |
| 5 | 1,58 | 17 | 1,0 | 6,0 |
| 6 | 2,34 | 25 | 0,8 | 5,6 |
| 7 | 2,23 | 110 | 0,4 | 1,8 |
| 8 | 1,60 | 245 | 0,2 | 0,4 |

**Revendications**

**Revendications pour les Etats contractants suivants : AT, BE, CH, DE, DK, FR, GB, GR, IT, LI, LU, NL, SE**

**1.** Dérivé de tétrahydro-2,3,6,7,1H,5H,11H-(1) benzopyrano (6,7,8,ij) quinolizinone-11 répondant à la formule :

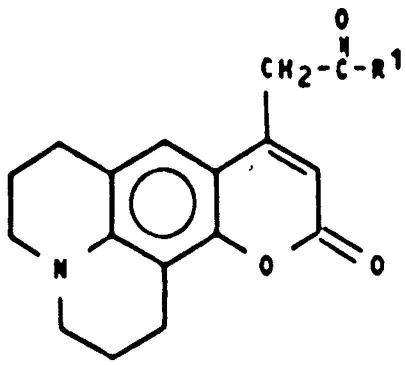

(I)

dans laquelle $R^1$ représente :

1°) $-NH-(CH_2)_n-R^2$

avec n étant un nombre entier allant de 1 à 20 et $R^2$ représentant $-N=C=S$ ou $NH-CO-CH_2X$ avec X représentant I, Br ou Cl, ou

2°) $-NH-(CH_2-CH_2-O)_m-CH_2-CH_2-R^3$

avec m un nombre entier allant de 1 à 30 et $R^3$ représentant un groupe choisi parmi les groupes de formule

$-NH_2$

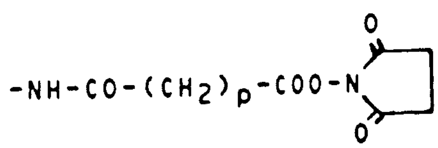

$-NH-CO-(CH_2)_p-CO-NH-NH_2$,

$-N=C=S$, et

$-NH-CO-CH_2X$

dans lesquelles p est un nombre entier allant de 1 à 10 et X représente Br, Cl ou I.

**2.** Dérivé de tétrahydro-2,3,6,7,1H,5H,11H-(1) benzopyrano (6,7,8,ij) quinolizinone-11, selon la revendication 1, caractérisé en ce qu'il répond à la formule :

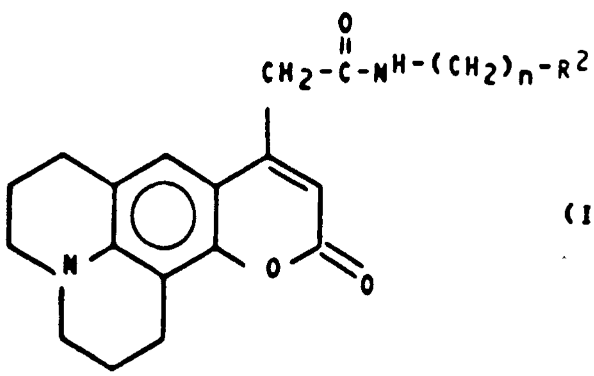

(II)

dans laquelle n est un nombre entier allant de 1 à 20 et $R^2$ représente $-N=C=S$ ou $NH-CO-CH_2X$ avec X représentant I, Br ou Cl.

**3.** Dérivé de tétrahydro-2,3,6,7,1H,5H,11H-(1) benzopyrano (6,7,8,ij) quinolizinone-11 selon la revendication 2, caractérisé en ce que n est égal à 4.

4. Dérivé de tétrahydro-2,3,6,7,1H,5H,11H-(1) benzopyrano (6,7,8,ij) quinolizinone-11, selon la revendication 1, caractérisé en ce qu'il répond à la formule :

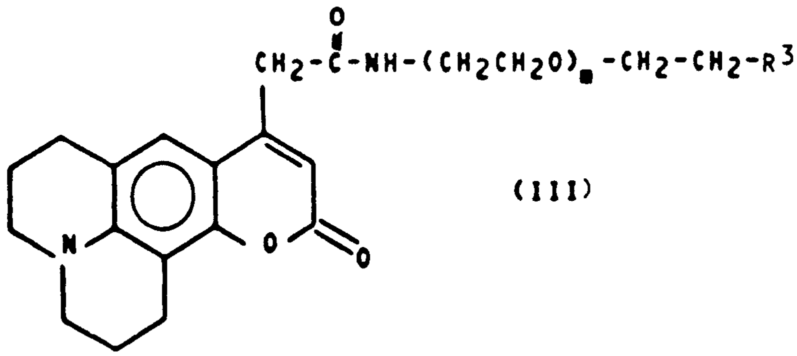

(III)

dans laquelle m est un nombre entier allant de 1 à 30 et $R^3$ est tel que défini dans la revendication 1.

5. Dérivé de tétrahydro-2,3,6,7,1H,5H,11H-(1) benzopyrano (6,7,8,ij) quinolizinone-11 selon la revendication 4, caractérisé en ce que m est égal à 5.

6. Dérivé de tétrahydro-2,3,6,7,1H,5H,11H-(1) benzopyrano (6,7,8,ij) quinolizinone-11 selon la revendication 5, caractérisé en ce que $R^3$ est $NH_2$.

7. Dérivé de tétrahydro-2,3,6,7,1H,5H,11H-(1)benzopyrano (6,7,8,ij) quinolizinone-11 selon la revendication 5, caractérisé en ce que $R^3$ est

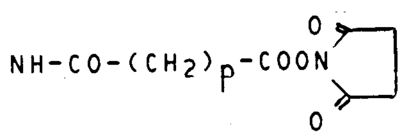

avec p étant un nombre entier allant de 1 à 10.

8. Dérivé de tétrahydro-2,3,6,7,1H,5H,11H-(1) benzopyrano (6,7,8,ij) quinolizinone-11 selon la revendication 7, caractérisé en ce que p est égal à 2.

9. Dérivé de tétrahydro-2,3,6,7,1H,5H,11H-(1) benzopyrano (6,7,8,ij) quinolizinone-11 selon la revendication 5, caractérisé en ce que $R^3$ est NH $CO(CH_2)_pCONH$ $NH_2$.

10. Dérivé de tétrahydro-2,3,6,7,1H,5H,11H(1) benzopyrano (6,7,8,ij) quinolizinone-11 selon la revendication 9, caractérisé en ce que p est égal à 2.

11. Procédé de détection d'un composé comportant une fonction amine primaire ou secondaire, caractérisé en ce qu'il consiste à faire réagir le composé à détecter avec un dérivé répondant à la formule :

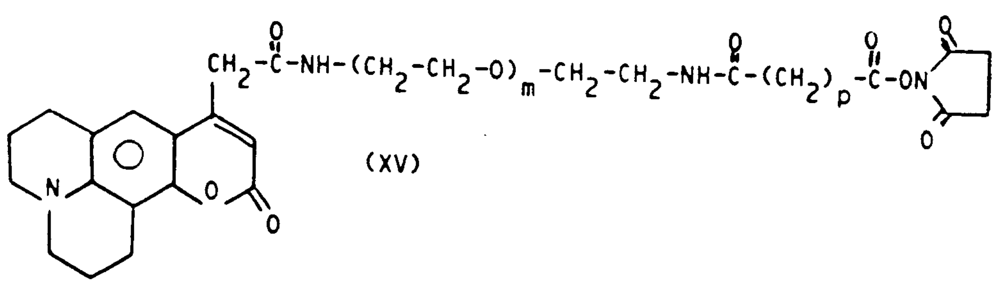

(XV)

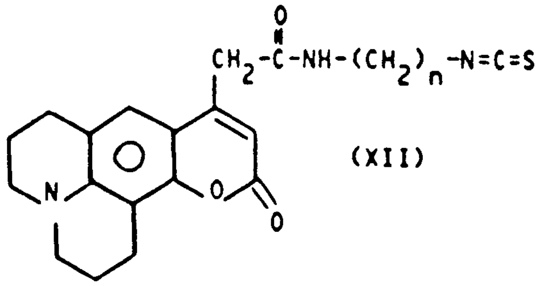

(XII) ou

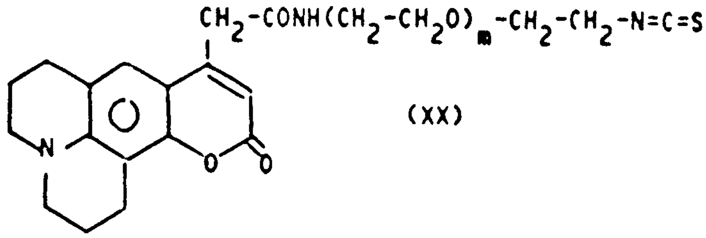

(XX)

dans laquelle m est un nombre entier de 1 à 30, n est un nombre entier allant de 1 à 20 et p est un nombre entier allant de 1 à 10, pour former un dérivé du composé à détecter, et à détecter ensuite ce dérivé par absorptiométrie, fluorimétrie, ou chimiluminescence.

**12.** Procédé de détection d'un composé comportant un groupe SH, caractérisé en ce qu'il consiste à faire réagir le dérivé de formule :

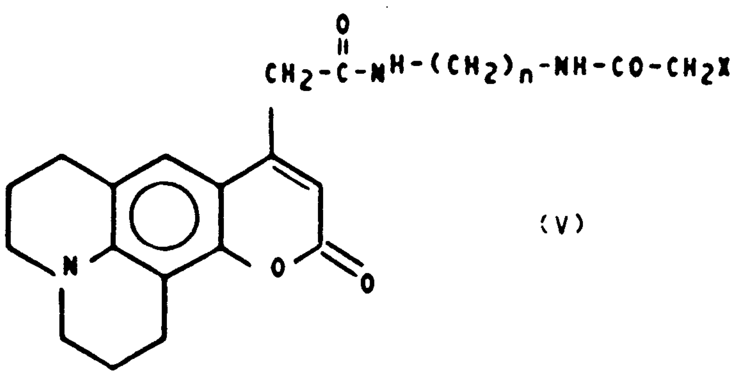

(V)

ou

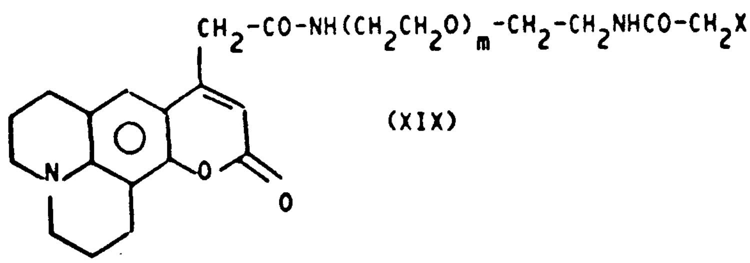

(XIX)

dans laquelle m est un nombre entier allant de 1 à 30, n est un nombre entier allant de 1 à 20 et X représente I, Br ou Cl, pour former un dérivé du composé à détecter, et à détecter ensuite ce dérivé par absorptiométrie, fluorimétrie, ou chimiluminescence.

**13.** Procédé de détection d'un composé comportant un groupe -COO-, caractérisé en ce que l'on fait réagir ce composé avec un dérivé de formule :

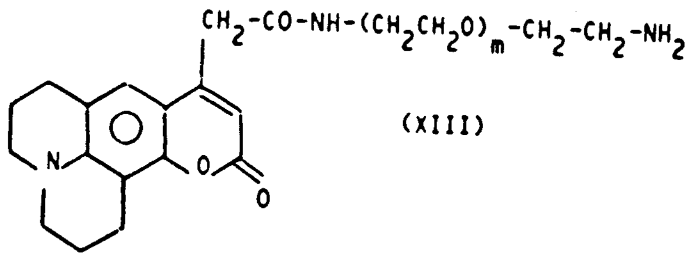

(XIII)

dans laquelle m est un nombre entier de 1 à 30, pour former un dérivé du composé à détecter, et en ce que l'on détecte ensuite ce dérivé par absorptiométrie, fluorimétrie, ou chimiluminescence.

**14.** Procédé selon l'une quelconque des revendications 11 à 13, caractérisé en ce que l'on réalise la détection par chimiluminescence en utilisant un ester oxalique et du peroxyde d'hydrogène.

**15.** Procédé selon la revendication 14, caractérisé en ce que l'ester oxalique est le bis-(trichloro-2,4,6 phényl)oxalate ou le bis-(dinitro 2,4 phényl)oxalate.

**Revendications pour l'Etat contractant suivant : ES**

**1.** Procédé de préparation d'un dérivé de tétrahydro-2,3,6,7,1H,5H,11H-(1) benzopyrano (6,7,8,ij) quinolizinone-11 répondant à la formule:

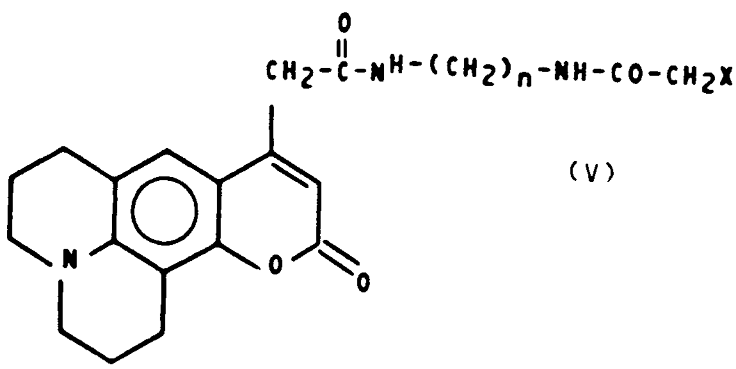

(V)

dans laquelle n est un nombre entier allant de 1 à 20 et X représente I, Br ou Cl, caractérisé en ce qu'il consiste à faire réagir un Dérivé de tétrahydro-2,3,6,7,1H,5H,11H-(1) benzopyrano (6,7,8,ij) quinolizinone-11 de formule

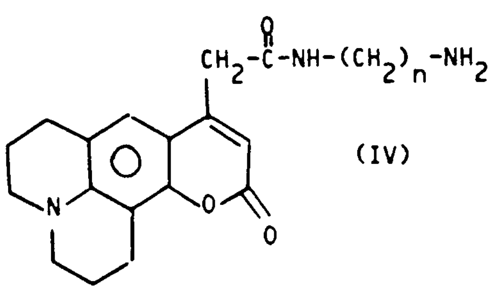

(IV)

avec un anhydride halogéno acétique, en présence d'une trialkyl amine.

**2.** Procédé de préparation d'un dérivé de tétrahydro-2,3,6,7,1H,5H,11H-(1) benzopyrano (6,7,8, ij) quinolizinone-11 répondant à la formule:

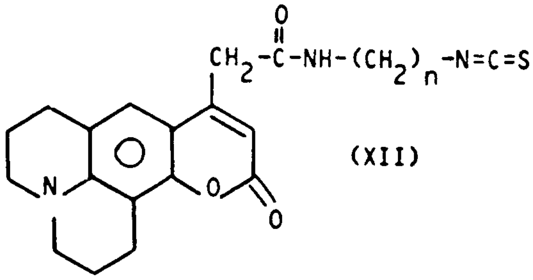

(XII)

dans laquelle n est un nombre entier de 1 à 20, caractérisé en ce qu'il consiste à faire réagir un dérivé de formule:

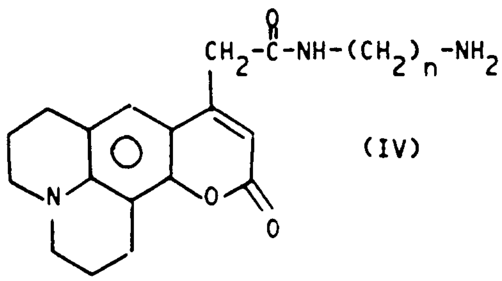

(IV)

avec le thiocarbonyl diimidazole.

3. Procédé selon l'une quelconque des revendications 1 et 2, caractérisé en ce que l'on prépare le dérivé de formule (IV) en effectuant les étapes successives suivantes:
1°) faire réagir la 8-hydroxyjulolidine de formule:

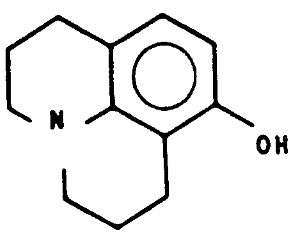

(VI)

avec un ester alkylique de l'acide oxo-3-glutarique de formule:

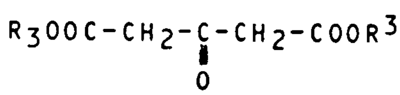

dans laquelle $R^3$ est un radical alkyle, pour former un composé de formule:

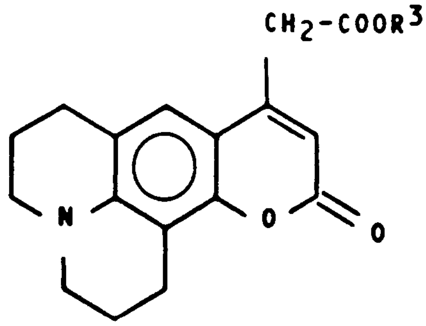

(VII)

2°) hydrolyser l'ester alkylique de formule (VII) obtenu précédemment pour obtenir l'acide de formule:

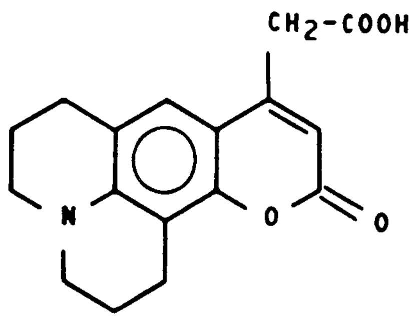

(VIII)

3°) faire réagir l'acide de formule (VIII) ainsi obtenu avec l'oxalate de dihydroxysuccinimide de formule:

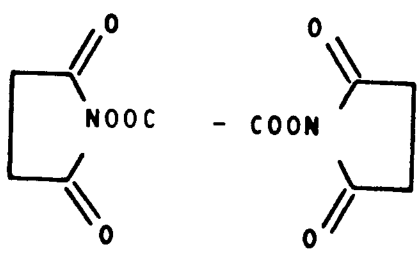

(IX)

ou l'hydroxysuccinimide en présence d'un agent de couplage pour former le dérivé de formule:

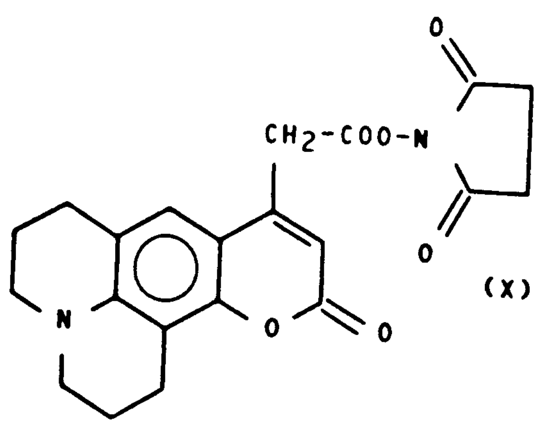

(X)

4°) faire réagir le dérivé de formule (X)

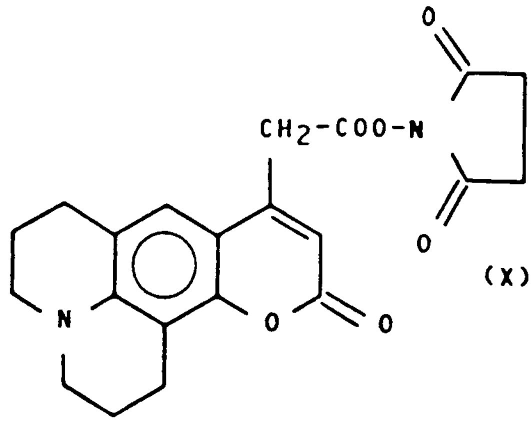

(X)

avec un diamino alcane de formule:

$H_2N-(CH_2)_n-NH_2$ (XI)

dans laquelle n a la signification donnée ci-dessus.

**4.** Procédé de préparation d'un Dérivé de tétrahydro-2,3,6,7,1H,5H,11H-(1) benzopyrano (6,7,8,ij) quinolizinone-11 de formule:

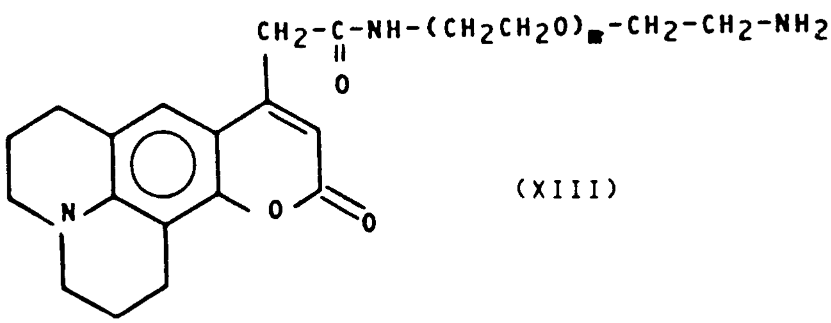

(XIII)

dans laquelle m est un nombre entier allant de 1 à 30, caractérisé en ce qu'il consiste à faire reagir le composé de formule:

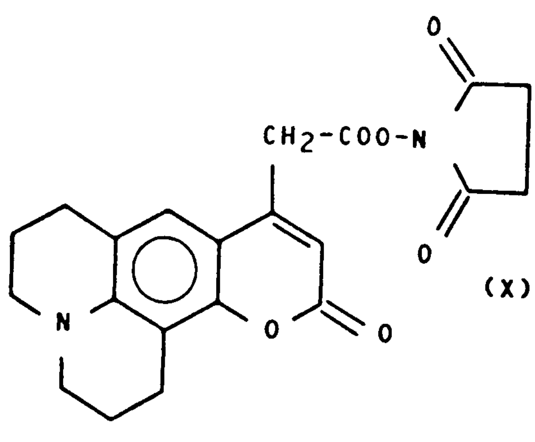

(X)

avec un excès de poly(oxy-1,2 éthane diyl) diamine de formule:

$H_2N-(CH_2CH_2O)_m-CH_2-CH_2NH_2$ (XIV)

dans laquelle m a la signification donnée ci-dessus.

**5.** Procédé selon la revendication 4, caractérisé en ce que l'on prépare le composé de formule (X) en effectuant les étapes successives suivantes:

1°) faire réagir la 8-hydroxyjulolidine de formule:

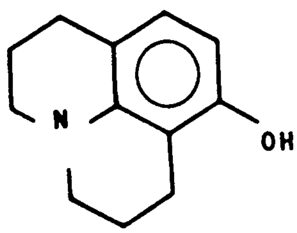

(VI)

avec un ester alkylique de l'acide oxo-3-glutarique de formule:

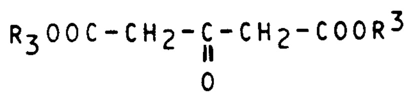

dans laquelle $R^3$ est un radical alkyle, pour former un composé de formule:

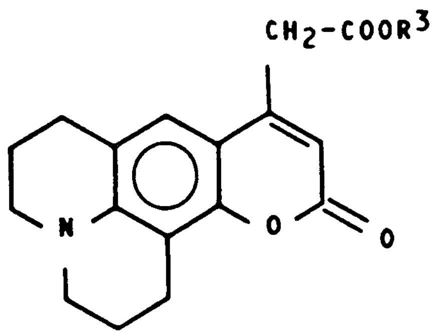

(VII)

2°) hydrolyser l'ester alkylique de formule (VII) obtenu précédemment pour obtenir l'acide de formule:

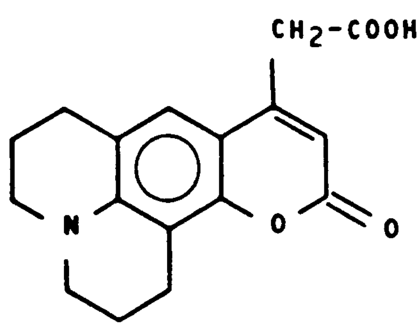

(VIII)

3°) faire réagir l'acide de formule (VIII) ainsi obtenu avec l'oxalate de dihydroxysuccinimide de formule:

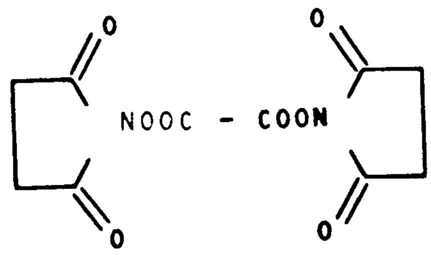

(IX)

pour former le dérivé de formule

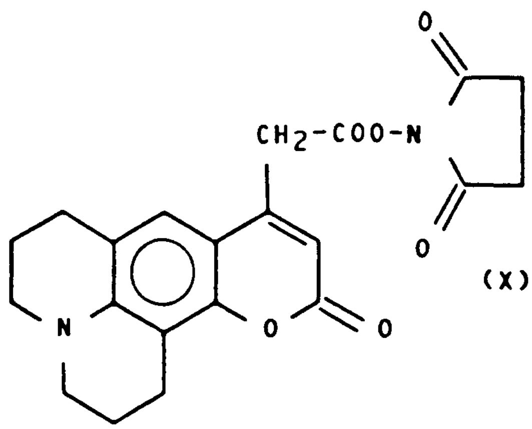

(X)

6. Procédé de préparation d'un dérivé de tétrahydro-2,3,6,7,1H,5H,11H-(1) benzopyrano (6,7,8,ij) quinolizinone-11

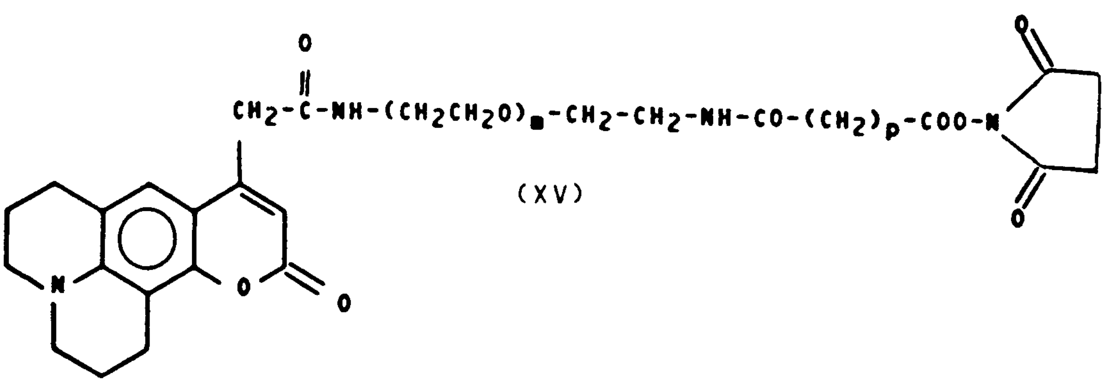

(XV)

dans laquelle m est un nombre entier allant de 1 à 30 et p est un nombre entier allant de 1 à 10, caractérisé en ce qu'il consiste:

1°) à faire réagir un composé de formule

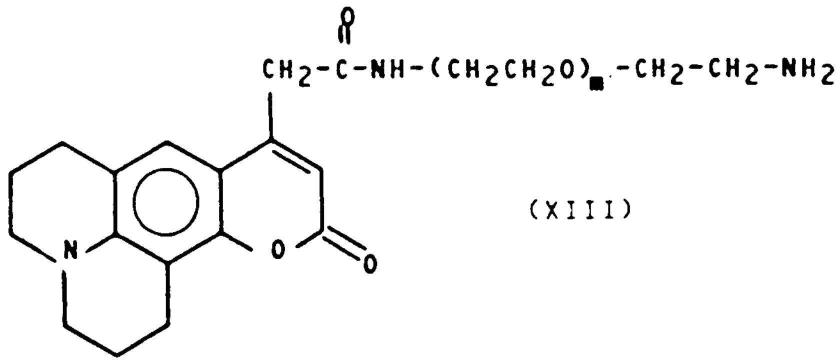

(XIII)

dans laquelle m a la signification donnée ci-dessus avec un anhydride de diacide de formule:

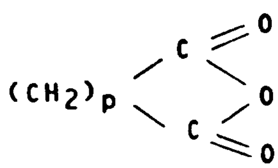

(XVI)

pour obtenir un acide de formule:

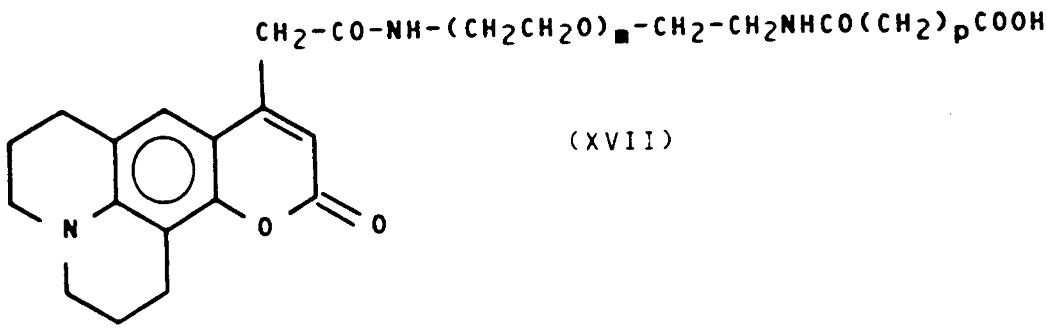

(XVII)

2°) à faire réagir ensuite l'acide de formule (XVII) avec l'hydroxysuccinimide en présence d'un agent de couplage pour former le dérivé de formule (XV).

**7.** Procédé de préparation d'un dérivé de tétrahydro-2,3,6,7,1H,5H,11H-(1) benzopyrano (6,7,8,ij) quinolizinone-11 répondant à la formule:

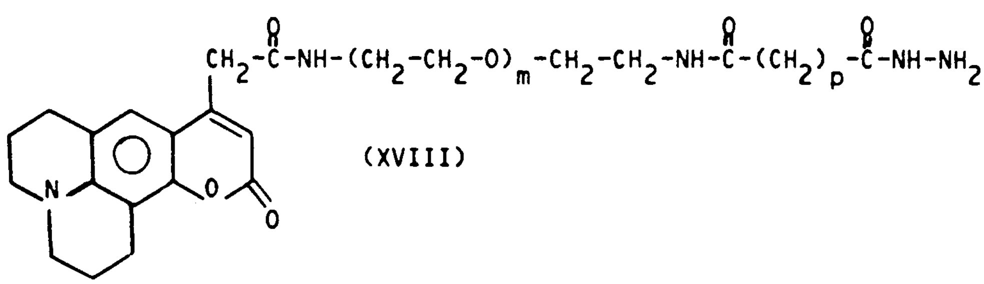

(XVIII)

dans laquelle m est un nombre entier de 1 à 30 et p est un nombre entier de 1 à 10, caractérisé en ce qu'il consiste à faire réagir un dérivé de tétrahydro-2,3,6,7,1H,5H,11H-(1) benzopyrano (6,7,8,ij) quinolizinone-11 de formule:

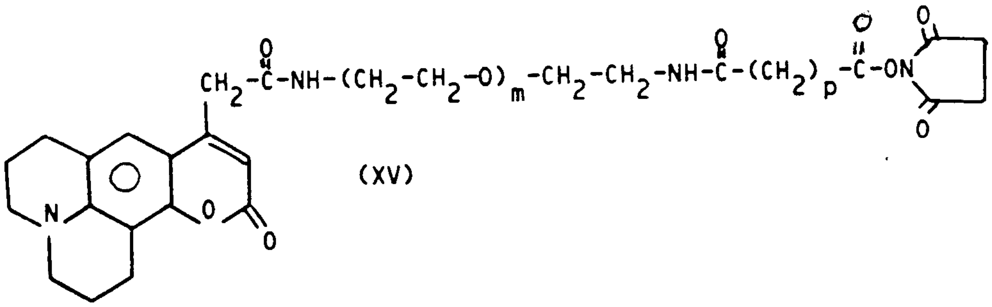

(XV)

avec un excès d'hydrate d'hydrazine.

**8.** Procédé selon la revendication 7, caractérisé en ce que le dérivé de formule (XV) est préparé par le procédé de la revendication 6.

**9.** Procédé de préparation d'un dérivé de tétrahydro-2,3,6,7,1H,5H,11H-(1) benzopyrano (6,7,8,ij) quinolizinone-11 de formule:

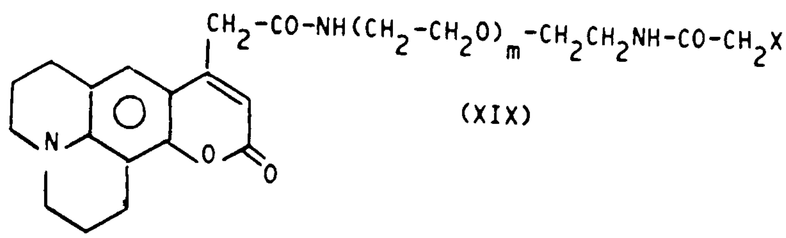

(XIX)

dans laquelle m est un nombre entier allant de 1 à 30, caractérisé en ce qu'il consiste à faire réagir un dérivé de tétrahydro-2,3,6,7,1H,5H,11H-(1) benzopyrano (6,7,8,ij) quinolizinone-11 de formule:

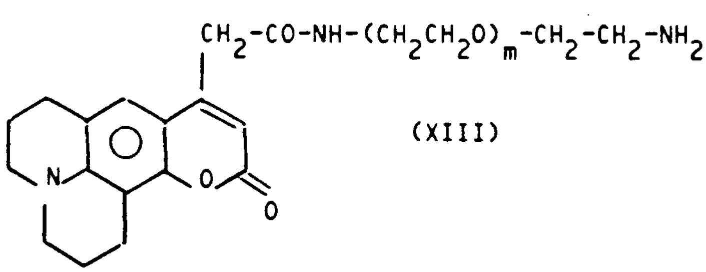

(XIII)

avec un anhydride halogénoacétique, en présence d'une trialkylamine.

**10.** Procédé de préparation d'un dérivé de tétrahydro-2,3,6,7,1H,5H,11H-(1) benzopyrano (6,7,8,ij) quinolizinone-11 répondant à la formule:

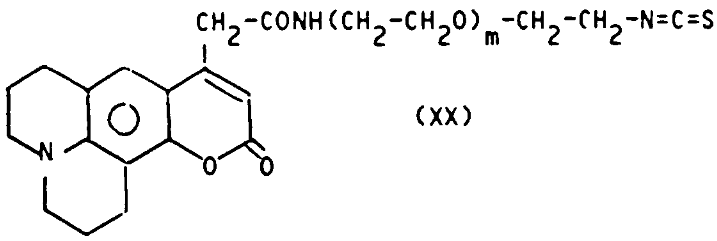

(XX)

dans laquelle m est un nombre entier de 1 à 30, caractérisé en ce qu'il consiste à faire réagir un dérivé de tétrahydro-2,3,6,7,1H,5H,11H-(1)benzopyrano (6,7,8,ij) quinolizinone-11 de formule:

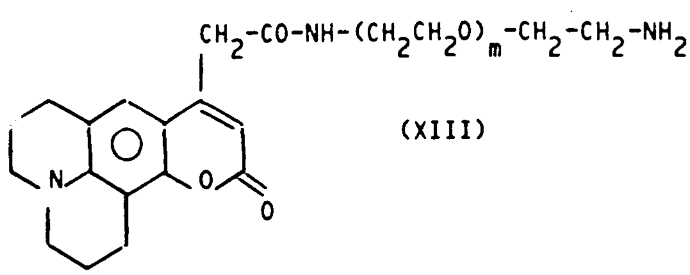

(XIII)

avec du thiocarbonyldiimidazole.

11. Procédé selon l'une quelconque des revendications 9 et 10, caractérisé en ce que le dérivé de formule (XIII) est préparé par le procédé de la revendication 4.

12. Procédé de détection d'un composé comportant un groupe amine primaire ou secondaire, caractérisé en ce que l'on fait réagir ce composé avec un dérivé de formule:

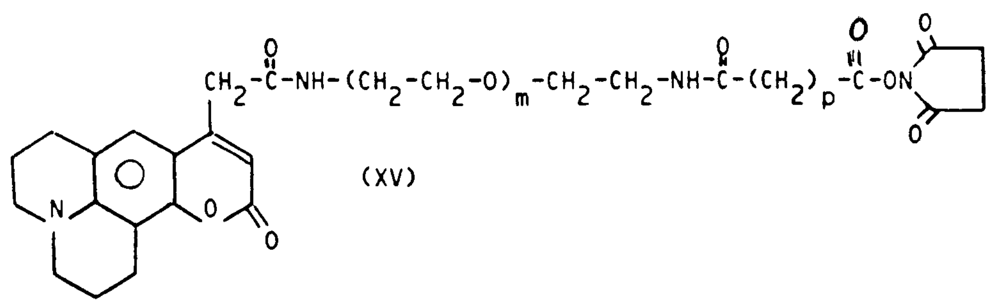

(XV)

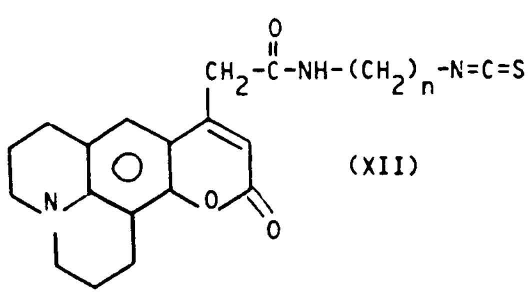

(XII)

ou

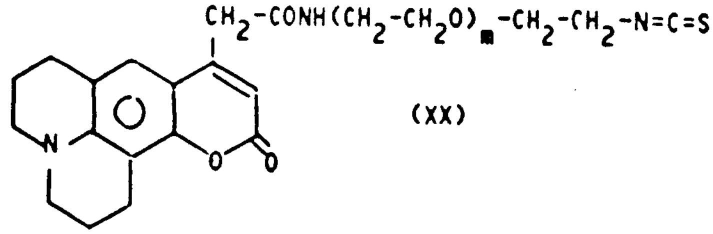

(XX)

dans laquelle m est un nombre entier de 1 à 30, n est un nombre entier allant de 1 à 20 et p est un nombre entier allant de 1 à 10, pour former un dérivé du composé à détecter, et à détecter ensuite ce dérivé par absorptiométrie, fluorimétrie, ou chimiluminescence.

**13.** Procédé de détection d'un composé comportant un groupe SH, caractérisé en ce qu'il consiste à faire réagir le dérivé de formule :

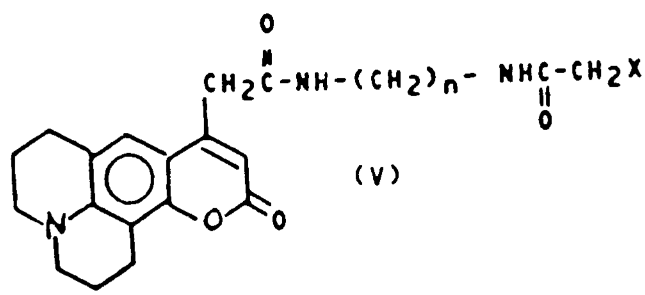

(V)

ou

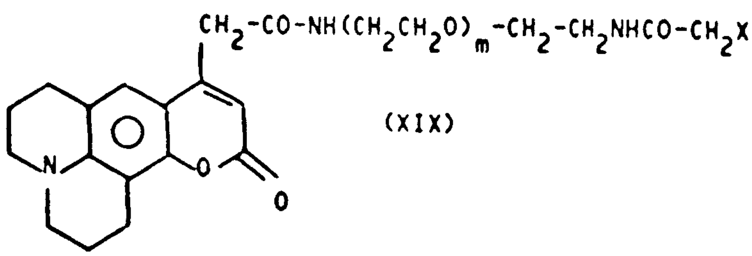

(XIX)

dans laquelle m est un nombre entier allant de 1 à 30, n est un nombre entier allant de 1 à 20 et X représente I, Br ou Cl, pour former un dérivé du composé à détecter, et à détecter ensuite ce dérivé par absorptiométrie, fluorimétrie, ou chimiluminescence.

**14.** Procédé de détection d'un composé comportant un groupe -COO-, caractérisé en ce que l'on fait réagir ce composé avec un dérivé de formule :

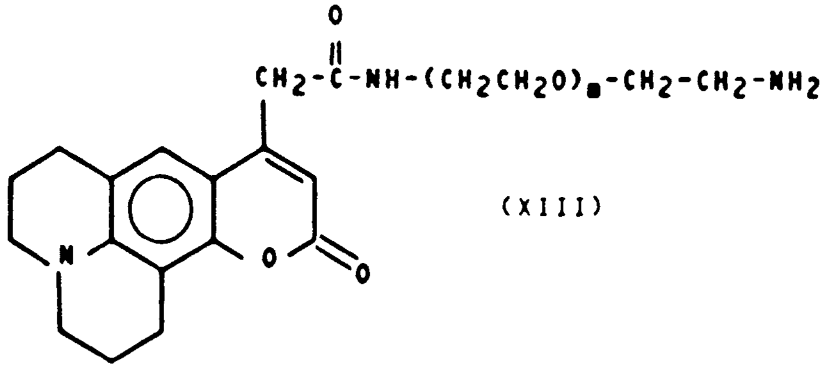

(XIII)

dans laquelle m est un nombre entier de 1 à 30, pour former un dérivé du composé à détecter, et en ce que l'on détecte ensuite ce dérivé par absorptiométrie, fluorimétrie, ou chimiluminescence.

**15.** Procédé selon l'une quelconque des revendications 12 à 14, caractérisé en ce que l'on réalise la détection par chimiluminescence en utilisant un ester oxalique et du peroxyde d'hydrogène.

**16.** Procédé selon la revendication 15, caractérisé en ce que l'ester oxalique est le bis-(trichloro-2,4,6 phényl)oxalate ou le bis-(dinitro 2,4 phényl)oxalate.

**17.** Procédé selon la revendication 1, caractérisé en ce que n est égal à 4 et X représente I.

**18.** Procédé selon la revendication 4, caractérisé en ce que m est égal à 5.

**19.** Procédé selon la revendication 6, caractérisé en ce que m est égal à 5 et p est égal à 2.

**20.** Procédé selon la revendication 7, caractérisé en ce que m est égal à 5 et p est égal à 2.

**21.** Procédé selon la revendication 2, caractérisé en ce que n est égal à 4.

**Claims**

**Claims for the following Contracting States : AT, BE, CH, DE, DK, FR, GB, GR, IT, LI, LU, NL, SE**

**1.** Derivative of tetrahydro-2,3,6,7,1H,5H,11H-(1)benzopyrano(6,7,8,ij)quinolizinone-11 in accordance with formula:

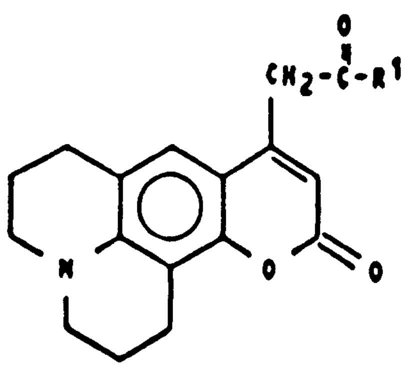

(I)

in which $R^1$ represents:

1) $-NH-(CH_2)_n-R^2$ with n being an integer from 1 to 20 and $R^2$ representing $-N=C=S$ or $NH-CO-CH_2X$ with X representing I, Br or Cl, or

2) $-NH-(CH_2-CH_2-O)_m-CH_2-CH_2-R^3$ with m being an integer from 1 to 30 and $R^3$ representing a group chosen from among those of formula:

-$NH_2$

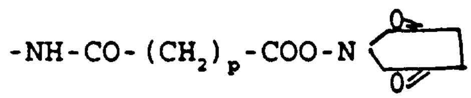

-$NH$-$CO$-$(CH_2)_p$-$CO$-$NH$-$NH_2$,
-$N=C=S$, and
-$NH$-$CO$-$CH_2X$
in which p is an integer from 1 to 10 and X represents Br, Cl or I.

**2.** Derivative of tetrahydro-2,3,6,7,1H,5H,11H-(1)benzopyrano(6,7,8,ij)quinolizinone-11 according to claim 1, characterized in that it complies with formula:

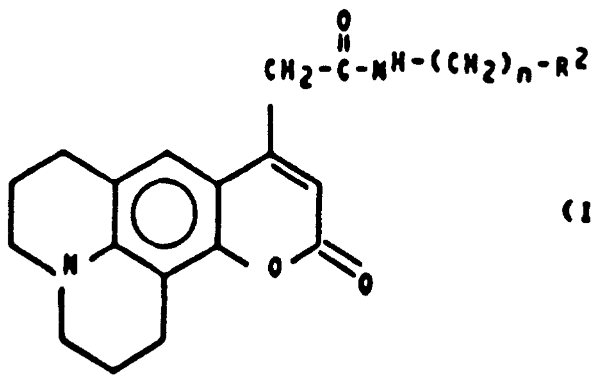

(II)

in which n is an integer from 1 to 20 and $R^2$ represents -$N=C=S$ or $NH$-$CO$-$CH_2X$ with X representing I, Br or Cl.

**3.** Derivative of tetrahydro-2,3,6,7,1H,5H,11H-(1)benzopyrano(6,7,8,ij)quinolizinone-11 according to claim 2, characterized in that n is equal to 4.

**4.** Derivative of tetrahydro-2,3,6,7,1H,5H,11H-(1)benzopyrano(6,7,8,ij)quinolizinone-11 according to claim 1, characterized in that it complies with formula:

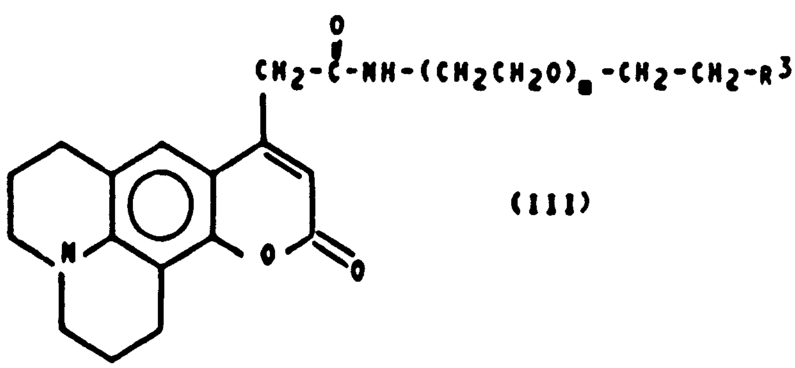

(III)

in which m is an integer from 1 to 30 and $R^3$ is as defined in claim 1.

**5.** Derivative of tetrahydro-2,3,6,7,1H,5H,11H-(1)benzopyrano(6,7,8,ij)quinolizinone-11 according to claim 4, characterized in that m is equal to 5.

6. Derivative of tetrahydro-2,3,6,7,1H,5H,11H-(1)benzopyrano(6,7,8,ij)quinolizinone-11 according to claim 5, characterized in that $R^3$ is $NH_2$.

7. Derivative of tetrahydro-2,3,6,7,1H,5H,11H-(1)benzopyrano(6,7,8,ij)quinolizinone-11 according to claim 5, characterized in that $R^3$ is

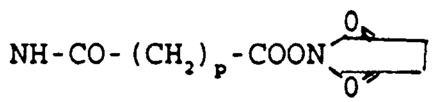

with p being an integer from 1 to 10.

8. Derivative of tetrahydro-2,3,6,7,1H,5H,11H-(1)benzopyrano(6,7,8,ij)quinolizinone-11 according to claim 7, characterized in that p is equal to 2.

9. Derivative of tetrahydro-2,3,6,7,1H,5H,11H-(1)benzopyrano(6,7,8,ij)quinolizinone-11 according to claim 5, characterized in that $R^3$ is

$NH\ CO(CH_2)_pCONH\ NH_2$.

10. Derivative of tetrahydro-2,3,6,7,1H,5H,11H-(1)benzopyrano(6,7,8,ij)quinolizinone-11 according to claim 9, characterized in that p is equal to 2.

11. Process for the detection of a compound having a primary or secondary amino function, characterized in that it consists of reacting the compound to be detected with a derivative in accordance with formula:

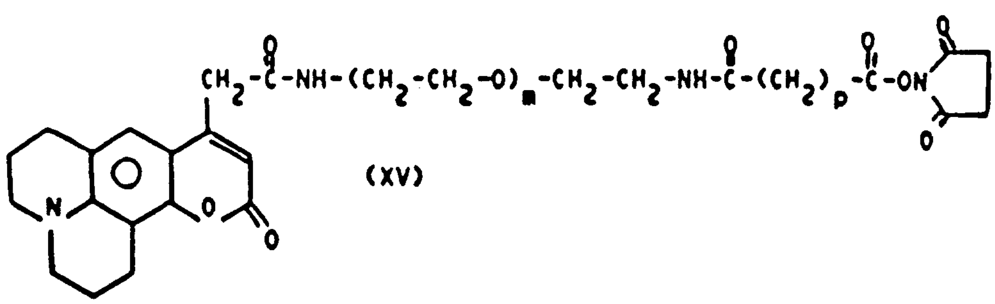

(XV)

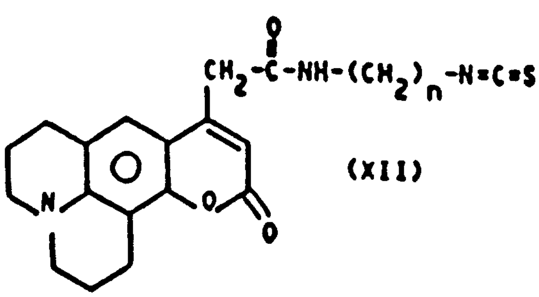

(XII)

or

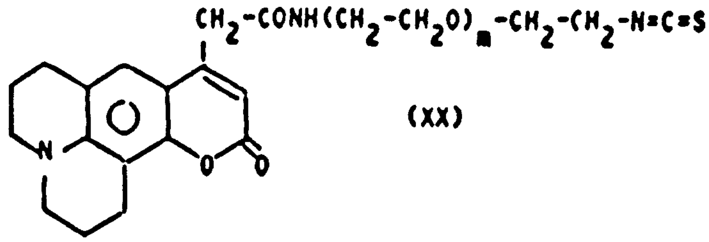

(XX)

in which m is an integer from 1 to 30, n is an integer from 1 to 20 and p is an integer from 1 to 10, in order to form a derivative of the compound to be detected, followed by the detection of said derivative by absorptiometry, fluorimetry or chemiluminescence.

**12.** Process for the detection of a compound having a SH group, characterized in that it consists of reacting the derivative of formula:

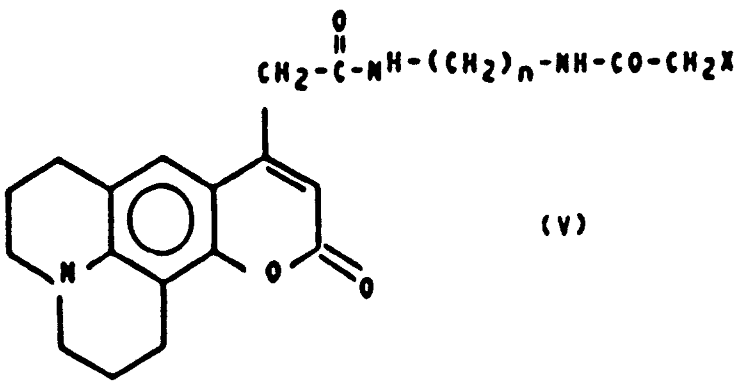

(V)

or

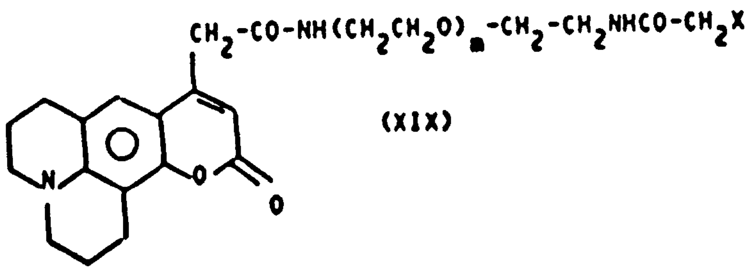

(XIX)

in which m is an integer from 1 to 30, n is an integer from 1 to 20 and X represents I, Br or Cl, to form a derivative of the compound to be detected, and then detecting the said derivative by absorptiometry, fluorimetry or chemiluminescence.

**13.** Process for the detection of a compound having a -COO-group, characterized in that said compound is reacted with a derivative of formula:

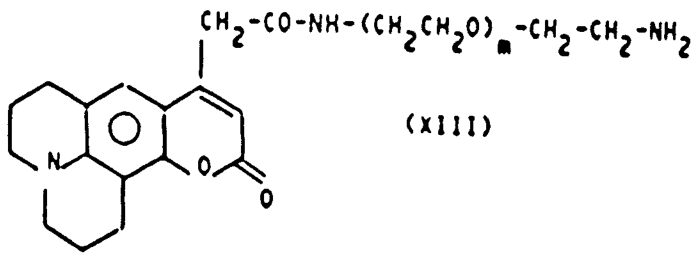

(XIII)

in which m is an integer from 1 to 30, in order to form a derivative of the compound to be detected and in that said derivative is then detected by absorptiometry, fluorimetry or chemiluminescence.

**14.** Process according to any one of the claims 11 to 13, characterized in that detection takes place by chemiluminescence using an oxalic ester and hydrogen peroxide.

**15.** Process according to claim 14, characterized in that the oxalic ester is bis-(trichloro-2,4,6-phenyl)-oxalate or bis-(dinitro-2,4-phenyl)-oxalate.

**Claims for the following Contracting State : ES**

**1.** Process for the preparation of a derivative of tetrahydro-2,3,6,7,1H,5H,11H-(1)benzopyrano(6,7,8,ij) quinolizinone-11 in accordance with the formula:

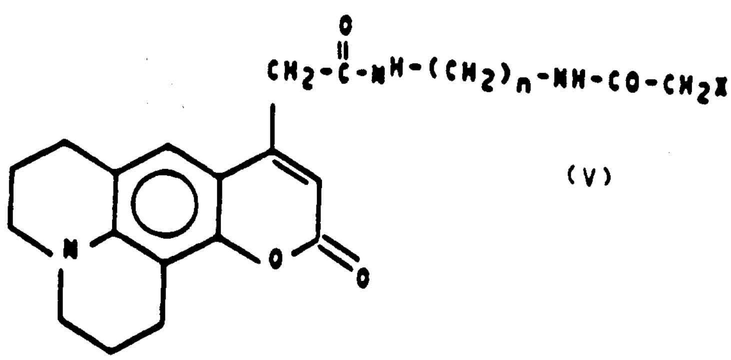

(V)

in which n is an integer from 1 to 20 and X represents I, Br or Cl, characterized in that it comprises reacting a derivative of tetrahydro-2,3,6,7,1H,5H,11H-(1)benzopyrano(6,7,8,ij) quinolizinone-11 of formula:

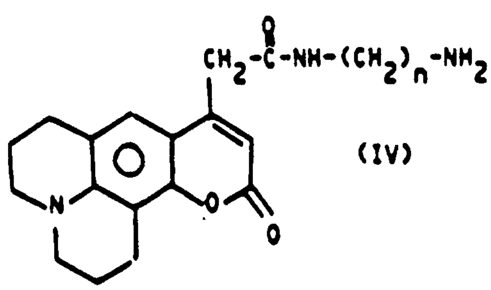

(IV)

with a haloacetic anhydride in the presence of a trialkyl amine.

**2.** Process for the preparation of a derivative of tetrahydro-2,3,6,7,1H,5H,11H-(1)benzopyrano(6,7,8,ij) quinolizinone-11 in accordance with formula:

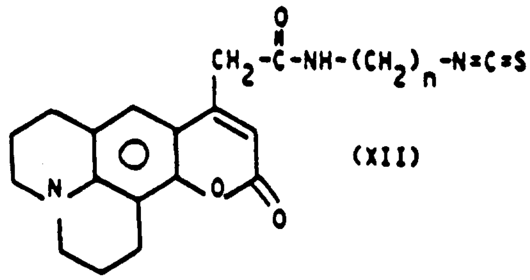

(XII)

in which n is an integer from 1 to 20, characterized in that it consists of reacting a derivative of formula:

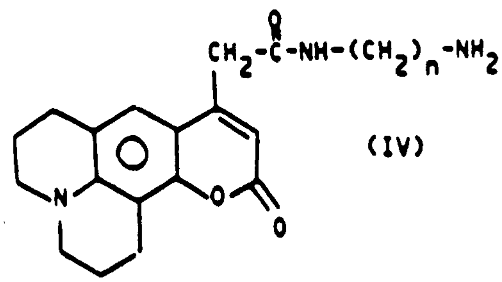

(IV)

with thiocarbonyl diimidazole.

3. Process according to either of the claims 1 and 2, characterized in that the derivative of formula (IV) is prepared by performing the following successive stages:

1) reacting 8-hydroxyjulolidine of formula:

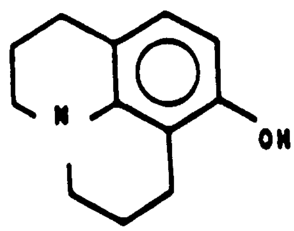

(VI)

with an oxo-3-giutaric acid alkyl ester of formula:

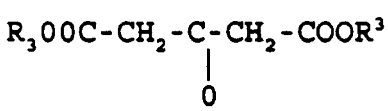

in which $R^3$ is an alkyl radical, in order to form a compound of formula:

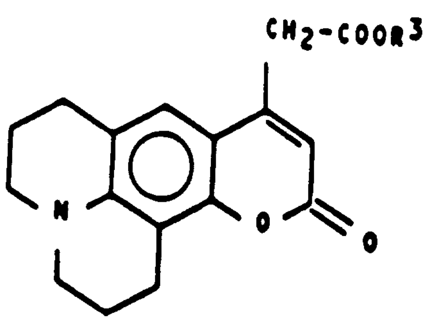

(VII)

2) hydrolyzing the alkyl ester of formula (VII) obtained hereinbefore in order to obtain the acid of formula:

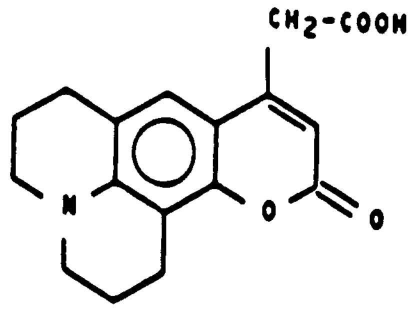

(VIII)

3) reacting the acid of formula (VIII) obtained in this way with the dihydroxysuccinimide oxalate of formula:

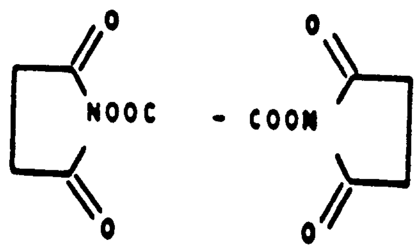

(IX)

or hydroxysuccinimide in the presence of a coupling agent in order to form the derivative of formula:

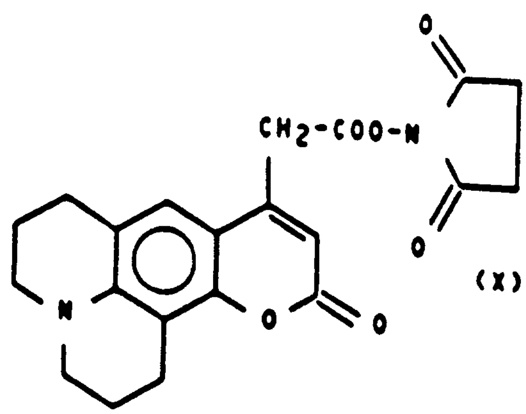

(X)

4) reacting the derivative of formula (X):

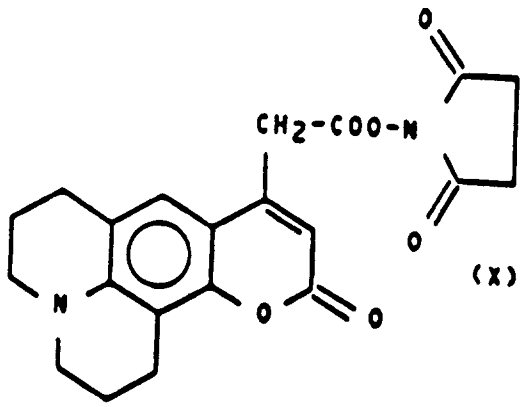

(X)

with a diamino alkane of formula:

$H_2N\text{-}(CH_2)_n\text{-}NH_2$ (XI)

in which n has the meaning given hereinbefore.

**4.** Process for the preparation of a derivative of tetrahydro-2,3,6,7,1H,5H,11H-(1)benzopyrano(6,7,8,ij) quinoiizinone-11 of formula:

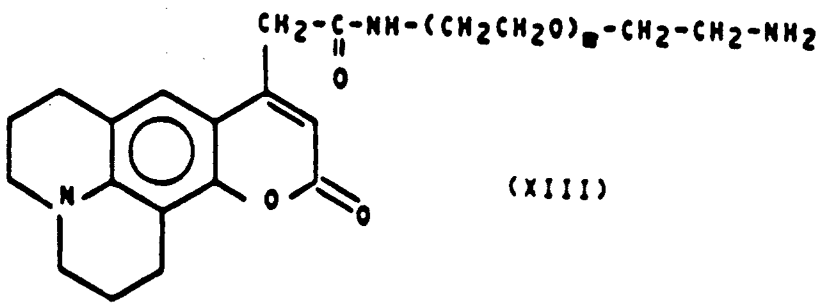

(XIII)

in which m is an integer from 1 to 30, characterized in that it comprises reacting the compound of formula:

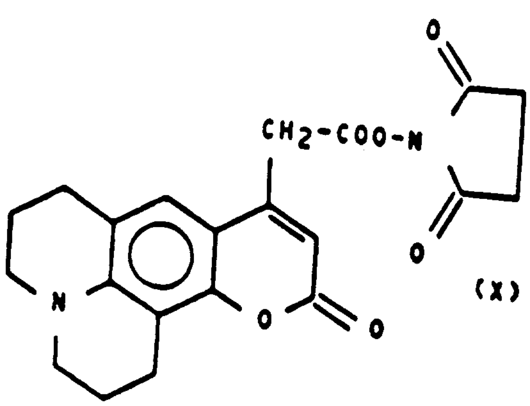

(X)

with an excess of poly(oxy-1,2 ethane diyl) diamine of formula:

$H_2N\text{-}(CH_2CH_2O)_m\text{-}CH_2\text{-}CH_2NH_2$ (XIV)

in which m has the meaning given hereinbefore.

**5.** Process according to claim 4, characterized in that the compound of formula (X) is prepared by performing the following successive stages:

1) reacting 8-hydroxyjulolidine of formula:

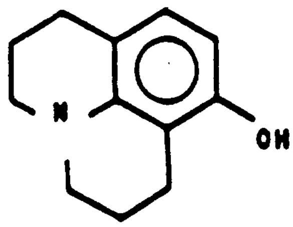

(VI)

with an oxo-3-glutaric acid alkyl ester of formula:

$$R_3OOC-CH_2-\underset{\underset{O}{\|}}{C}-CH_2-COOR^3$$

in which $R^3$ is an alkyl radical, to form a compound of formula:

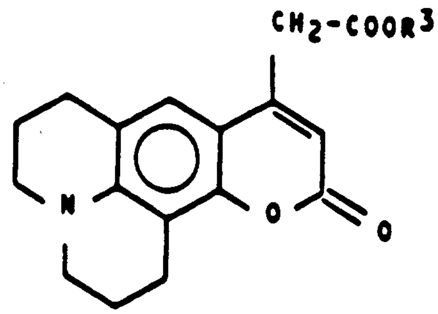

(VII)

2) hydrolyzing the alkyl ester of formula (VII) obtained previously to obtain the acid of formula:

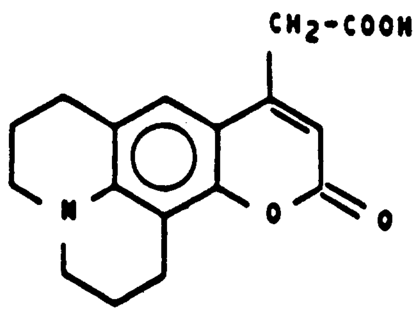

(VIII)

3) reacting the acid of formula (VIII) obtained in this way with the dihydroxysuccinimide oxalate of formula:

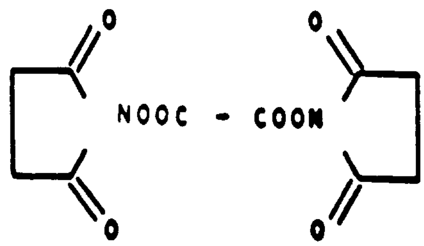

(IX)

to form the derivative of formula:

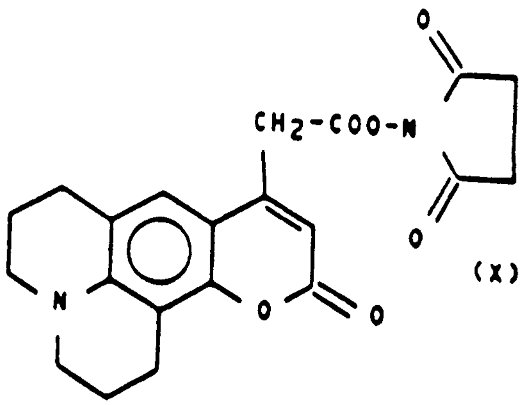

(X)

6. Process for the preparation of a derivative of tetrahydro-2,3,6,7,1H,5H,11H-(1)benzopyrano(6,7,8,ij) quinolizinone-11

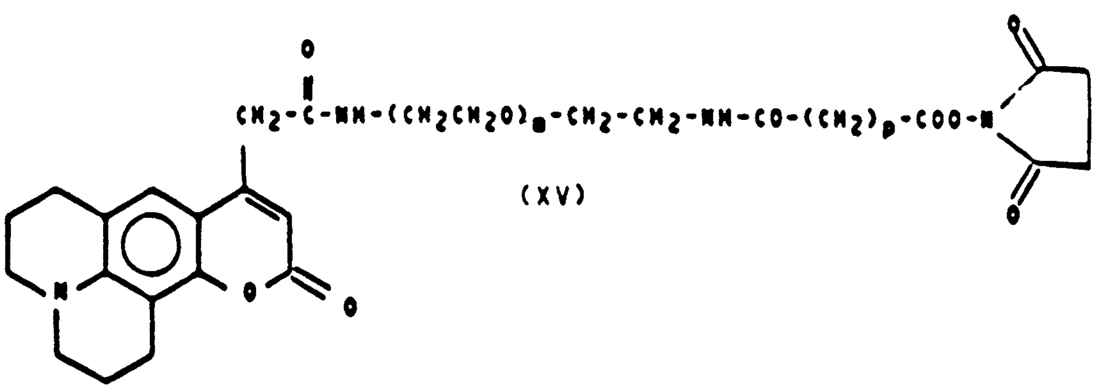

(XV)

in which m is an integer from 1 to 30 and p an integer from 1 to 10, characterized in that it comprises:

1) reacting a compound of formula:

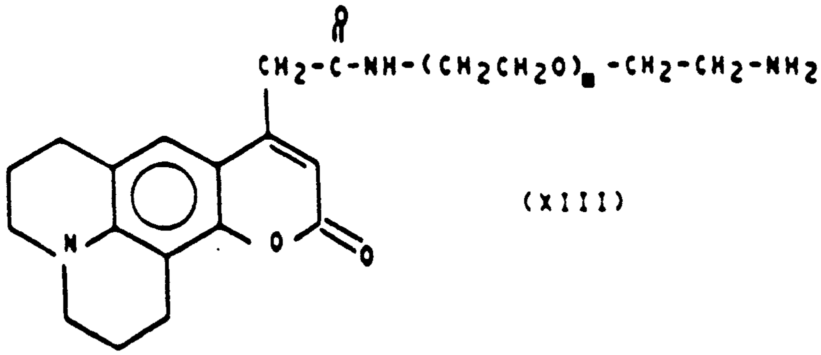

(XIII)

in which m has the meaning given hereinbefore with a diacid anhydride of formula:

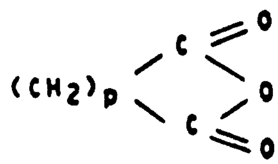

(XVI)

to obtain an acid of formula:

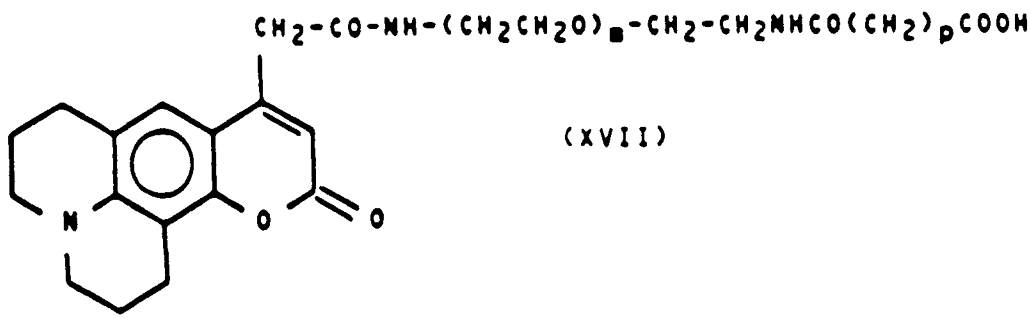

(XVII)

2) then reacting the acid of formula (XVII) with hydroxysuccinimide in the presence of a coupling agent to form the derivative of formula (XV).

**7.** Process for the preparation of a derivative of tetrahydro-2,3,6,7,1H,5H,11H-(1)benzopyrano(6,7,8,ij) quinolizinone-11 in accordance with the formula:

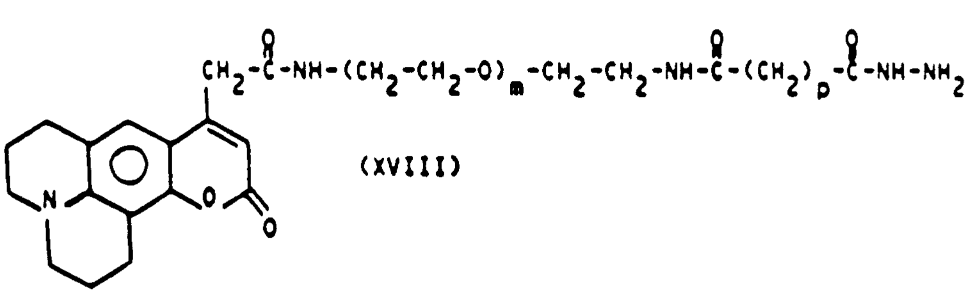

(XVIII)

in which m is an integer from 1 to 30 and p an integer from 1 to 10, characterized in that it comprises reacting a derivative of tetrahydro-2,3,6,7,1H,5H,11H-(1)benzopyrano(6,7,8,ij) quinolizinone-11 of formula:

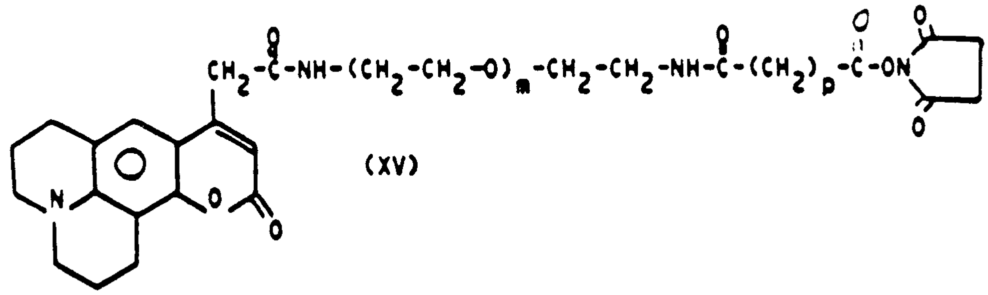

(XV)

with a hydrazine hydrate excess.

**8.** Process according to claim 7, characterized in that the derivative of formula (XV) is prepared by the process of claim 6.

**9.** Process for the preparation of a derivative of tetrahydro-2,3,6,7,1H,5H,11H-(1)benzopyrano(6,7,8,ij) quinolizinone-11 of formula:

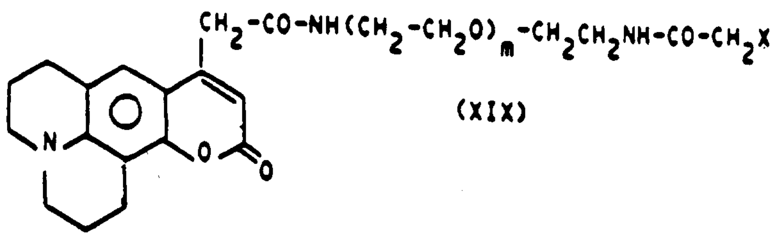

(XIX)

in which m is an integer from 1 to 30, characterized in that it comprises reacting a derivative of tetrahydro-2,3,6,7,1H,5H,11H-(1)benzopyrano(6,7,8,ij) quinolizinone-11 of formula:

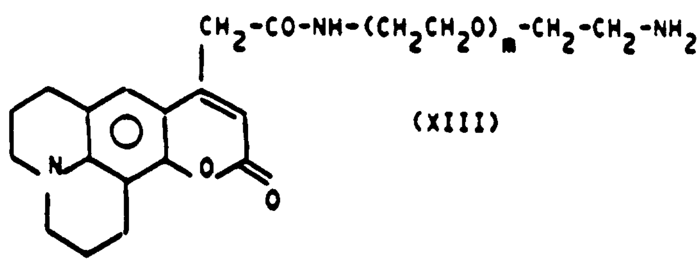

(XIII)

with a haloacetic anhydride in the presence of a trialkyl amine.

**10.** Process for the preparation of a derivative of tetrahydro-2,3,6,7,1H,5H,11H-(1)benzopyrano(6,7,8,ij) quinolizinone-11 in accordance with the formula:

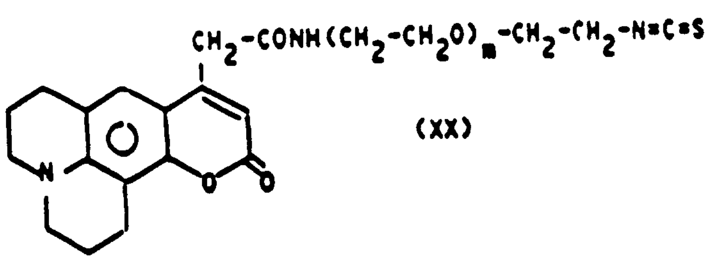

(XX)

in which m is an integer from 1 to 30, characterized in that it comprises reacting a derivative of

tetrahydro-2,3,6,7,1H,5H,11H-(1)benzopyrano(6,7,8,ij) quinolizinone-11 of formula:

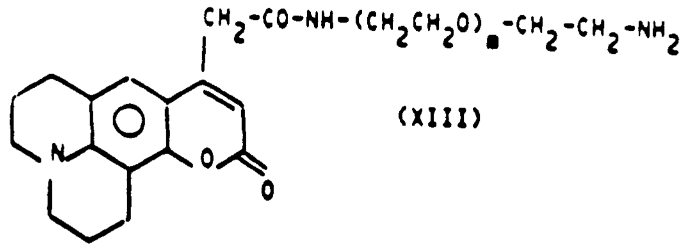

(XIII)

with thiocarbonyl diimidazole.

**11.** Process according to either of the claims 9 and 10, characterized in that the derivative of formula (XIII) is prepared by the process of claim 4.

**12.** Process for the detection of a compound having a primary or secondary amine group, characterized in that said compound is reacted with a derivative of formula:

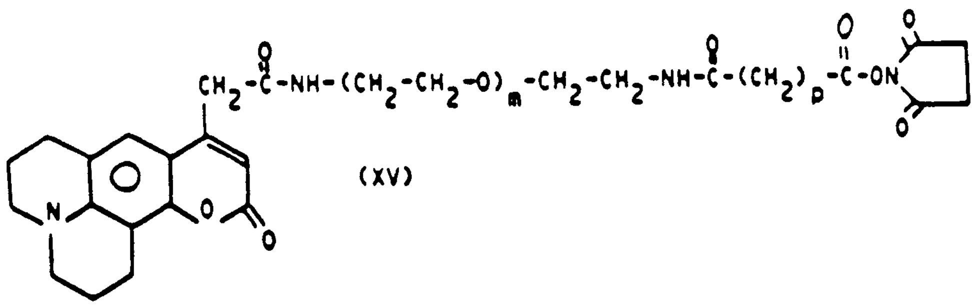

(XV)

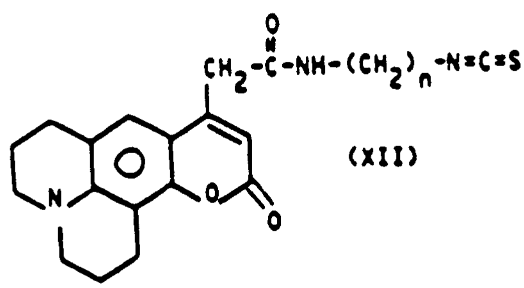

(XII)

or

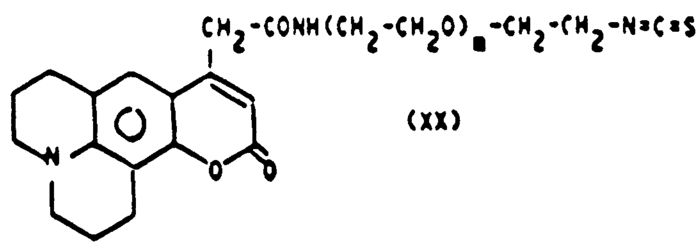

(XX)

in which m is an integer from 1 to 30, n is an integer from 1 to 20 and p is an integer from 1 to 10, in order to form a derivative of the compound to be detected, and then detecting said compound by absorptiometry, fluorimetry and chemiluminescence.

**13.** Process for the detection of a compound having a SH group, characterized in that it comprises reacting the derivative of formula:

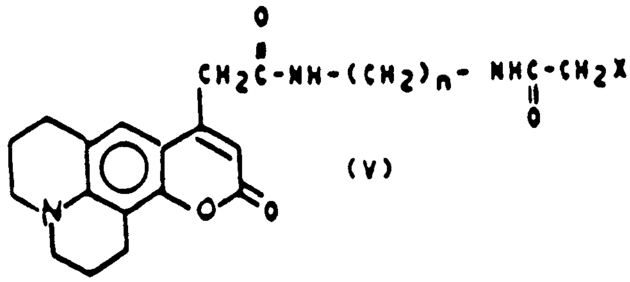

(V)

or

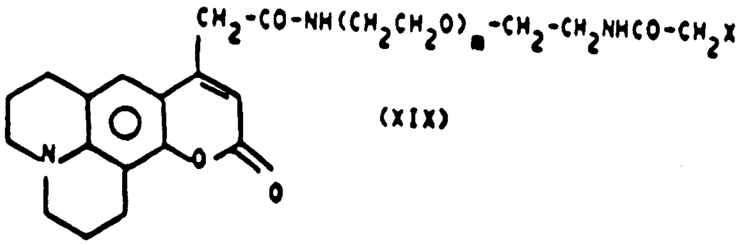

(XIX)

in which m is an integer from 1 to 30, n an integer from 1 to 20 and X represents I, Br or Cl, in order to form a derivative of the compound to be detected and then detecting said derivative by absorptiometry, fluorimetry or chemiluminescence.

**14.** Process for the detection of a compound having a -COO-group, characterized in that said compound is reacted with a derivative of formula:

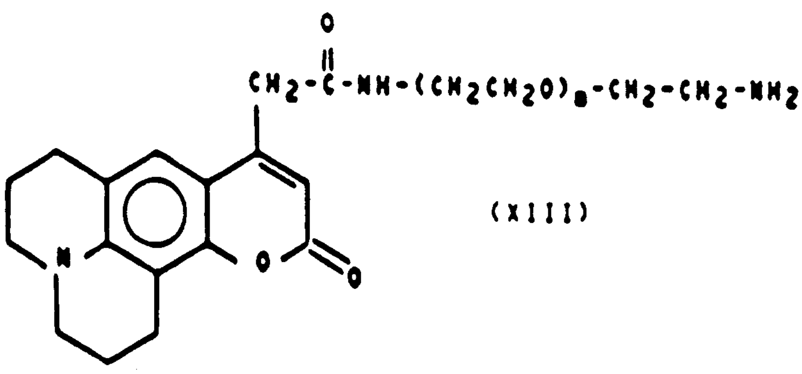

(XIII)

in which m is an integer from 1 to 30 in order to form a derivative of the compound to be detected and in that said derivative is then detected by absorptiometry, fluorimetry or chemiluminescence.

**15.** Process according to any one of the claims 12 to 14, characterized in that the detection takes place by chemiluminescence using an oxalic ester or hydrogen peroxide.

**16.** Process according to claim 15, characterized in that the oxalic ester is bis-(trichloro-2,4,6 phenyl)-oxalate or bis-(dinitro 2,4 phenyl)oxalate.

**17.** Process according to claim 1, characterized in that n is equal to 4 and X represents I.

**18.** Process according to claim 4, characterized in that m is equal to 5.

**19.** Process according to claim 6, characterized in that m is equal to 5 and p equal to 2.

**20.** Process according to claim 7, characterized in that m is equal to 5 and p is equal to 2.

**21.** Process according to claim 2, characterized in that n is equal to 4.

**Patentansprüche**
**Patentansprüche für folgende Vertragsstaaten : AT, BE, CH, DE, DK, FR, GB, GR, IT, LI, LU, NL, SE**

**1.** Derivat des Tetrahydro-2,3,6,7,1H,5H,11H-Benzopyrano-(6,7,8,ij)-Chinolizinon-11 mit der Formel:

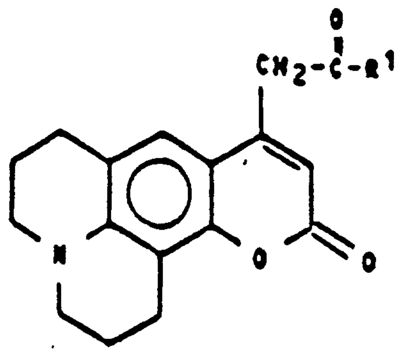

(I)

in der $R^1$ der folgenden Formel entspricht:

1°) $-NH-(CH_2)_n-R^2$, wobei n eine ganze Zahl zwischen 1 und 20 ist und $R^2$ für die Formeln $-N=C=S$ oder $NH-CO-CH_2X$ steht, in denen X für die Elemente I, Br oder Cl steht, oder

2°) $-NH-(CH_2-CH_2-O)m$ $-CH_2-CH_2-R^3$, wobei m eine ganze Zahl zwischen 1 und 30 ist und $R^3$ eine Gruppe darstellt, die aus den folgenden Formelgruppen ausgewählt ist:

$-NH_2$

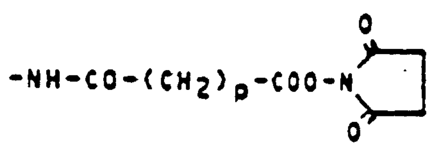

$-NH-CO-(CH_2)_p-CO-NH-NH_2$,

$-N=C=S$, et

$-NH-CO-CH_2X$

wobei p eine ganze Zahl zwischen 1 und 10 ist und X für die Elemente Br, Cl oder I steht.

**2.** Derivat des Tetrahydro-2,3,6,7,1H,5H,11H-(1)-Benzopyrano-(6,7,8,ij)-Chinolizinon-11 nach Anspruch 1, dadurch gekennzeichnet, daß es der Formel

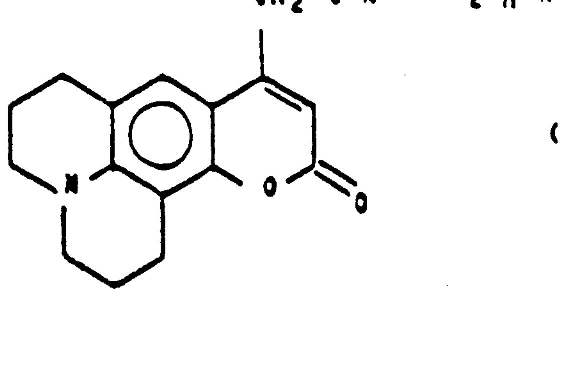

(II)

entspricht, wobei n eine ganze Zahl zwischen 1 und 20 ist und $R^2$ für die Formeln N=C=S oder NH-CO-$CH_2X$ steht, in denen X für die Elemente I, Br oder Cl steht.

3. Derivat des Tetrahydro-2,3,6,7,1H,5H,11H-(1)-Benzopyrano-(6,7,8,ij)-Chinolizinon-11 nach Anspruch 2, dadurch gekennzeichnet, daß n gleich 4 ist.

4. Derivat des Tetrahydro-2,3,6,7,1H,5H,11H-(1)-Benzopyrano-(6,7,8,ij)-Chinolizinon-11 nach Anspruch 1, dadurch gekennzeichnet, daß es der Formel:

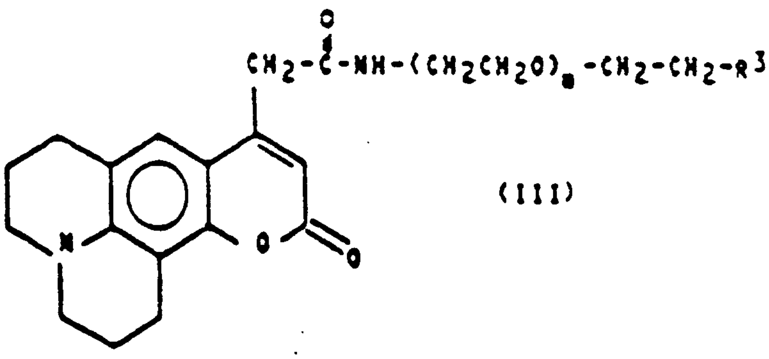

(III)

entspricht, wobei m eine ganze Zahl zwischen 1 und 30 ist und $R^3$ für die in Anspruch 1 definierten Formelgruppen steht.

5. Derivat des Tetrahydro-2,3,6,7,1H,5H,11H-(1)-Benzopyrano-(6,7,8,ij)-Chinolizinon-11 nach Anspruch 4, dadurch gekennzeichnet, daß m gleich 5 ist.

6. Derivat des Tetrahydro-2,3,6,7,1H,5H,11H-(1)-Benzopyrano-(6,7,8,ij)-Chinolizinon-11 nach Anspruch 5, dadurch gekennzeichnet, daß $R^3$ die Formel $NH_2$ hat.

7. Derivat des Tetrahydro-2,3,6,7,1H,5H,11H-(1)-Benzopyrano-(6,7,8,ij)-Chinolizinon-11 nach Anspruch 5, dadurch gekennzeichnet, daß $R^3$ der Formel

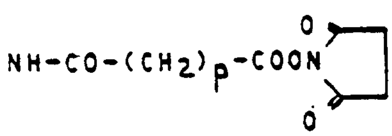

entspricht, wobei p eine ganze Zahl zwischen 1 und 10 ist.

8. Derivat des Tetrahydrano-2,3,6,7,1H,5H,11H-(1)-Benzopyrano-(6,7,8,ij)-Chinolizinon-11 nach Anspruch 7, dadurch gekennzeichnet, daß p gleich 2 ist.

9. Derivat des Tetrahydrano-2,3,6,7,1H,5H,11H-(1)-Benzopyrano-(6,7,8,ij)-Chinolizinon-11 nach Anspruch 5, dadurch gekennzeichnet, daß $R^3$ der Formel NH $CO(CH_2)_pCONH$ $NH_2$ entspricht.

10. Derivat des Tetrahydrano-2,3,6,7,1H,5H,11H-(1)-Benzopyrano-(6,7,8,ij)-Chinolizinon-11 nach Anspruch 9, dadurch gekennzeichnet, daß p gleich 2 ist.

11. Verfahren zum Nachweis einer Verbindung, die eine primäre oder sekundäre Aminfunktion hat, dadurch gekennzeichnet, daß man die nachzuweisende Verbindung mit einem Derivat reagieren läßt, das den folgenden Formeln entspricht:

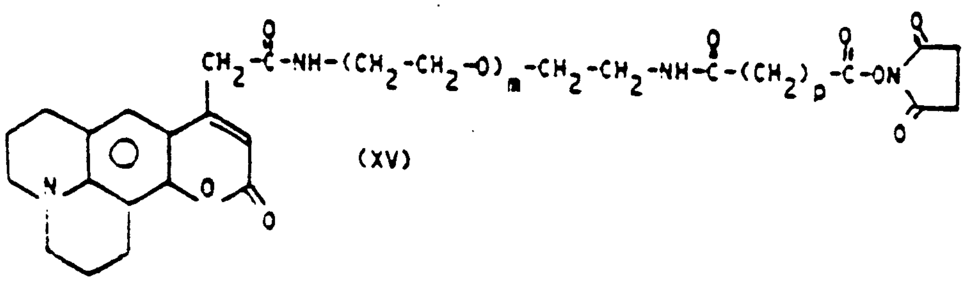

(XV)

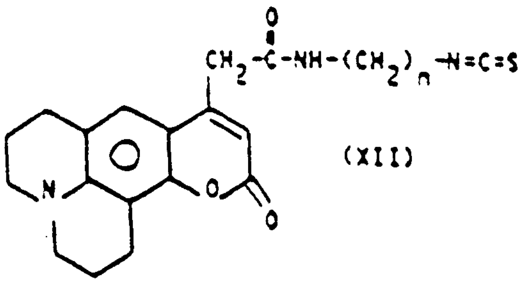

(XII)

oder

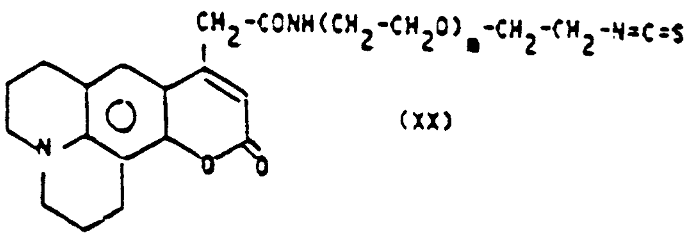

(XX)

wobei m eine ganze Zahl zwischen 1 und 30, n eine ganze Zahl zwischen 1 und 20 und p eine ganze Zahl zwischen 1 und 10 ist, und daß ein Derivat der nachzuweisenden Verbindung gebildet wird, um anschließend das Derivat durch Absorptionsmessung, Fluorometrie oder Chemolumineszenz nachzuweisen.

**12.** Verfahren zum Nachweis einer Verbindung, die eine SH-Gruppe enthält, dadurch gekennzeichnet, daß man daß Derivat mit der Formel:

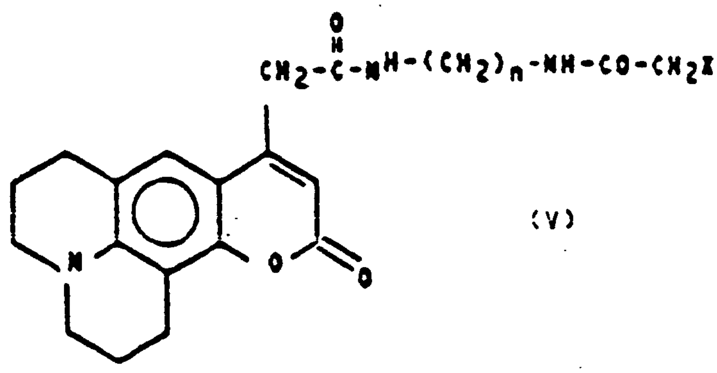

(V)

oder

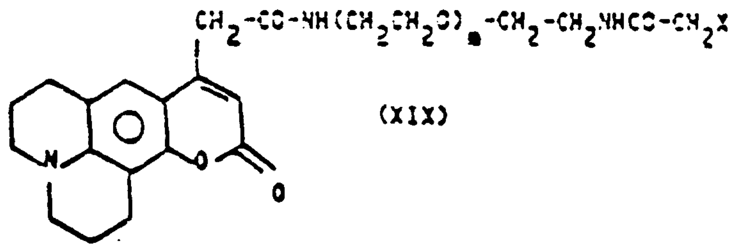

(XIX)

reagieren läßt, wobei m eine ganze Zahl zwischen 1 und 30, n eine ganze Zahl zwischen 1 und 20 ist und X für die Elemente I, Br oder Cl steht, um ein Derivat der nachzuweisenden Verbindung zu bilden, um anschließend das Derivat durch Absorptionsmessung, Fluorometrie oder Chemolumineszenz nachzuweisen.

**13.** Verfahren zum Nachweis einer Verbindung, die eine COO-Gruppe enthält, dadurch gekennzeichnet, daß man die Verbindung mit einem Derivat mit der Formel:

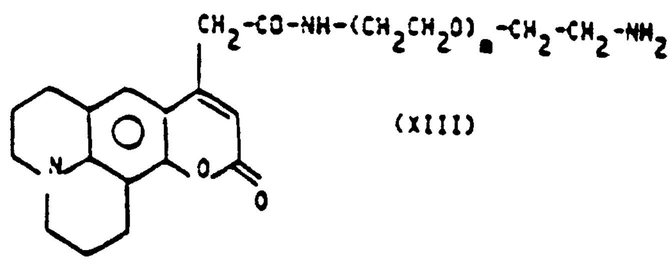

(XIII)

reagieren läßt, wobei m eine ganze Zahl zwischen 1 und 30 ist, um ein Derivat der nachzuweisenden Verbindung zu bilden, und das dadurch gekennzeichnet ist, daß anschließend dieses Derivat durch Absorptionsmessung, Fluorometrie oder Chemolumineszenz nachgewiesen wird.

**14.** Verfahren nach einem der Ansprüche 11 bis 13, dadurch gekennzeichnet, daß das der Nachweis durch Chemolumineszenz unter Verwendung eines Oxalsäureesters und von Wasserstaffperoxid geführt wird.

**15.** Verfahren nach Anspruch 14, dadurch gekennzeichnet, daß als Oxalsäureester bis-(Trichloro-2,4,6-Phenyl)-Oxalat oder bis-(Dinitro-2,4-Phenyl)-Oxalat verwendet wird.

**Patentansprüche für folgenden Vertragsstaat : ES**

1. Verfahren zur Herstellung eines Derivats des Tetrahydro-2,3,6,7,1H,5H,11H-(1)-Benzopyrano-(6,7,8,ij)-Chinolizinon-11 mit der Formel:

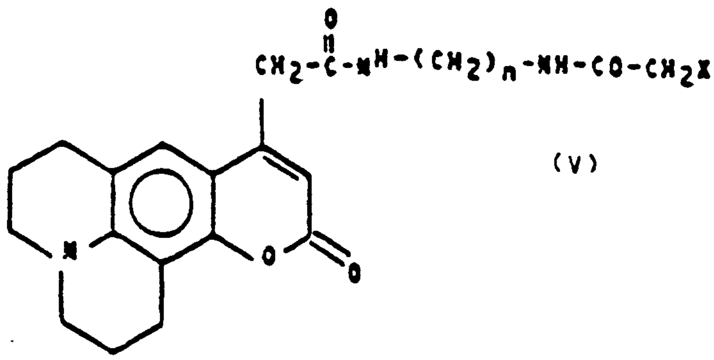

(V)

wobei n eine ganze Zahl zwischen 1 und 20 ist und X für die Elemente I, Br oder Cl steht, dadurch gekennzeichnet, daß man ein Derivat des Tetrahydro-2,3,6,7,1H,5H,11H-(1)-Benzopyrano-(6,7,8,ij)-Chinolizinon-11 mit der Formel:

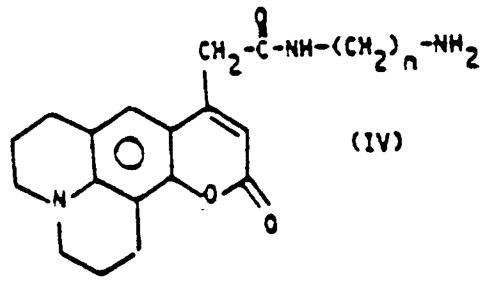

(IV)

mit einem Wasserstoffanhydrid der Essigsäure in Trialkylamin reagieren läßt.

2. Verfahren zur Herstellung eines Derivats des Tetrahydro-2,3,6,7,1H,5H,11H-(1)-Benzopyrano-(6,7,8,ij)-Chinolizinon-11 mit der Formel:

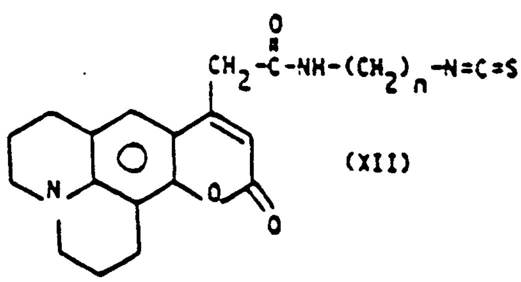

(XII)

, wobei n eine ganze Zahl zwischen 1 und 20 ist, dadurch gekennzeichnet, daß man ein Derivat mit der Formel:

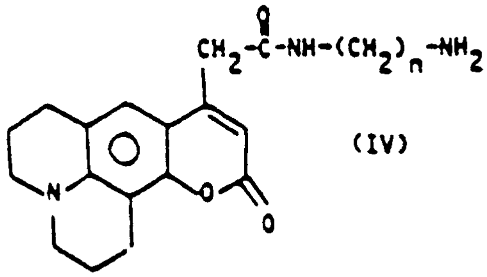

(IV)

mit Thiokarbonyl-Diimidazol reagieren läßt.

**3.** Verfahren nach einem der Ansprüche 1 und 2, dadurch gekennzeichnet, daß ein Derivat mit der Formel (IV) hergestellt wird, indem man nacheinander die folgenden Arbeitsschritte durchführt:
1°) Das 8-Hydroxyjulolidin mit der Formel:

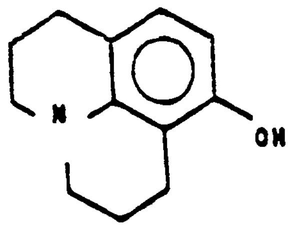

(VI)

wird mit einem Alkylester der Oxo-3-Glutarsäure mit der Formel:

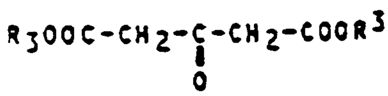

zur Reaktion gebracht, wobei $R^3$ ein Alkylradikal ist, mit dem eine Verbindung mit der Formel:

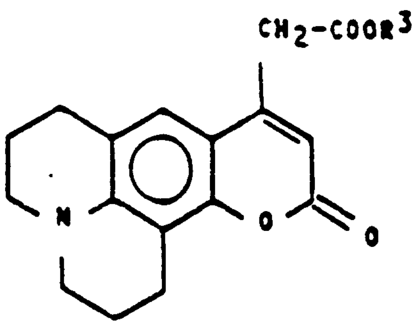

(VII)

gebildet wird.
2°) Der im vorangegangen Arbeitsschritt erhaltene Alkylester wird hydrolysiert, um die Säure mit der Formel:

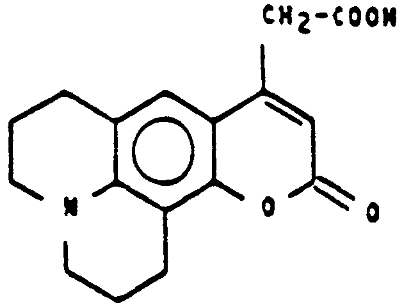

(VIII)

zu erhalten.

3°) Die so entstandene Säure mit der Formel (VIII) läßt man mit dem Oxalat des Dihydroxysuccinimid mit der Formel:

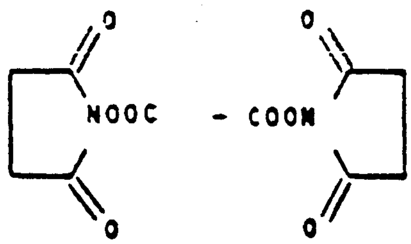

(IX)

oder

das Hydroxysuccinimid unter Zusatz einer Kupplersubstanz reagieren, um das Derivat mit der Formel:

(Formel X) zu bilden.

4°) Das Derivat mit der Formel

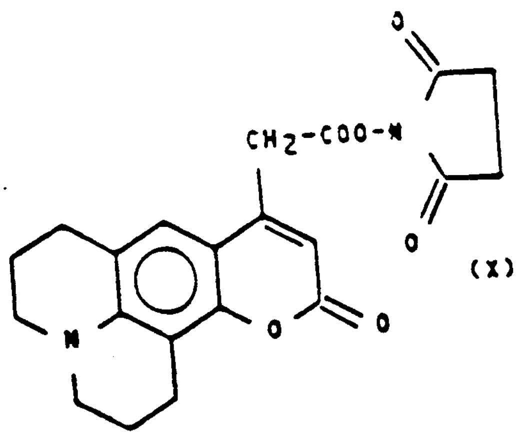

(X)

läßt man mit einem Diaminoalkan mit der Formel

$H_2N-(CH_2)_n-NH_2$ (XI)

reagieren, wobei n die o.g. Bedeutung hat.

**4.** Verfahren zur Herstellung eines Derivats des Tetrahydro-2,3,6,7,1H,5H,11H-(1)-Benzopyrano-(6,7,8,ij)-Chinolizinon-11 mit der Formel:

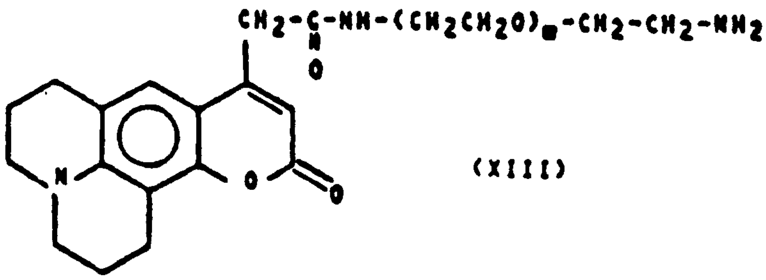

(XIII)

, wobei m eine ganze Zahl zwischen 1 und 30 ist, dadurch gekennzeichnet, daß man die Verbindung mit der Formel:

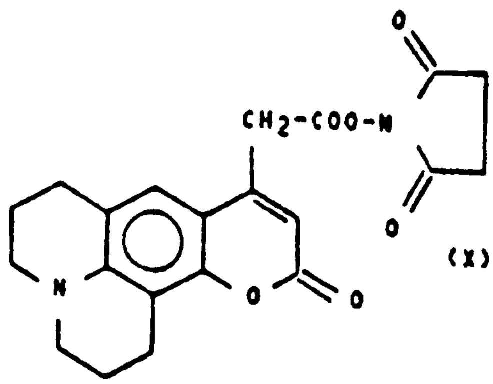

(X)

mit einer überschüssigen Menge an Poly(oxy-1,2-Äthan-diyl)-Diamin mit der Formel:

$H_2N-(CH_2CH_2O)_m-CH_2-CH_2NH_2$ (XIV)

reagieren läßt, wobei m die o.g. Bedeutung hat.

**5.** Verfahren nach Anspruch 4, dadurch gekennzeichnet, daß die Verbindung mit der Formel (X) hergestellt wird, indem man nacheinander die folgenden Arbeitsschritte ausführt:
1°) Das 8-Hydroxyjulolidin mit der Formel:

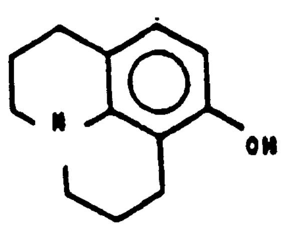

(VI)

läßt man mit einem Alkylester der Oxo-3-Glutarsäure mit der Formel:

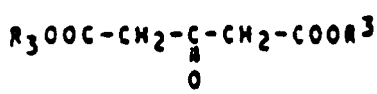

reagieren, wobei $R^3$ ein Alkylradikal ist, um eine Verbindung mit der Formel:

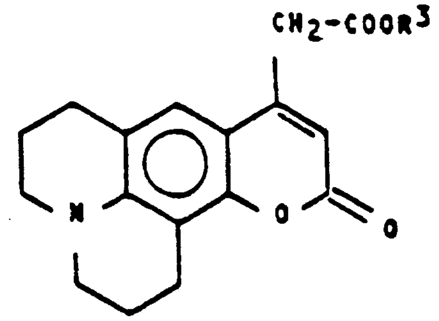

(VII)

zu bilden.

2°) Der im vorangegangenen Arbeitsschritt erhaltene Alkylester mit der Formel (VII) wird hydrolysiert, um die Säure mit der Formel:

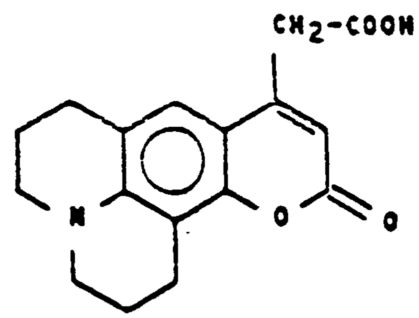

(VIII)

zu erhalten.

3°) Die so erhaltene Säure mit der Formel (VIII) läßt man mit dem Oxalat des Dihydroxysuccinimid mit der Formel:

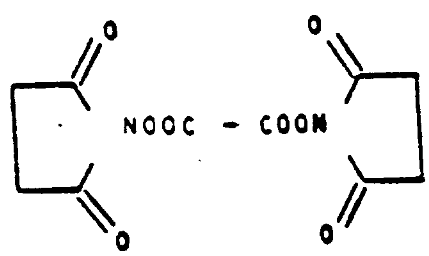

(IX)

reagieren, um das Derivat mit der Formel

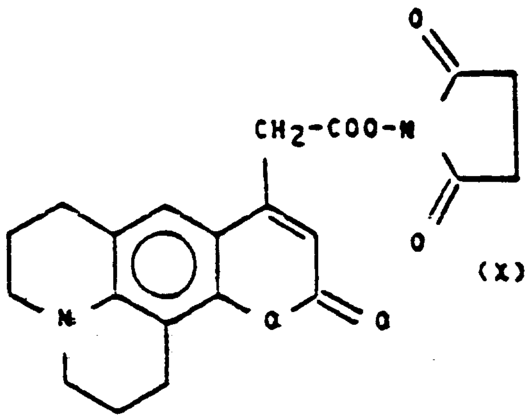

(X)

**6.** Verfahren zur Herstellung eines Derivats des Tetrahydro-2,3,6,7,1H,5H,11H-(1)-Benzopyrano-(6,7,8,ij)-Chinolizinon-11

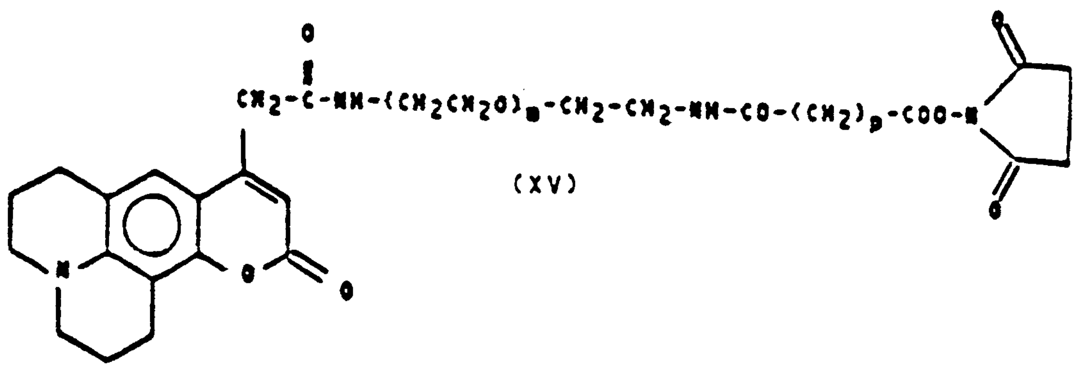

(XV)

, bei dem m eine ganze Zahl zwischen 1 und 30 und p eine ganze Zahl zwischen 1 und 10 ist, dadurch gekennzeichnet, daß:

1°) man eine Verbindung mit der Formel

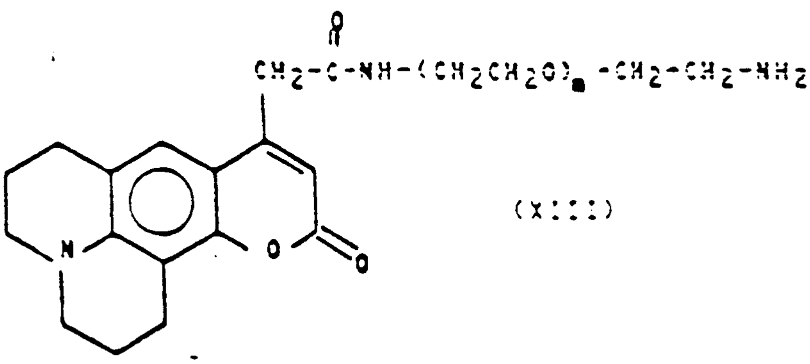

(XIII)

, bei der m die o.g. Bedeutung hat, mit einem zweisäurigen Anhydrid mit der Formel

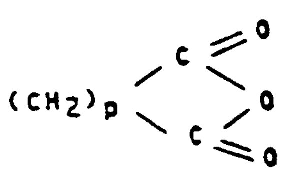

(XVI)

reagieren läßt, um eine Säure mit der Formel:

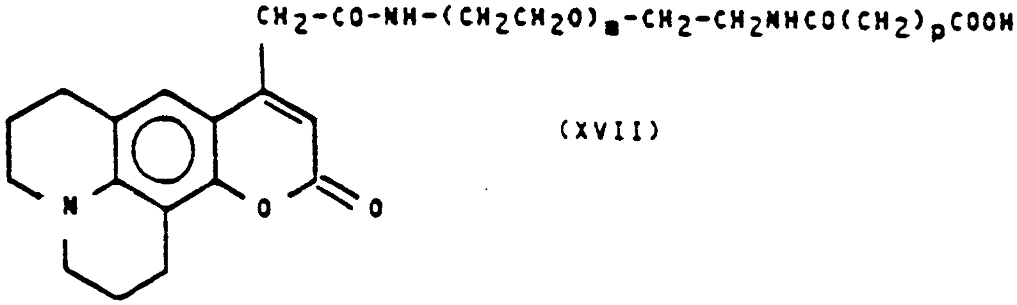

(XVII)

zu erhalten.

2°) man anschließend die Säure mit der Formel (XVII) mit dem Hydroxysuccinimid unter Zusatz einer Kupplersubstanz reagieren läßt, um das Derivat mit der Formel (XV) zu bilden.

**7.** Verfahren zur Herstellung eines Derivats des Tetrahydro-2,3,6,7,1H,5H,11H-(1)-Benzopyrano-(6,7,8,ij)-Chinolizinon-11, das der Formel:

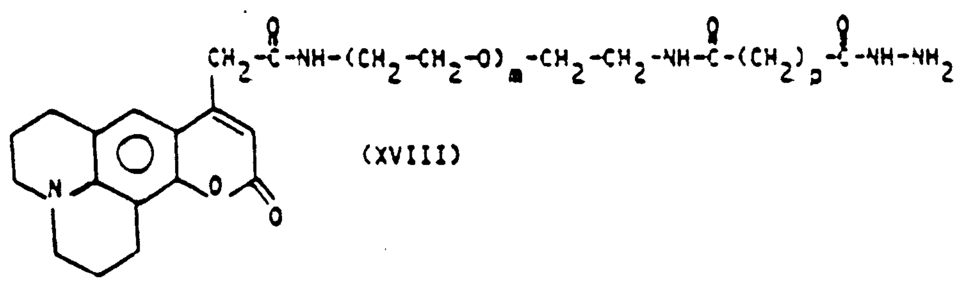

(XVIII)

entspricht, wobei m eine ganze Zahl zwischen 1 und 30 und p eine ganze Zahl zwischen 1 und 10 ist, dadurch gekennzeichnet, daß man ein Derivat des Tetrahydro-2,3,6,7,1H,5H,11H-(1)-Benzopyrano-(6,7,8,ij)-Chinolizinon-11 mit der Formel:

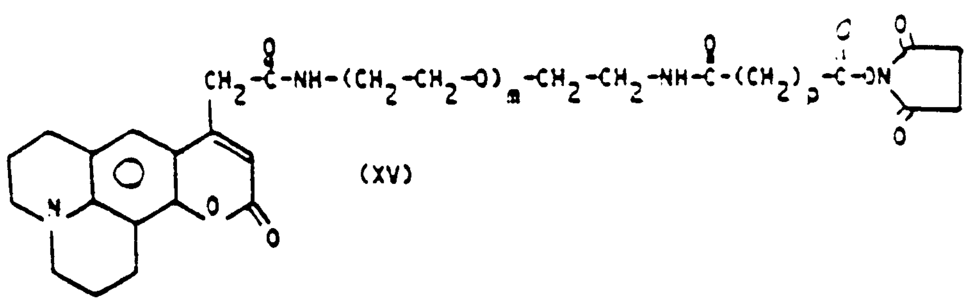

(XV)

mit einer überschüssigen Menge an Hydrazinhydrat reagieren läßt.

**8.** Verfahren nach Anspruch 7, dadurch gekennzeichnet, daß das Derivat mit der Formel (XV) durch das Verfahren nach Anspruch 6 hergestellt wird.

**9.** Verfahren zur Herstellung eines Derivats des Tetrahydro-2,3,6,7,1H,5H,11H-(1)-Benzopyrano-(6,7,8,ij)-Chinolizinon-11 mit der Formel:

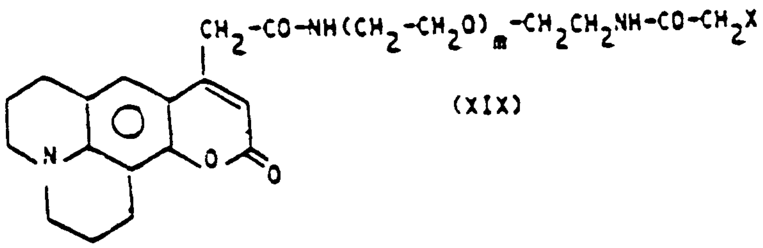

(XIX)

, wobei m eine ganze Zahl zwischen 1 und 30 ist, dadurch gekennzeichnet, daß man ein Derivat des Tetrahydro-2,3,6,7,1H,5H,11H-(1)-Benzopyrano-(6,7,8,ij)-Chinolizinon-11 mit der Formel:

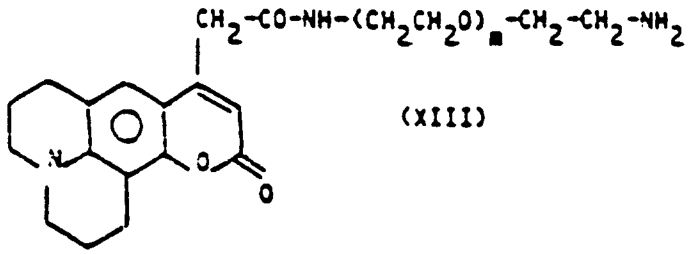

(XIII)

mit einem Wasserstoffanhydrid der Essigsäure in Trialkylamin reagieren läßt.

**10.** Verfahren zur Herstellung eines Derivats des Tetrahydro-2,3,6,7,1H,5H,11H-(1)-Benzopyrano-(6,7,8,ij)-Chinolizinon-11, das der Formel:

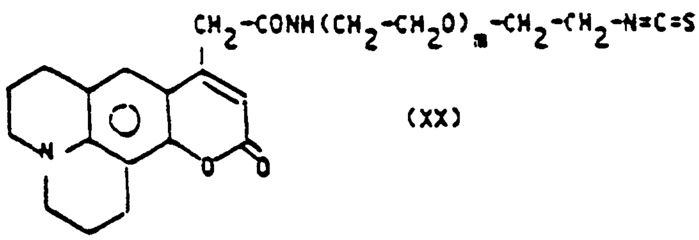

(XX)

entspricht, wobei m eine ganze Zahl zwischen 1 und 30 ist, dadurch gekennzeichnet, daß man ein Derivat des Tetrahydro-2,3,6,7,1H,5H,11H-(1)-Benzopyrano-(6,7,8,ij)-Chinolizinon-11 mit der Formel:

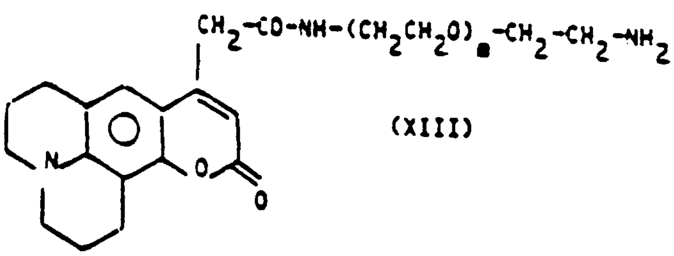

(XIII)

mit mit dem Thiokarbonyldiimidazol reagieren läßt.

**11.** Verfahren nach einem der Ansprüche 9 und 10, dadurch gekennzeichnet, daß das Derivat mit der Formel (XIII) nach dem Verfahren von Anspruch 4 hergestellt wird.

**12.** Verfahren zum Nachweis einer Verbindung die eine primäre oder sekundäre Amingruppe enthält, dadurch gekennzeichnet, daß man diese Verbindung mit einem Derivat mit der Formel:

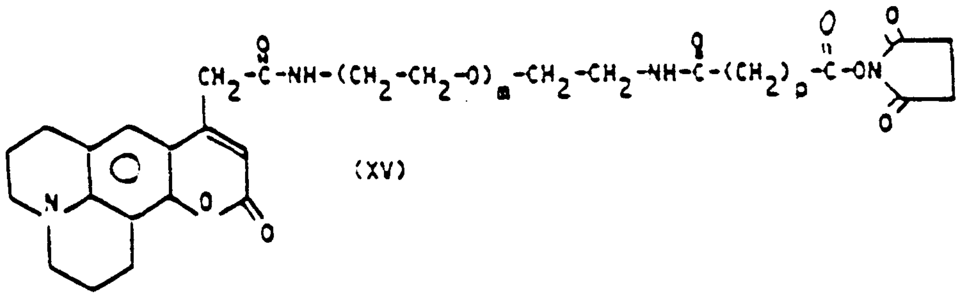

(XV)

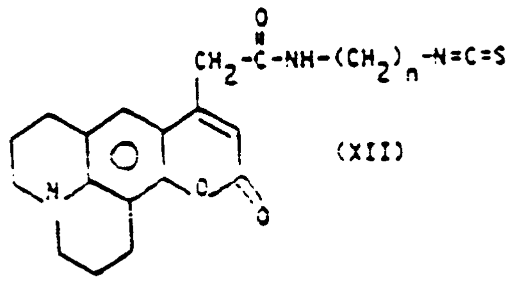

(XII)

oder

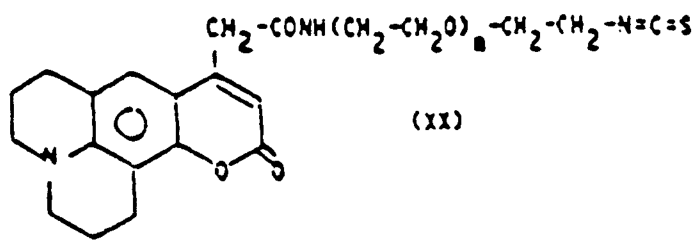

(XX)

reagieren läßt, wobei m eine ganze Zahl zwischen 1 und 30, n eine ganze Zahl zwischen 1 und 20 und p eine ganze Zahl zwischen 1 und 10 ist, um ein Derivat der nachzuweisenden Verbindung zu bilden und um anschließend das Derivat durch Absorptionsmessung, Fluorometrie oder Chemolumineszenz nachzuweisen.

**13.** Verfahren zum Nachweis einer Verbindung, die eine SH-Gruppe enthält, dadurch gekennzeichnet, daß man das Derivat mit der Formel:

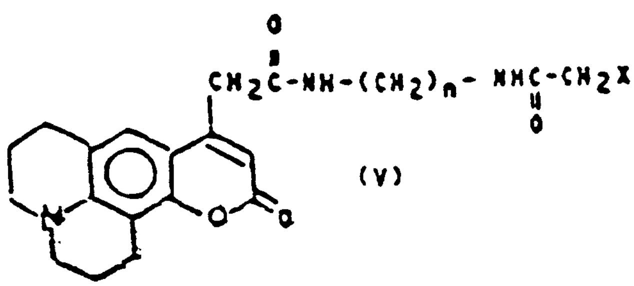

(V)

oder

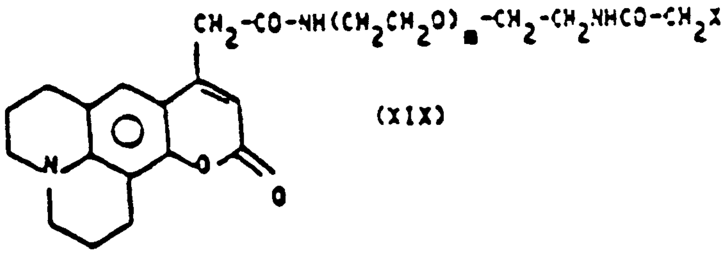

(XIX)

reagieren läßt, bei denen m eine ganze Zahl zwischen 1 und 30 und n eine ganze Zahl zwischen 1 und 20 ist und X für die Elemente I, Br, Cl steht, um ein Derivat der nachzuweisenden Verbindung zu bilden, um das Derivat anschließend durch Absorptionsmessung, Fluorometrie oder Chemolumineszenz nachgewiesen wird.

**14.** Verfahren zum Nachweis einer Verbindung, die eine COO-Gruppe enthält, dadurch gekennzeichnet, daß man diese Verbindung mit einem Derivat mit der Formel:

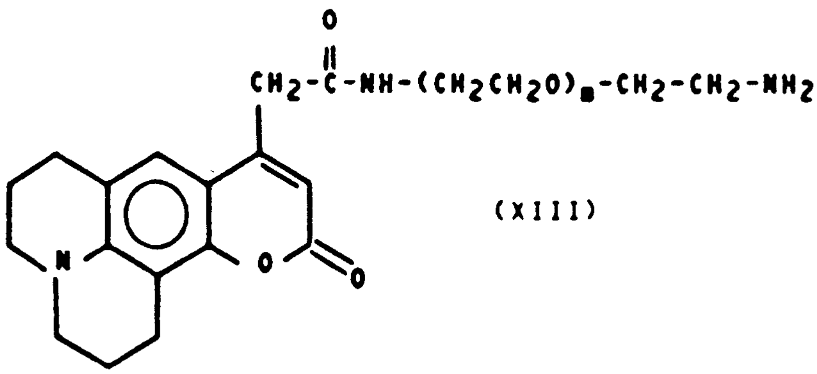

(XIII)

reagieren läßt, wobei m eine ganze Zahl zwischen 1 und 30 ist, um ein Derivat der nachzuweisenden Verbindung zu bilden und um dieses Derivat anschließend durch Absorptionsmessung, Fluorometrie und Chemolumineszenz nachzuweisen.

**15.** Verfahren nach einem der Ansprüche 12 bis 14, dadurch gekennzeichnet, daß der Nachweis durch Chemolumineszenz unter Verwendung eines Oxalsäureesters und von Wasserstoffperoxid durchgeführt wird.

**16.** Verfahren nach Anspruch 15, dadurch gekennzeichnet, daß als Oxalsäureester bis-(Trichloro-2,4,6-Phenyl)-Oxalat oder bis(Dinitro-2,4-Phenyl)-Oxalat verwendet wird.

**17.** Verfahren nach Anspruch 1, dadurch gekennzeichnet, daß n gleich 4 ist und X für das Element I steht.

**18.** Verfahren nach Anspruch 4, dadurch gekennzeichnet, daß m gleich 5 ist.

**19.** Verfahren nach Anspruch 6, dadurch gekennzeichnet, daß m gleich 5 und p gleich 2 ist.

**20.** Verfahren nach Anspruch 7, dadurch gekennzeichnet, daß m gleich 5 und p gleich 2 ist.

**21.** Verfahren nach Anspruch 2, dadurch gekennzeichnet, daß n gleich 4 ist.

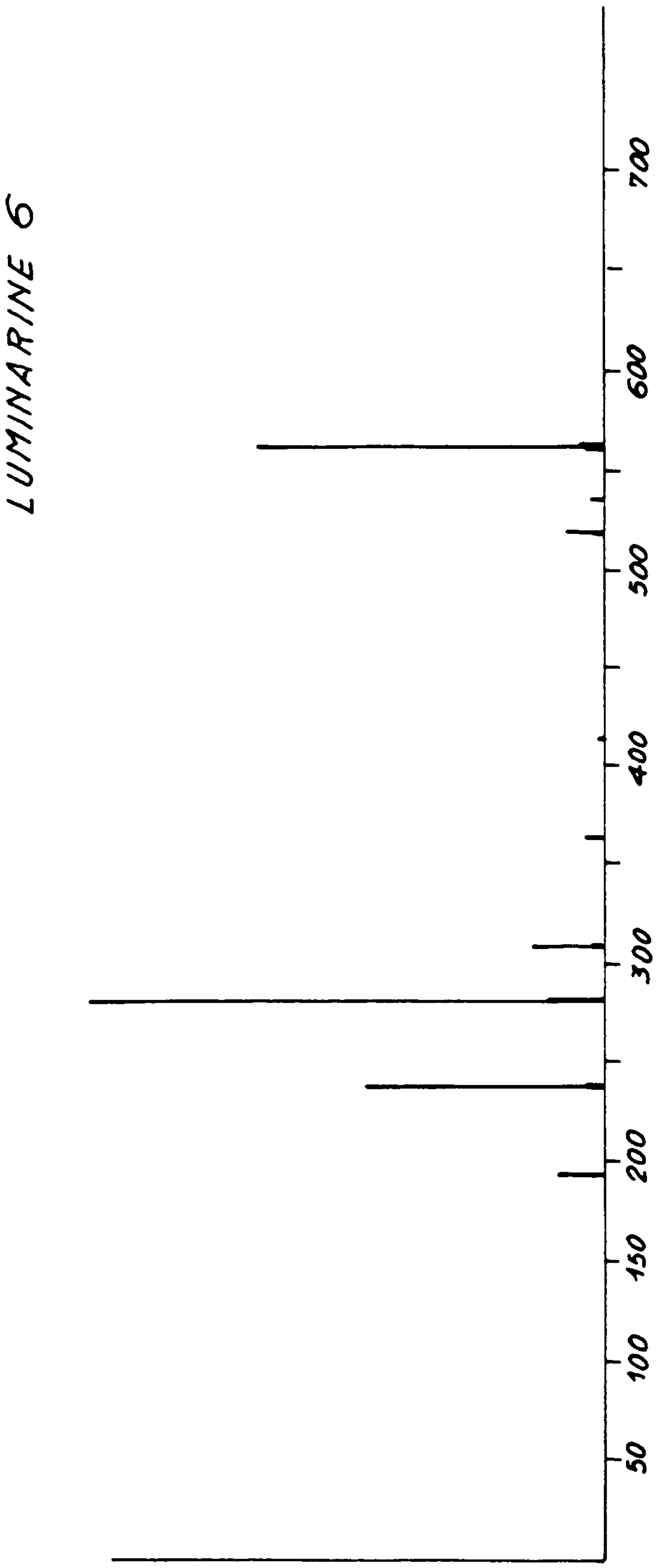


FIG. 1

ACIDE DE FORMULE (XVIIa)

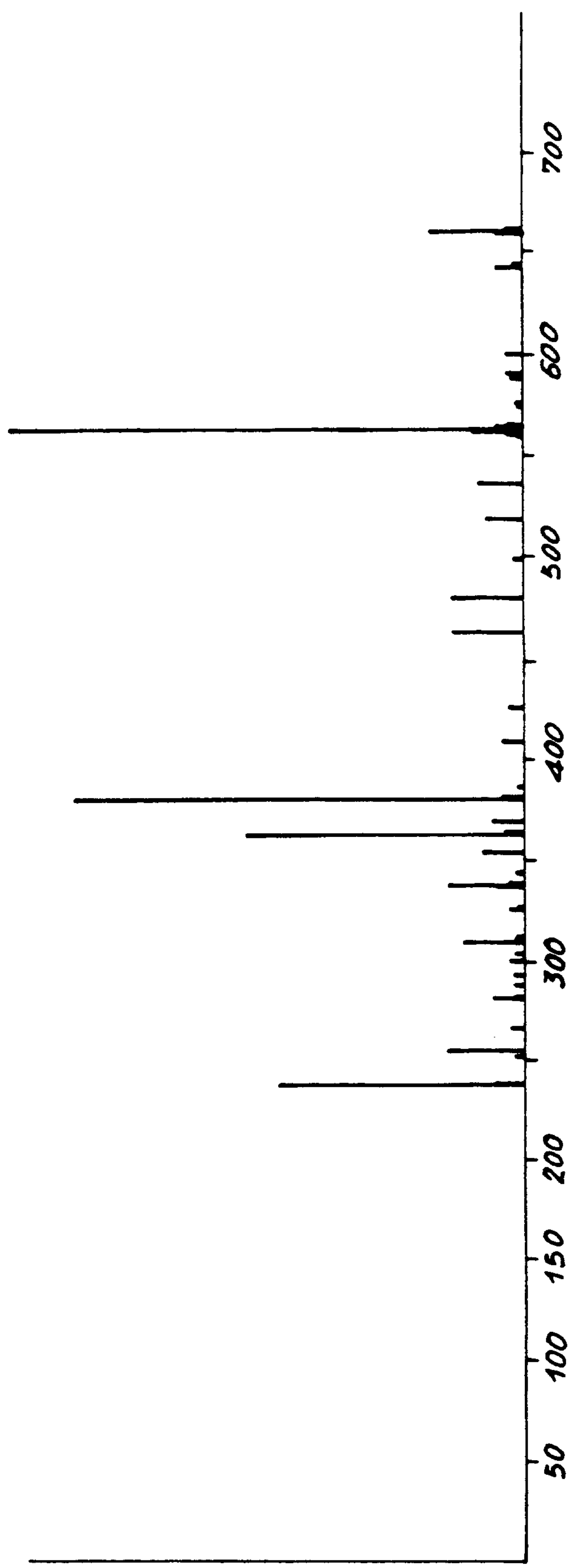


FIG. 2

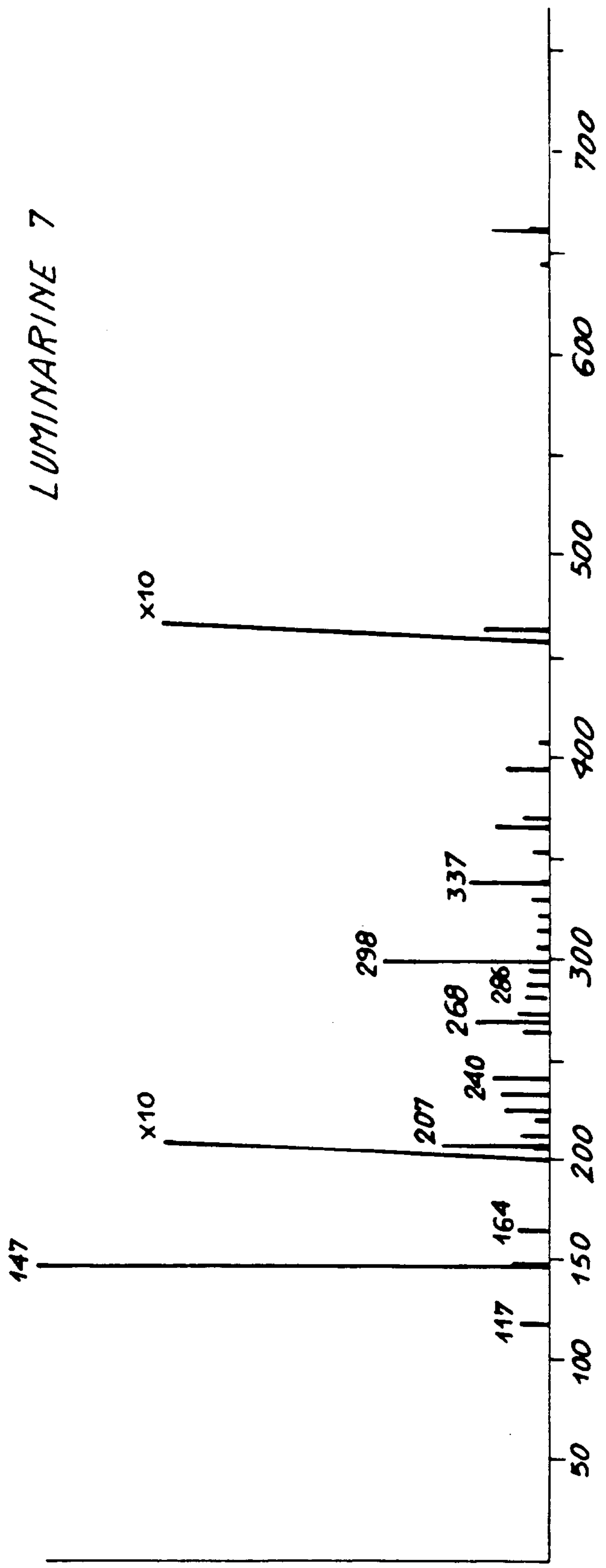
LUMINARINE 7
x10
x10
147
117
164
207
240
268
286
298
337
50
100
150
200
300
400
500
600
700

FIG. 3

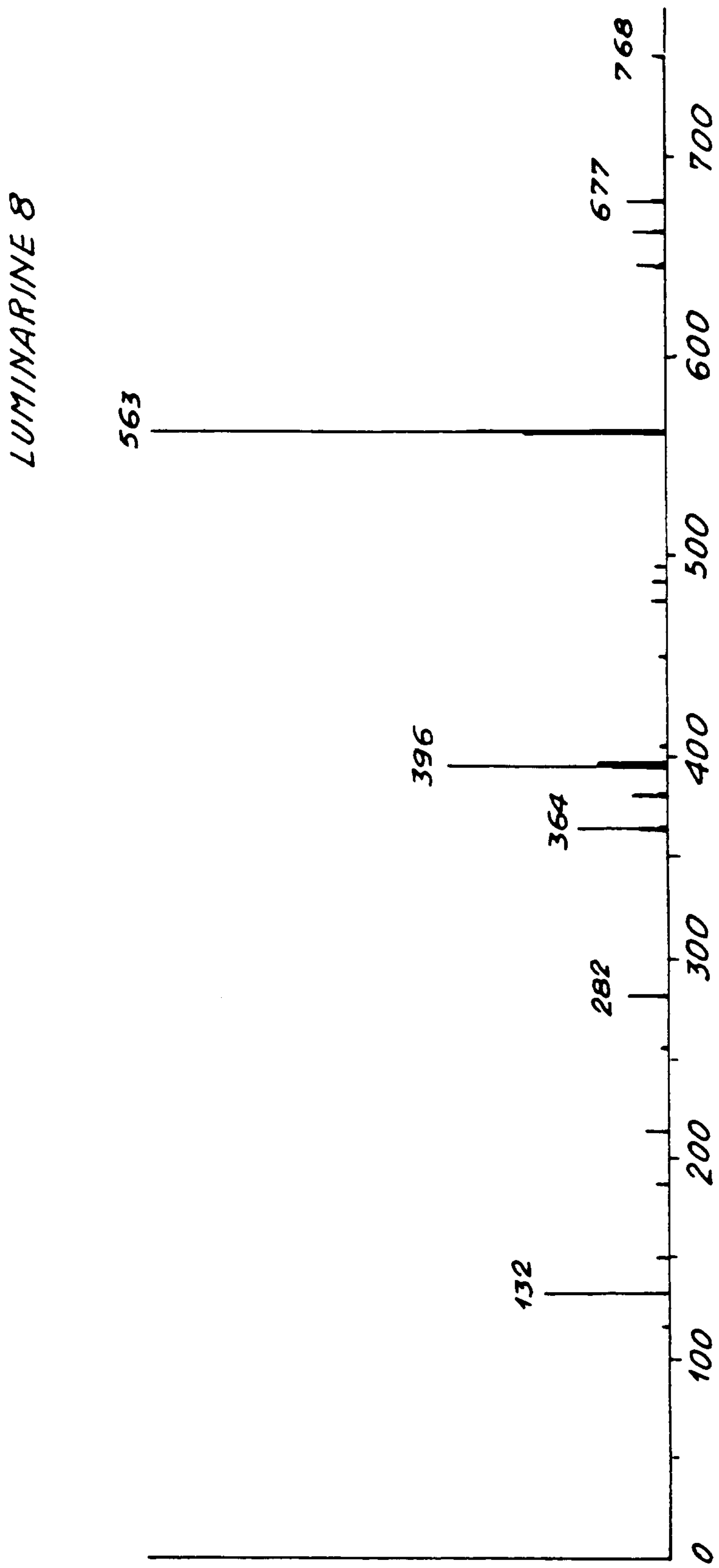
LUMINARINE 8
563
396
364
282
132
677
768
0
100
200
300
400
500
600
700

FIG. 4

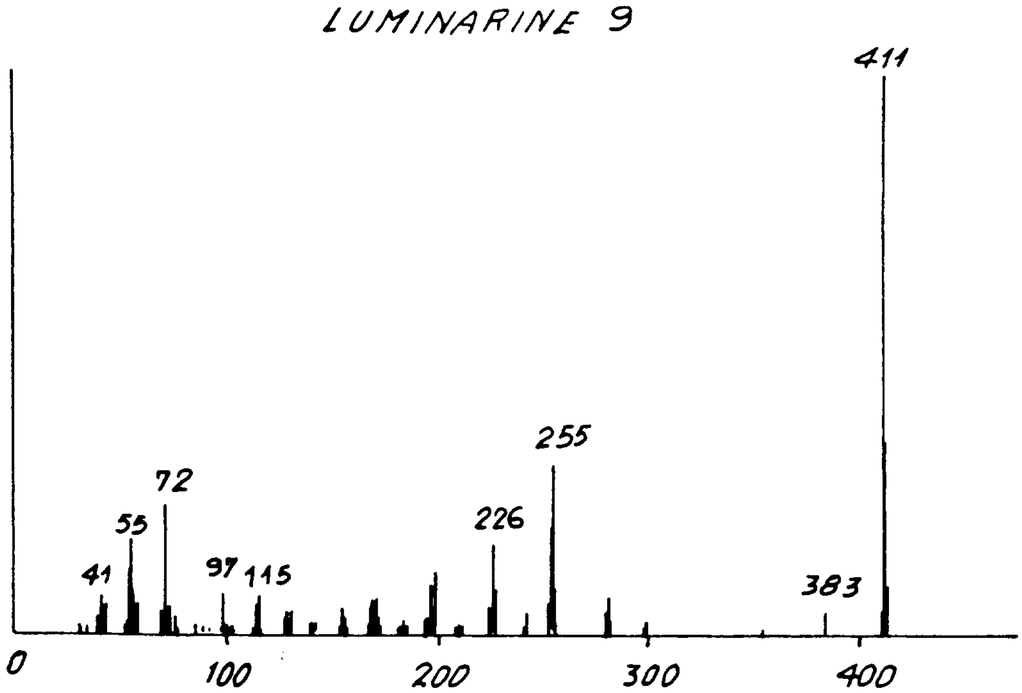
LUMINARINE 9
411
255
72
226
55
41
97
115
383
0
100
200
300
400

FIG. 5

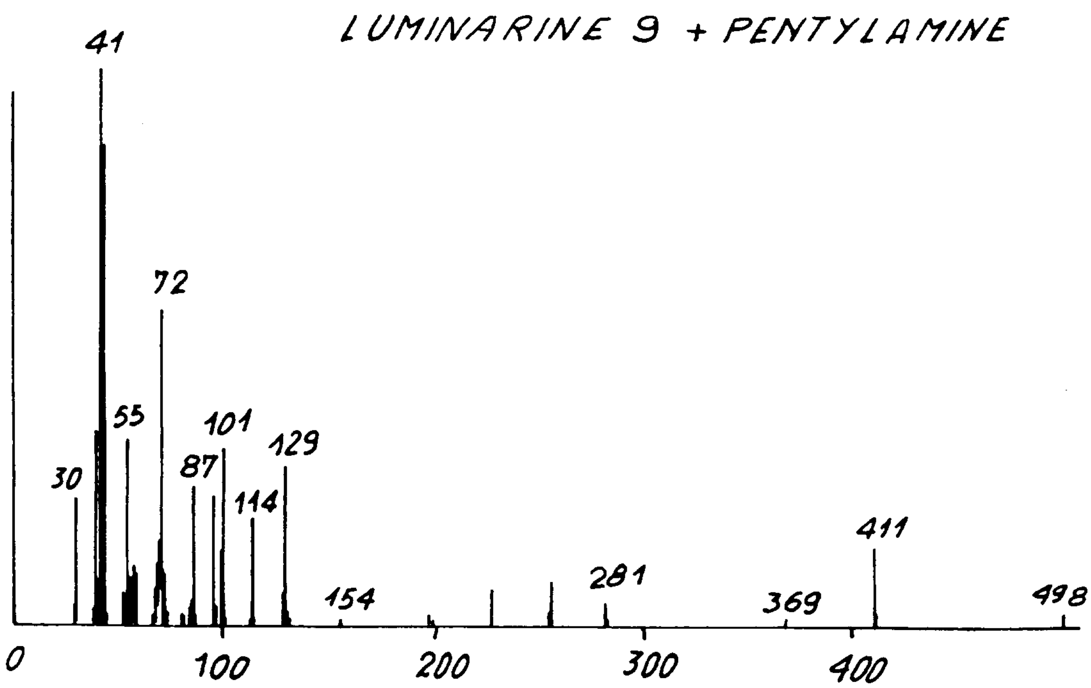
LUMINARINE 9 + PENTYLAMINE
41
72
55
101
129
30
87
114
411
281
154
369
498
0
100
200
300
400

FIG. 6